# EUROPEAN PATENT SPECIFICATION

(11) **EP 4 565 235 B1**
(45) Date of publication and mention of the grant of the patent: **02.09.2026**
(21) Application number: 23761669.3
(22) Date of filing: 04.08.2023
(51) Int. Cl.: A61K 31/4725, A61P 9/12, A61K 45/06

(54) **THE ALDOSTERONE SYNTHASE INHIBITOR BAXDROSTAT FOR USE IN THE TREATMENT OF UNCONTROLLED OR TREATMENT-RESISTANT HYPERTENSION (TRH)**
DER ALDOSTERON-SYNTHASE-INHIBITOR BAXDROSTAT ZUR VERWENDUNG BEI DER BEHANDLUNG VON UNKONTROLLIERTER ODER BEHANDLUNGSRESISTENTER HYPERTONIE (TRH)
L'INHIBITEUR DE LA SYNTHASE DE L'ALDOSTÉRONE BAXDROSTAT POUR SON UTILISATION DANS LE TRAITEMENT DE L'HYPERTENSION NON CONTRÔLÉE OU RÉSISTANTE AU TRAITEMENT (TRH)

(30) Priority: 05.08.2022 US 202263395680 P; 02.09.2022 US 202263374410 P; 03.10.2022 US 202263378120 P; 21.11.2022 US 202263384594 P; 09.12.2022 US 202263386683 P
(43) Date of publication of application: 11.06.2025
(73) Proprietor: CinCor Pharma, Inc., Wilmington, DE 19850 (US)
(72) Inventor: PEARCE, Catherine, Cincinnatti, Ohio 45242 (US)
(74) Representative: AstraZeneca Intellectual Property
(86) International application number: PCT/US2023/029493
(87) International publication number: WO 2024/030615

(56) References cited:
- YUGAR-TOLEDO JUAN CARLOS ET AL: "Managing resistant hypertension: focus on mineralocorticoid-receptor antagonists", VASCULAR HEALTH AND RISK MANAGEMENT, vol. Volume 13, 1 October 2017 (2017-10-01), pages 403 - 411, XP093093505, Retrieved from the Internet <URL:https://www.dovepress.com/getfile.php?fileID=38912> DOI: 10.2147/VHRM.S138599
- SPARKS STEVEN M. ET AL: "Development of Highly Selective Pyrimidine-Based Aldosterone Synthase (CYP11B2) Inhibitors", ACS MEDICINAL CHEMISTRY LETTERS, vol. 10, no. 7, 7 June 2019 (2019-06-07), US, pages 1056 - 1060, XP093093452, ISSN: 1948-5875, DOI: 10.1021/acsmedchemlett.9b00152
- BOGMAN KATRIJN ET AL: "Preclinical and Early Clinical Profile of a Highly Selective and Potent Oral Inhibitor of Aldosterone Synthase (CYP11B2)", HYPERTENSION, vol. 69, no. 1, 1 January 2017 (2017-01-01), US, pages 189 - 196, XP093093520, ISSN: 0194-911X, DOI: 10.1161/HYPERTENSIONAHA.116.07716
- SCHNEIDER MARKUS P. ET AL: "Specific Aldosterone Synthase Inhibition : A Potential Improvement Over Mineralocorticoid Receptor Antagonism?", HYPERTENSION, vol. 69, no. 1, 1 January 2017 (2017-01-01), US, pages 11 - 12, XP093093498, ISSN: 0194-911X, DOI: 10.1161/HYPERTENSIONAHA.116.07939
- OLIVERAS A ET AL: "Resistant hypertension: patient characteristics, risk factors, co-morbidities and outcomes", JOURNAL OF HUMAN HYPERTENSION, NATURE PUBLISHING GROUP, GB, vol. 28, no. 4, 29 August 2013 (2013-08-29), pages 213 - 217, XP037722207, ISSN: 0950-9240, [retrieved on 20130829], DOI: 10.1038/JHH.2013.77
- FORZANO IMMA ET AL: "The selective aldosterone synthase inhibitor Baxdrostat significantly lowers blood pressure in patients with resistant hypertension", FRONTIERS IN ENDOCRINOLOGY, vol. 13, 9 December 2022 (2022-12-09), XP093093412, DOI: 10.3389/fendo.2022.1097968
- FREEMAN MASON W. ET AL: "Phase 2 Trial of Baxdrostat for Treatment-Resistant Hypertension", THE NEW ENGLAND JOURNAL OF MEDICINE, vol. 388, no. 5, 2 February 2023 (2023-02-02), US, pages 395 - 405, XP093093437, ISSN: 0028-4793, DOI: 10.1056/NEJMoa2213169

## Description

### CROSS-REFERENCE TO RELATED APPLICATIONS

This application claims priority to U.S. Provisional Patent Application No. 63/395,680, filed August 5, 2022; U.S. Provisional Patent Application No. 63/374,410, filed September 2, 2022; U.S. Provisional Patent Application No. 63/378,120, filed October 3, 2022; U.S. Provisional Patent Application No. 63/384,594, filed November 21, 2022; and U.S. Provisional Patent Application No. 63/386,683, filed December 9, 2022.

### TECHNICAL FIELD

The disclosure provides compounds for use in the treatment of uncontrolled or treatment-resistant hypertension in a human in need of treatment thereof, comprising administering during a treatment period, 1 mg/day or 2 mg/day of (R)-Compound 1 to the human, wherein the human is on a stable background anti-hypertensive regimen prior to the administration and during the treatment period.

Any references in the description to methods of treatment refer to the compounds, pharmaceutical compositions and medicaments of the invention for use in a method of treatment of the human body by therapy.

### BACKGROUND

Hypertension is responsible for more combined years of lives lost or lived with disability than any other disease. Resistant hypertensive patients represent an especially high-risk subgroup of hypertensive patients as their blood pressures are refractory to the effects of the most potent anti-hypertensive medications available, leaving them exposed to the injurious effects of chronically elevated blood pressures on their hearts, brains, and kidneys. In a five-year retrospective longitudinal cohort study of almost 500,000 Kaiser Permanente southern California.

Resistant hypertensive patients were shown to have markedly enhanced risks for stroke and end stage renal disease, 16 and 32% respectively, as well as higher risk of heart failure, ischemic heart disease and death when compared to patients with non-resistant hypertension. Not only are these conditions associated with some of the most severe and consequential medical catastrophes that patients and their families can experience, but they also account for a substantial fraction of the hospital admissions and healthcare expenditures in the U.S.

Uncontrolled hypertension is hypertension not controlled by one or more antihypertensives and is associated with the same adverse medical consequences, though without having established in those patients that their blood pressures will prove ultimately resistant to triple or greater drug therapy. While physicians and patients have many therapeutic drug classes to choose from for the treatment of hypertension, few agents have been demonstrated to be effective in the resistant hypertension (rHTN) population.

Individuals with uHTN or rHTN have a substantially increased risk of suffering cardiovascular and renal diseases, as failure to achieve blood pressure targets leaves them exposed to the injurious effects of chronically elevated blood pressure in heart, brain and kidneys.

Current guidelines that target lower blood pressure levels than ever before, and the existence of millions of rHTN and uncontrolled hypertension (uHTN) patients in the U.S. speak to a large unmet medical need for novel antihypertensive medications that can work well with existing generic medications.

### SUMMARY

The disclosure provides (R)-Compound 1 for use in the treatment of uncontrolled or treatment-resistant hypertension in a human in need of treatment thereof, comprising administering during a treatment period, 1 mg/day or 2 mg/day of (R)-Compound 1 to the human, wherein the human is on a stable background anti-hypertensive regimen prior to the administration and during the treatment period

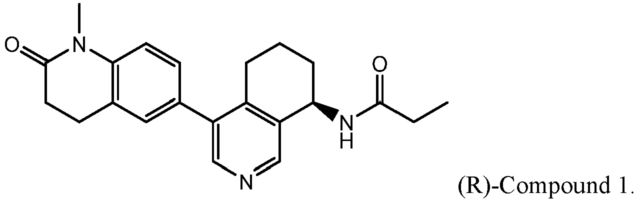

### BRIEF DESCRIPTION OF THE DRAWINGS

FIG. 1 depicts a study design.
FIG. 2 depicts mean change from baseline in SBP, mITT dataset.
FIG. 3A depicts mean serum aldosterone.
FIG. 3B depicts 24-hour urine aldosterone.
FIG. 3C depicts plasma renin activity.
FIG. 3D depicts serum total cortisol.
FIG. 4A depicts baxdrostat summary of treatment effect in aldosterone and renin by gender (serum aldosterone).
FIG. 4B depicts baxdrostat summary of treatment effect in aldosterone and renin by gender (plasma renin activity).

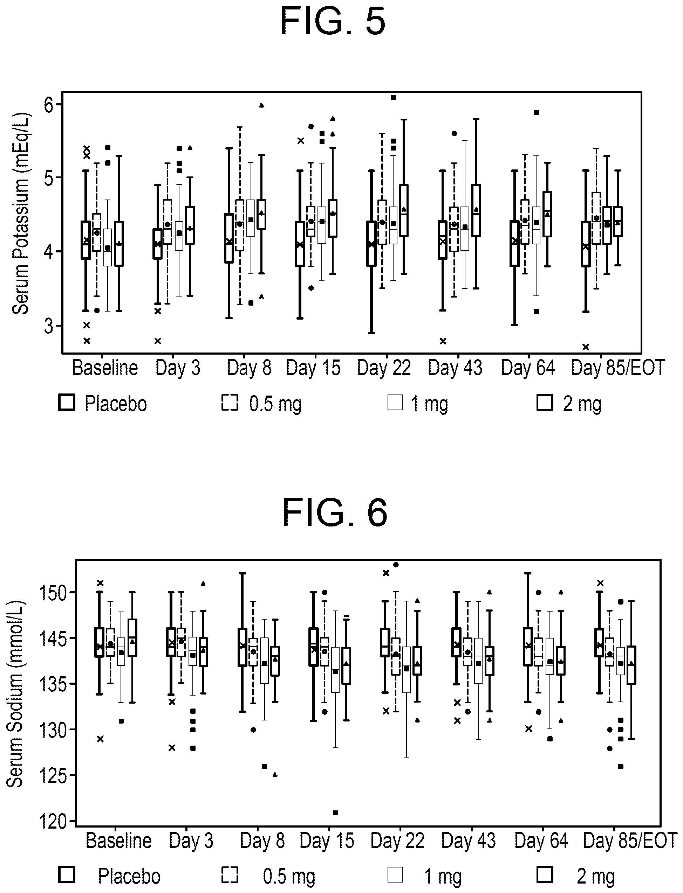

FIG. 5 depicts serum potassium concentration, safety population. Baseline is defined as the measurement at Randomization (Day 1/Visit 4). If missing, baseline is the last measurement prior to the first dose of double-blind study drug. The lower and upper boundary of the box represent the first and third quartile, the line within the box represents the median, the dot within the box represents the mean, whiskers are the minimum and maximum observed data points within 1.5 x the interquartile range, and the dots outside the box represent observed data points outside the 1.5 x interquartile range boundaries.
FIG. 6 depicts serum sodium concentration, safety population. Baseline is defined as the measurement at Randomization (Day 1/Visit 4). If missing, baseline is the last measurement prior to the first dose of double-blind study drug. The lower and upper boundary of the box represent the first and third quartile, the line within the box represents the median, the dot within the box represents the mean, whiskers are the minimum and maximum observed data points within 1.5 x the interquartile range, and the dots outside the box represent observed data points outside the 1.5 x interquartile range boundaries.

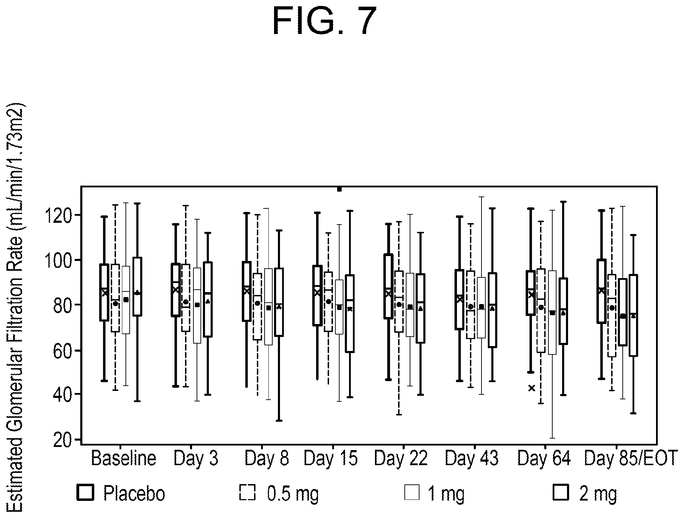

FIG. 7 depicts estimated glomerular filtration rate, safety population. Baseline is defined as the measurement at Randomization (Day 1/Visit 4). If missing, baseline is the last measurement prior to the first dose of double-blind study drug. The lower and upper boundary of the box represent the first and third quartile, the line within the box represents the median, the dot within the box represents the mean, whiskers are the minimum and maximum observed data points within 1.5 x the interquartile range, and the dots outside the box represent observed data points outside the 1.5 x interquartile range boundaries.
FIG. 8 depicts blinded SBP in patients in Study 124.
FIG. 9 depicts the Study 123 design.

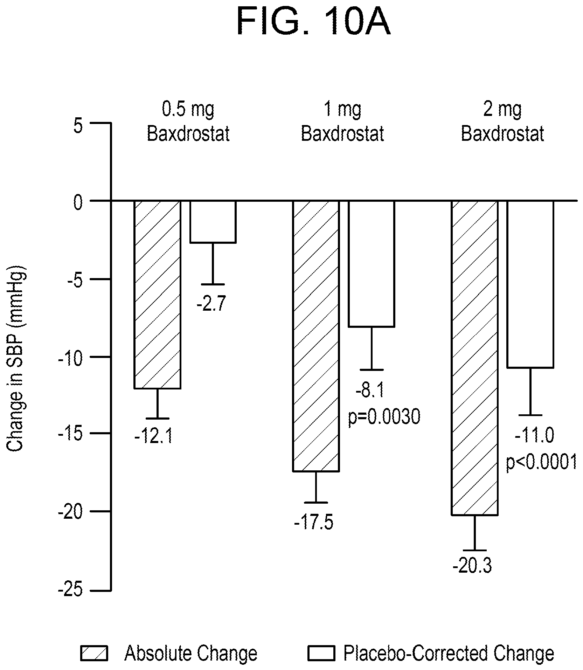

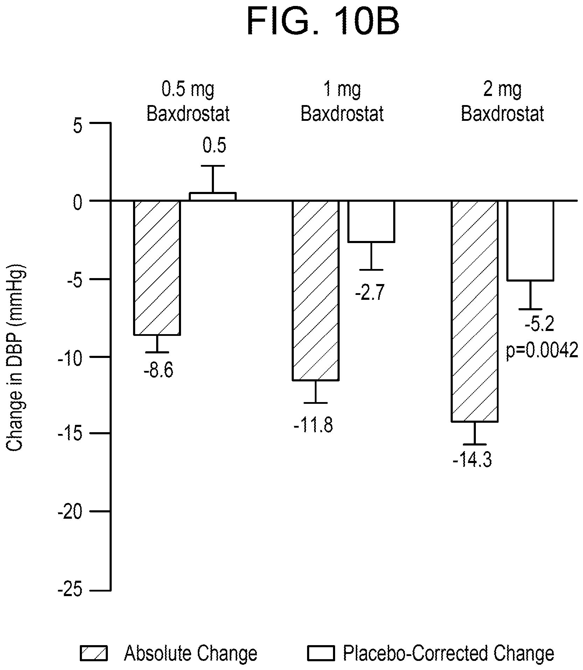

FIGs. 10A and 10B are bar graphs showing that baxdrostat causes dose-dependent decreases in systolic (FIG. 10A) and diastolic (FIG. 10B) blood pressure in patients with resistant hypertension. Data are least squares (LS) mean change ± standard error (SE) and placebo-corrected change ± SE. Baseline is defined as the measurement at randomization. The LS means, SEs, and p-values are from a mixed model repeated measures model with change from baseline as the dependent variable with randomized treatment, visit, and treatment-by-visit interaction as fixed categorical effects; and baseline mean seated systolic blood pressure and baseline glomerular filtration rate as continuous covariates. P values are shown for significant placebo-corrected changes.

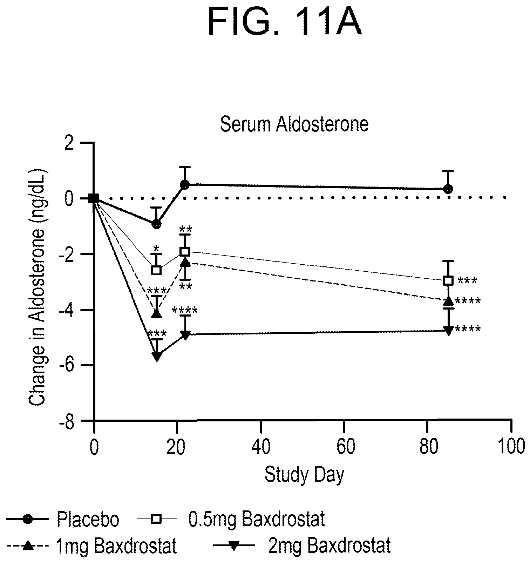

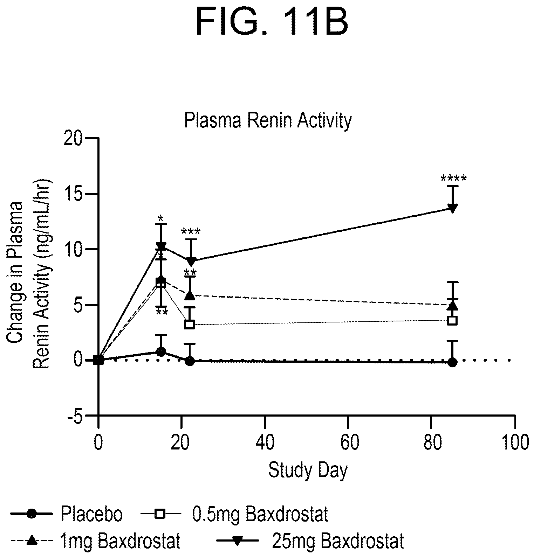

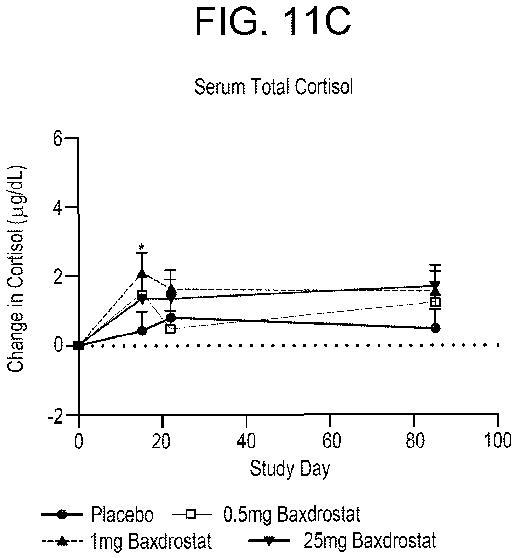

FIGs. 11A-11C are line graphs showing that Baxdrostat reduces serum (FIG. 11A) aldosterone and increases plasma renin activity (FIG. 11B) without reducing serum total cortisol (FIG. 11C). Data are LS means ± SE compared to baseline. Baseline is defined as the measurement at randomization. The LS means, SEs, and p-values are from an analysis of covariance model with change from baseline as the dependent variable with baseline mean seated SBP and baseline glomerular filtration rate as continuous covariates. Statistical significance between baxdrostat and placebo, *p≤0.05; **p≤0.01; ***p≤0.001; ****p≤0.0001.
FIGs. 12A and 12B are line graphs of plasma baxdrostat concentration vs. time. Data are mean ± standard deviation.
FIG. 13 is the study design for the interim results of Example 9.
FIG. 14 is a schematic of the patient disposition for Part 1 of the study of Example 9.
FIG. 15A is a bar graph showing the change in systolic blood pressure (mmHg) for Part 1, Week 8 of Example 9. FIG. 15B is a bar graph showing the change in systolic blood pressure (mmHg) for Part 1, Week 8 of Example 9, for non-Hispanic/Latino patients. In these figures, *Nominal p value and SBP primary endpoint is change in blood pressure vs placebo.
FIG. 16 is a line graph showing the mean change on sited SBP over time for the all Intent-To- Treat Population.
FIG. 17A is a line graph showing aldosterone changes for the complete patient population of Example 9. FIG. 17B is a line graph showing aldosterone changes for the Hispanic patient population of Example 9. FIG. 17C is a line graph showing aldosterone changes for the non-Hispanic patient population of Example 9.
FIG. 18A is a line graph showing plasma renin activity changes for the complete patient population of Example 9. FIG. 17B is a line graph showing plasma renin activity changes for the Hispanic patient population of Example 9. FIG. 17C is a line graph showing plasma renin activity changes for the non-Hispanic patient population of Example 9.
FIG. 19 is a line graph showing mean serum potassium levels over time.
FIG. 20 is a line graph showing plasma metformin concentration vs. time. In this figure, data are mean ± standard deviation.
FIGs. 21A-21B are dot plots of plasma baxdrostat PK parameters vs. baseline eGFR. In these figures, plasma baxdrostat Cₘₐₓ (FIG. 21A), AUC₀₋ₗₐₛₜ (FIG. 21B), AUC_{0-inf} (FIG. 21C), and t_{1/2} (FIG. 21D) vs. baseline eGFR. Baseline was defined as the last measurement prior to the dose of baxdrostat. Baseline eGFR value is presented as a continuous variable. Linear regression analysis was performed using Graphpad Prism 7.03.
FIG. 22 is a line graph of plasma baxdrostat-M concentration vs. time plasma concentration (ng/mL) by renal function group from 0-168 hours. In this figure, data are mean ± standard deviation.
FIG. 23 is a line graph of cumulative amount of baxdrostat excreted in urine. In this figure, cumulative concentration (µg) of baxdrostat by renal function group from 0-168 hours. Data are mean ± standard deviation.
FIG. 24 is the study design for cohort 1. In this figure, n = number of participants in that category.
FIG. 25 is the study design for cohort 2. In this figure, n = number of participants in that category.

### DETAILED DESCRIPTION OF ILLUSTRATIVE EMBODIMENTS

The disclosure may be more fully appreciated by reference to the following description, including the following definitions and examples. Certain features of the disclosed compositions and methods which are described herein in the context of separate aspects, may also be provided in combination in a single aspect. Alternatively, various features of the disclosed compositions and methods that are, for brevity, described in the context of a single aspect, may also be provided separately or in any sub combination. The appended claims define the scope of the invention. Unless otherwise defined, all technical and scientific terms used herein have the same meaning as commonly understood by one of ordinary skill in the art to which this disclosure pertains. The terminology used in the description is for describing particular embodiments only and is not intended to be limiting of the disclosure.

In the disclosure, the singular forms "a," "an," and "the" include the plural reference, and reference to a particular numerical value includes at least that particular value, unless the context clearly indicates otherwise.

When a value is expressed as an approximation by use of the descriptor "about" it will be understood that the particular value forms another embodiment. In general, use of the term "about" indicates approximations that can vary depending on the desired properties sought to be obtained by the disclosed subject matter and is to be interpreted in the specific context in which it is used, based on its function. The person skilled in the art will be able to interpret this as a matter of routine. In some cases, the number of significant figures used for a particular value may be one non-limiting method of determining the extent of the word "about." In other cases, the gradations used in a series of values may be used to determine the intended range available to the term "about" for each value. Where present, all ranges are inclusive and combinable. That is, references to values stated in ranges include every value within that range.

When a list is presented, unless stated otherwise, it is to be understood that each individual element of that list and every combination of that list is to be interpreted as a separate embodiment. For example, a list of embodiments presented as "A, B, or C" is to be interpreted as including the embodiments, "A," "B," "C," "A or B," "A or C," "B or C," or "A, B, or C."

It is to be appreciated that certain features of the invention which are, for clarity, described herein in the context of separate embodiments, may also be provided in combination in a single embodiment. That is, unless obviously incompatible or excluded, each individual embodiment is deemed to be combinable with any other embodiment(s) and such a combination is considered to be another embodiment. Conversely, various features of the invention that are, for brevity, described in the context of a single embodiment, may also be provided separately or in any sub-combination. It is further noted that the claims may be drafted to exclude an optional element. As such, this statement is intended to serve as antecedent basis for use of such exclusive terminology as "only" and the like in connection with the recitation of claim elements, or use of a "negative" limitation.

### Administration Methods

The disclosure is directed to baxdrostat ((+)-(R)-N-(4-(1-methyl-2-oxo-1,2,3,4-tetrahydroquinolin-6-yl)-5,6,7,8-tetrahydroisoquinolin-8-yl)propionamide) for use in the treatment of uncontrolled or treatment-resistant hypertension in a human in need of treatment thereof, comprising administering during a treatment period, 1 mg/day or 2 mg/day of baxdrostat to the human, wherein the human is on a stable background anti-hypertensive regimen prior to the administration and during the treatment period 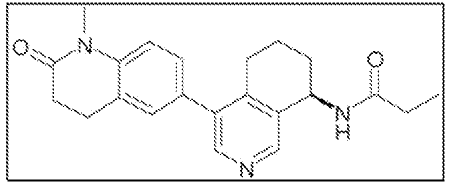 baxdrostat

The terms "baxdrostat" and "(R)-Compound 1" as used herein are interchangeable and refer to (R)-N-(4-(1-methyl-2-oxo-1,2,3,4-tetrahydroquinolin-6-yl)-5,6,7,8-tetrahydroisoquinolin-8-yl)propionamide ((R)-Compound 1) having the following structure:

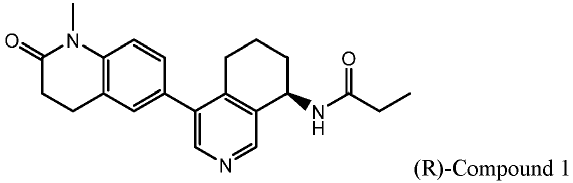

The disclosure also contemplates salts of (R)-Compound 1, and their use in the methods described herein. In some embodiments, the salt is pharmaceutically acceptable. "Pharmaceutically acceptable" refers to properties and/or substances that are acceptable to the patient/human from a pharmacological/toxicological vantage, and to the manufacturing pharmaceutical chemist from a physical/chemical vantage regarding composition, formulation, stability, patient acceptance, and bioavailability.

A pharmaceutically acceptable salt of (R)-Compound 1 includes salts with a pharmaceutically acceptable acid or base, *e.g.,* inorganic acids, *e.g.,* hydrochloric, sulfuric, phosphoric, diphosphoric, hydrobromic, hydroiodic, nitric, and phosphoric acid and organic acids, *i.e.,* adipic, citric, fumaric, maleic, malic, malonic, mandelic, ascorbic, oxalic, succinic, tartaric, benzoic, acetic, methanesulphonic, ethanesulphonic, benzenesulphonic, cyclohexylsulfamic (cyclamic), edisylate, glutaric, or p-toluenesulfonic acid. Pharmaceutically acceptable bases include alkali metal, *e.g*., sodium or potassium, and alkali earth metal, *e.g.,* calcium or magnesium, hydroxides, and organic bases, *e.g.,* alkyl amines, arylalkyl amines and heterocyclic amines.

The terms "subject" and "patient" are used interchangeably and typically refer to mammals. In some embodiments, the patient or subject is a human. In other embodiments, the patient or subject is a veterinary or farm animal, a domestic animal or pet, or animal used for conducting clinical research. In some embodiments, the subject or patient is at least 18 years of age, *i.e.,* an adult.

According to the disclosure, baxdrostat lower bloods pressure in the treatment of hypertension in combination with other antihypertensive agents. The intended population for baxdrostat is for treatment-resistant hypertension (rHTN), defined as hypertension that is being treated unsuccessfully with ≥3 antihypertensive agents, and uncontrolled hypertension (uHTN), defined as hypertension that is being unsuccessfully treated with 1 or 2 antihypertensive agents.

Hypertension is defined as a systolic blood pressure (SBP) of >130 mmHg or a diastolic blood pressure (DBP) of > 80 mmHg (Table 1). The goal of treatment is to achieve an SBP of < 130 mmHg and a DBP of 80 mmHg.

| **Table 1: Categories of Blood Pressure for Adults in the US** | |
|---|---|
| **Blood Pressure Category** | **Definition** |
| Normal | SBP <120 mmHg and DBP <80 mmHg |
| Elevated | SBP 120-129 mmHg and DBP <80 mmHg |
| Stage 1 hypertension | SBP 130-139 mmHg and DBP 80-89 mmHg |
| Stage 2 hypertension | SBP ≥ 140 mmHg and DBP ≥90 mmHg |
| Uncontrolled hypertension | Blood pressure that remains elevated above goal secondary to poor adherence and/or inadequate treatment regimen |
| Resistant hypertension | Blood pressure that remains elevated above goal despite concurrent use of 3 antihypertensive agents of different classes, one of which must be a diuretic |
| Controlled resistant hypertension | Blood pressure achieves target values on ≥4 antihypertensive agents |
| Abbreviations: DBP, diastolic blood pressure; SBP, systolic blood pressure | |

Hypertension includes stages 1 and 2 hypertension and hypertensive crisis. In some embodiments, the patient/human has stage 1 hypertension with a systolic pressure of about 130 to about 139 mm Hg and/or a diastolic pressure of about 80 to about 89 mm Hg. In other embodiments, the patient/human has stage 2 hypertension with a systolic pressure of about 140 mm Hg or higher and/or a diastolic pressure of about 90 mm Hg or higher. In further embodiments, the patient/human has hypertensive crisis with a blood pressure measurement higher than about 180/120 mm Hg. "Hypertension" also includes resistant hypertension (rHTN) and uncontrolled hypertension (uHTN). In some embodiments, the human has rHTN. In other embodiments, the human has uHTN. In other embodiments, the subject has chronic kidney disease. In further embodiments, the subject has chronic kidney disease and rHTN. In yet other embodiments, In certain aspects, the subject has chronic kidney disease and uHTN.

### Prior to (R)-Compound 1 Administration

In certain embodiments, the human has an estimated glomerular filtration rate (eGFR) of ≥ 45 mL/min/1.73m² prior to the administration of (R)-Compound 1. In certain aspects, the human has a eGFR of at least about 45, about 50, about 55, about 60, about 65, about 70, about 75, about 80, about 85, about 90, or about 95 mL/min/1.73m² prior to the administration of (R)-Compound 1. In other aspects, the human has a eGFR of about 45 to about 95, about 45 to about 90, about 45 to about 85, about 45 to about 80, about 45 to about 75, about 45 to about 70, about 45 to about 65, about 45 to about 60, about 45 to about 55, about 50 to about 90, about 50 to about 85, about 50to about 80., about 50 to about 75, about 50 to about 70, about 50 to about 65, about 50 to about 60, about 60 to about 90, about 60 to about 85, about 60 to about 80, about 60 to about 75, about 60 to about 70, about 70 to about 90, about 70 to about 85, about 70 to about 80, about 80 to about 90, or about 85 to about 90 prior to the administration of (R)-Compound 1.

In other embodiments, the human has a mean seated systolic blood pressure (siSBP) of ≥130 mmHg, prior to the administration of (R)-Compound 1. In certain aspects, the human has a mean seated systolic blood pressure (siSBP) of ≥140 mmHg, prior to the administration of (R)-Compound 1. In other aspects, the human has a mean seated systolic blood pressure (siSBP) of ≥140 mmHg to <170 mmHg, prior to the administration of (R)-Compound 1. In further aspects, the human has a siSBP of about 140, about 145, about 150, about 155, about 160, or about 165 mmHg, prior to the administration of (R)-Compound 1. In other aspects, the human has a siSBP of about 140 to about 165, about 140 to about 160, about 140 to about 155, about 140 to about 150, about 145 to about 165, about 145 to about 160, about 145 to about 155, about 150 to about 165, about 150 to about 160, or about 160 to about 165 mmHg, prior to the administration of (R)-Compound 1. In further aspects, the human has a siSBP of <145 mmHg, prior to the administration of (R)-Compound 1. In yet other aspects, the human has a siSBP of ≥145 mmHg, prior to the administration of (R)-Compound 1.

In further embodiments, the human has a serum potassium level ≥3.5 and < 5.0 mmol/L prior to the administration of (R)-Compound 1. In certain aspects, the human has a serum potassium level of about 3.5, about 3.75, about 4, about 4.25, about 4.5, about 4.75, or about 5, prior to the administration of (R)-Compound 1. In other aspects, the human has a serum potassium level of about 3.5 to about 5, about 3.5 to about 3.75, about 3.5 to about 4, about 3.5 to about 3.75, about 3.75 to about 5, about 3.75 to about 4.75, about 3.75 to about 4.5, about 3.75 to about 4.25, about 3.75 to about 4, about 4 to about 5, about 4 to about 4.75, about 4 to about 4.5, about 4 to about 4.25, about 4.25 to about 5, about 4.25 to about 4.75, about 4.25 to about 4.5, about 4.5 to about 5, about 4.5 to about 4.75, or about 4.75 to about 5, prior to the administration of (R)-Compound 1.

In yet other embodiments, the human has a morning cortisol level > 3 µg/dL prior to the administration of (R)-Compound 1. In certain aspects, the human has a morning cortisol level greater than about 3, about 4, about 5, about 7, about 10, about 12, about 15, about 17, about 20, about 22, or about 25 µg/dL prior to the administration of (R)-Compound 1. In other aspects, the human has a morning cortisol level of about 3 to about 25, about 3 to about 22, about 3 to about 20, about 3 to about 17, about 3 to about 15, about 3 to about 12, about 3 to about 10, about 3 to about 7, about 5 to about 25, about 5 to about 22, about 5 to about 20, about 5 to about 17, about 5 to about 15, about 5 to about 12, about 5 to about 10, about 10 to about 25, about 10 to about 22, about 10 to about 20, about 10 to about 17, about 10 to about 15, about 15 to about 25, about 15 to about 22, about 15 to about 20, or about 20 to about 25 µg/dL prior to the administration of (R)-Compound 1.

### After (R)-Compound 1 Administration

(R)-Compound is administered to the human in a treatment period. The term "treatment period" as used herein refers to a period of time in which it takes to administered (R)-Compound 1 to the human.

After treatment with (R)-Compound 1, the human's mean seated systolic blood pressure (siSBP) is decreased, as compared to baseline, at or after about 12 weeks of treatment, as compared to a placebo. In some embodiments, the administration results in a seated systolic blood pressure (siSBP) of <130 mmHg after a 12-week treatment period. In further embodiments, administration of (R)-Compound 1 results in a decrease from baseline in siSBP by at least about 1, at least about 2, at least about 3, at least about 4, at least about 5, at least about 6, at least about 7, at least about 8, at least about 9, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, at least about 25, at least about 26, at least about 27, at least about 28, at least about 29, or at least about 30 mmHg, as compared to baseline, at or after about 12 weeks of treatment, as compared to a placebo. In other embodiments, administration of (R)-Compound 1 results in a decrease from baseline in siSBP by about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30 mmHg, as compared to baseline, at or after about 12 weeks of treatment, as compared to a placebo. In other embodiments, administration of (R)-Compound 1 results in a decrease from baseline in siSBP by about 1 to about 30, about 1 to about 25, about 1 to about 20, about 1 to about 15, about 1 to about 10, about 1 to about 5, about 5 to about 30, about 5 to about 25, about 5 to about 20, about 5 to about 15, about 5 to about 10, about 10 to about 30, about 10 to about 25, about 10 to about 20, about 10 to about 15, about 15 to about 30, about 15 to about 25, about 15 to about 20, about 20 to about 30, about 20 to about 25, or about 25 to about 30 mmHg, as compared to baseline, at or after about 12 weeks of treatment, as compared to a placebo. In further embodiments, administration of 2 mg/day (R)-Compound 1 results in a decrease in the human's mean seated systolic blood pressure (siSBP) as compared to baseline, at or after a 12-week treatment period, as compared to a placebo. In other embodiments, administration of 1 mg/day of the (R)-Compound 1 results in decrease in the human's mean seated systolic blood pressure (siSBP) as compared to baseline, at or after a 12-week treatment period, as compared to a placebo. In further embodiments, the human is administered 1 mg/day of (R)-Compound 1 and the administration results in about an 8 mmHg decrease in the human's mean seated systolic blood pressure, at or after a 12-week treatment period, as compared to baseline. In still other embodiments, the human is administered 1 mg/day of (R)-Compound 1 and the administration results in more than an 8 mmHg decrease in the human's mean seated systolic blood pressure, at or after a 12-week treatment period, as compared to baseline. In yet further embodiments, the human is administered 2 mg/day of (R)-Compound 1 and the administration results in about an 11 mmHg decrease in the human's mean seated systolic blood pressure, at or after a 12-week treatment period, as compared to baseline. In other embodiments, the human is administered 2 mg/day of (R)-Compound 1 and the administration results in more than an 11 mg Hg decrease in the human's mean seated systolic blood pressure, at or after a 12-week treatment period, as compared to baseline.

After treatment with (R)-Compound 1, the administration results in a decrease as compared to baseline in seated systolic blood pressure (siSBP), at or after a 24-week treatment period, as compared to placebo. In some embodiments, administration of (R)-Compound 1 results in a decrease from baseline in siSBP by at least about 1, at least about 2, at least about 3, at least about 4, at least about 5, at least about 6, at least about 7, at least about 8, at least about 9, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, at least about 25, at least about 26, at least about 27, at least about 28, at least about 29, or at least about 30 mmHg, as compared to baseline, at or after about 24 weeks of treatment, as compared to a placebo. In further embodiments, administration of (R)-Compound 1 results in a decrease from baseline in siSBP by about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30 mmHg, as compared to baseline, at or after about 24 weeks of treatment, as compared to a placebo. In other embodiments, administration of or (R)-Compound 1 results in a decrease from baseline in siSBP by about 1 to about 30, about 1 to about 25, about 1 to about 20, about 1 to about 15, about 1 to about 10, about 1 to about 5, about 5 to about 30, about 5 to about 25, about 5 to about 20, about 5 to about 15, about 5 to about 10, about 10 to about 30, about 10 to about 25, about 10 to about 20, about 10 to about 15, about 15 to about 30, about 15 to about 25, about 15 to about 20, about 20 to about 30, about 20 to about 25, or about 25 to about 30 mmHg, as compared to baseline, at or after about 24 weeks of treatment, as compared to a placebo.

After treatment with (R)-Compound 1, the human's seated diastolic blood pressure (siDBP) is decreased, as compared to baseline, at or after about 12 weeks of treatment, as compared to a placebo. In some embodiments, administration of (R)-Compound 1 results in a decrease from baseline in siDBP by at least about 1, at least about 2, at least about 3, at least about 4, at least about 5, at least about 6, at least about 7, at least about 8, at least about 9, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, at least about 25, at least about 26, at least about 27, at least about 28, at least about 29, or at least about 30 mmHg, as compared to baseline, at or after about 12 weeks of treatment, as compared to a placebo. In other embodiments, administration of (R)-Compound 1 results in a decrease from baseline in siDBP by about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30 mmHg, as compared to baseline, at or after about 12 weeks of treatment, as compared to a placebo. In other embodiments, administration of or (R)-Compound 1 results in a decrease from baseline in siDBP by about 1 to about 30, about 1 to about 25, about 1 to about 20, about 1 to about 15, about 1 to about 10, about 1 to about 5, about 5 to about 30, about 5 to about 25, about 5 to about 20, about 5 to about 15, about 5 to about 10, about 10 to about 30, about 10 to about 25, about 10 to about 20, about 10 to about 15, about 15 to about 30, about 15 to about 25, about 15 to about 20, about 20 to about 30, about 20 to about 25, or about 25 to about 330 mmHg, as compared to baseline, at or after about 12 weeks of treatment, as compared to a placebo.

After treatment with (R)-Compound 1, the human's ambulatory 24-hour average systolic blood pressure (SBP) is decreased, as compared to baseline, at or after about 12 weeks of treatment, as compared to a placebo. In other embodiments, administration of (R)-Compound 1 results in a decrease from baseline in ambulatory 24-hour average SBP by about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30 mmHg, as compared to baseline, at or after about 12 weeks of treatment, as compared to a placebo. In further embodiments, administration of or (R)-Compound 1 results in a decrease from baseline in ambulatory 24-hour average SBP by about 1 to about 30, about 1 to about 25, about 1 to about 20, about 1 to about 15, about 1 to about 10, about 1 to about 5, about 5 to about 30, about 5 to about 25, about 5 to about 20, about 5 to about 15, about 5 to about 10, about 10 to about 30, about 10 to about 25, about 10 to about 20, about 10 to about 15, about 15 to about 30, about 15 to about 25, about 15 to about 20, about 20 to about 30, about 20 to about 25, or about 25 to about 330 mmHg, as compared to baseline, at or after about 12 weeks of treatment, as compared to a placebo.

In some embodiments, the human's ambulatory 24-hour average diastolic blood pressure (DBP) is decreased, as compared to baseline, at or after about 12 weeks of treatment, as compared to a placebo. In further embodiments, administration of (R)-Compound 1 results in a decrease from baseline in ambulatory 24-hour average DBP by at least about 1, at least about 2, at least about 3, at least about 4, at least about 5, at least about 6, at least about 7, at least about 8, at least about 9, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, at least about 25, at least about 26, at least about 27, at least about 28, at least about 29, or at least about 30 mmHg, as compared to baseline, at or after about 12 weeks of treatment, as compared to a placebo. In other embodiments, administration of (R)-Compound 1 results in a decrease from baseline in ambulatory 24-hour average DBP by about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30 mmHg, as compared to baseline, at or after about 12 weeks of treatment, as compared to a placebo. In further embodiments, administration of or (R)-Compound 1 results in a decrease from baseline in ambulatory 24-hour average DBP by about 1 to about 30, about 1 to about 25, about 1 to about 20, about 1 to about 15, about 1 to about 10, about 1 to about 5, about 5 to about 30, about 5 to about 25, about 5 to about 20, about 5 to about 15, about 5 to about 10, about 10 to about 30, about 10 to about 25, about 10 to about 20, about 10 to about 15, about 15 to about 30, about 15 to about 25, about 15 to about 20, about 20 to about 30, about 20 to about 25, or about 25 to about 330 mmHg, as compared to baseline, at or after about 12 weeks of treatment, as compared to a placebo.

In certain aspects, the administration of the (R)-Compound 1 does not result in a clinically significant adverse event in the human, as compared to placebo. In other aspects, the administration does not result in a clinically significant lowering in mean serum cortisol levels, as compared to placebo.

In certain aspects, there is provided (R)-Compound 1 for use in any of the methods described herein.

"Treating" or variations thereof refers to eliminating or reducing at least one physical parameter of a disease or disorder, such as hypertension. In some embodiments, the disease or disorder is hypertension. In other embodiments, the disease or disorder is resistant hypertension. In some embodiments, the disease or disorder is uncontrolled hypertension.

### Pharmaceutical Compositions

The disclosure also provides pharmaceutical compositions comprising (R)-Compound 1 and one or more pharmaceutically acceptable excipients. In some embodiments, the pharmaceutical composition comprises lactose anhydrous, microcrystalline cellulose, croscarmellose sodium, colloidal silicon dioxide, and magnesium stearate.

The amount of (R)-Compound 1 used alone or in the pharmaceutical formulations may also be expressed by way of an amount. In some embodiments, the pharmaceutical formulations contain about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 1.5 mg, or about 2 mg of (R)-Compound 1. In other embodiments, the pharmaceutical formulations contain about 0.5 mg of (R)-Compound 1. In further embodiments, the pharmaceutical formulations contain about 1 mg of (R)-Compound 1. In yet other embodiments, the pharmaceutical formulations contain about 2 mg of (R)-Compound 1.

(R)-Compound 1, or a pharmaceutical composition containing (R)-Compound 1, is administered on a daily basis. In further embodiments, about 1 mg/day of (R)-Compound 1 is administered to the human. In yet other embodiments, about 2 mg/day of (R)-Compound 1 is administered to the human.

(R)-Compound 1 may be administered in a single dose or divided doses. In some embodiments, (R)-Compound 1 is administered in a single dose. In further embodiments, (R)-Compound 1 is administered in divided doses. In yet other embodiments, one tablet is administered. In still further embodiments, the tablet comprises 1 mg of (R)-Compound 1. In other embodiments, the tablet comprises 2 mg of (R)-Compound 1. One of skill in the art would be able to determine and use other combinations of the tablet doses based on the dosage of (R)-Compound 1 needed.

### Administration Routes

(R)-Compound 1 or pharmaceutical formulations containing the same may be administered by any acceptable route. In some embodiments, administration is oral, transdermal, parenteral, or a combination thereof. In further embodiments, administration is oral. In other embodiments, administration is transdermal. In still further embodiments, administration is parenteral.

(R)-Compound 1, or a pharmaceutical formulation containing (R)-Compound 1, may be formulated for administration in solid or liquid forms. In some embodiments, (R)-Compound 1, or a pharmaceutical formulation containing (R)-Compound 1, is formulated in the form of a tablet, caplet, capsule, powder, softgel, suspension or liquid, or a combination thereof. In other embodiments, (R)-Compound 1, or a pharmaceutical formulation containing (R)-Compound 1, is formulated in the form of a tablet. In further embodiments, (R)-Compound 1, or a pharmaceutical formulation containing (R)-Compound 1, is formulated in the form of a caplet. In yet other embodiments, (R)-Compound 1, or a pharmaceutical formulation containing (R)-Compound 1, is formulated in the form of a capsule. In still further embodiments, each tablet, caplet, capsule, powder, softgel, suspension or liquid dose contains about 0.5 mg, about 1 mg, about 1.5 mg, or about 2 mg of (R)-Compound 1. In other embodiments, each tablet, caplet, capsule, powder, softgel, suspension or liquid dose contains about 0.5 mg of (R)-Compound 1. In further embodiments, each tablet, caplet, capsule, powder, softgel, suspension or liquid dose contains about 1 mg of (R)-Compound 1. In yet other embodiments, each tablet, caplet, capsule, powder, softgel, suspension or liquid dose contains about 2 mg of (R)-Compound 1.

In certain aspects, the solid or liquid form contains the patient's/human's dose of (R)-Compound 1. In other embodiments, it may be necessary to administer two or more doses of the solid or liquid form, i.e., divided doses, to the patient//human in order to achieve the desired dosage for the patient/human. In some embodiments, a once daily 1 mg tablet containing (R)-Compound 1 is orally administered to the human. In other embodiments, a once daily 2 mg tablet containing (R)-Compound 1 is orally administered to the human.

### Anti-Hypertensive Regimens

In some embodiments, prior to administration of (R)-Compound 1, the human does not respond to one or more stable background anti-hypertensive regimen. A "stable background anti-hypertensive regimen" includes any regimen that lowers the patient's/human's blood pressure. The regimen may include performing one or more therapies such as daily activities or taking one or more antihypertensive agents. In some embodiments, the stable background anti-hypertensive regimen is one or more daily activities. Examples of daily activities that may be used to treat hypertension or primary aldosteronism include, without limitation, healthy eating, lowering salt intake, getting regular physical activity, maintaining a healthy weight, losing weight if advised by a physician, and limiting alcohol consumption. In other embodiments, the stable background anti-hypertensive regimen is an antihypertensive agent. In further embodiments, the hypertension is uncontrolled hypertension and the stable background anti-hypertensive regimen comprises at least two antihypertensive medications. In still other embodiments, the hypertension is treatment-resistant hypertension and the stable background anti-hypertensive regimen comprises at least three antihypertensive medications.

The term "antihypertensive agents" as used herein refers to a medication that lowers a patient's//human's blood pressure. In some embodiments, the antihypertensive agent is a diuretic, loop diuretic, beta-blocker, ACE inhibitor, angiotensin II receptor blocker, calcium channel blocker, alpha blocker, alpha-2 receptor agonist, combined alpha and beta-blocker, central agonists, peripheral adrenergic inhibitor, blood vessel dilator (vasodilator), or combination thereof. In some embodiments, the antihypertensive agent is a diuretic such as a thiazide diuretic, potassium-sparing diuretic, loop diuretic, or a combination diuretic. Examples of thiazide diuretics include chlorthalidone (Hygroton), chlorothiazide (Diuril), hydrochlorothiazide (Esidrix, Hydrodiuril, Microzide), indapamide (Lozol), or metolazone (Mykrox, Zaroxolyn). Examples of potassium-sparing diuretics include amiloride hydrochloride (Midamar), spironolactone (Aldactone), eplerenone (Inspra), or triamterene (Dyrenium). Examples of loop diuretics include furosemide (Lasix) or bumetanide (Bumex). Examples of the combination diuretic include amiloride hydrochloride + hydrochlorothiazide (Moduretic), spironolactone + hydrochlorothiazide (Aldactazide), or triamterene + hydrochlorothiazide (Dyazide, Maxzide). In other embodiments, the antihypertensive agent is a beta-blocker. Examples of beta-blockers include acebutolol (Sectral), atenolol (Tenormin), betaxolol (Kerlone), bisoprolol fumarate (Zebeta), carteolol hydrochloride (Cartrol), metoprolol tartrate (Lopressor), metoprolol succinate (Toprol-XL), nadolol (Corgard), penbutolol sulfate (Levatol), pindolol (Visken), propranolol hydrochloride (Inderal), solotol hydrochloride (Betapace), or timolol maleate (Blocadren). In further embodiments, the antihypertensive agent is a combination beta-blocker/diuretic. An example of the beta-blocker/diuretic combination is hydrochlorothiazide + bisoprolol (Ziac). In yet other embodiments, the antihypertensive agent is an ACE inhibitor. Examples of ACE inhibitors include benazepril hydrochloride (Lotensin), captopril (Capoten), enalapril maleate (Vasotec), fosinopril sodium (Monopril), lisinopril (Prinivel, Zestril), moexipril (Univasc), perindopril (Aceon), quinapril hydrochloride (Accupril), ramipril (Altace), or trandolapril (Mavik). In still further embodiments, the antihypertensive agent is an angiotensin II receptor blocker. Examples of angiotensin II receptor blockers include candesartan (Atacand), eprosartan mesylate (Teveten), irbesartan (Avapro), losartan potassium (Cozaar), telmisartan (Micardis), or valsartan (Diovan). In other embodiments, the antihypertensive agent is a calcium channel blocker. Examples of calcium channel blockers include amlodipine besylate (Norvasc, Lotrel), bepridil (Vasocor), diltiazem hydrochloride (Cardizem CD, Cardizem SR, Dilacor XR, Tiazac), felodipine (Plendil), isradipine (DynaCirc, DynaCirc CR), nicardipine (Cardene SR), nifedipine (Adalat CC, Procardia XL), nisoldipine (Sular), or verapamil hydrochloride (Calan SR, Covera HS, Isoptin SR, Verelan). In further embodiments, the antihypertensive agent is an alpha blocker. Examples of alpha blockers include doxazosin mesylate (Cardura), prazosin hydrochloride (Minipress), or terazosin hydrochloride (Hytrin). In still other embodiments, the antihypertensive agent is an alpha-2 receptor agonist. An example of an alpha-2 receptor agonist is methyldopa. In yet further embodiments, the antihypertensive agent is a combined alpha and beta-blocker. Examples of combined alpha and beta-blockers include carvedilol (Coreg) or labetalol hydrochloride (Normodyne, Trandate). In other embodiments, the antihypertensive agent is a central agonist. Examples of central agonists include alpha methyldopa (Aldomet), clonidine hydrochloride (Catapres), guanabenz acetate (Wytensin), or guanfacine hydrochloride (Tenex). In further embodiments, the antihypertensive agent is a peripheral adrenergic inhibitor. Examples of peripheral adrenergic inhibitors include guanadrel (Hylorel), guanethidine monosulfate (Ismelin), or reserpine (Serpasil). In yet other embodiment, the antihypertensive agent is a blood vessel dilator, *i.e.,* vasodilator. Examples of blood vessel dilators include hydralazine hydrochloride (Apresoline), or minoxidil (Loniten).

In some embodiments, the patient's/human's hypertension does not respond to one or more stable background anti-hypertensive regimen prior to administering (R)-Compound 1. In other embodiments, the patient's/human's hypertension does not respond to two stable background anti-hypertensive regimens prior to administering (R)-Compound 1. In further embodiments, the patient's/human's hypertension does not respond to three stable background anti-hypertensive regimens prior to administering (R)-Compound 1. In other embodiments, the patient's/human's hypertension does not respond to three or more stable background anti-hypertensive regimens prior to administering (R)-Compound 1.

The antihypertensive agent may be administered to the human in combination with (R)-Compound 1. In some embodiments, (R)-Compound 1 is administered to the human in combination with two antihypertensive agents during the treatment period. In other embodiments, (R)-Compound 1 is administered to the human in combination with three antihypertensive agents during the treatment period. In further embodiments, (R)-Compound 1 is administered to the human in combination with at least three antihypertensive agents during the treatment period. In yet other embodiments, (R)-Compound 1 is administered to the human in combination with two antihypertensive agents during the treatment period, wherein one antihypertensive agent is a diuretic. In still further embodiments, (R)-Compound 1 is administered to the human in combination with three antihypertensive agents during the treatment period, wherein one antihypertensive agent is a diuretic. In other embodiments, (R)-Compound 1 is administered to the human in combination with at least three antihypertensive agents during the treatment period, wherein one antihypertensive agent is a diuretic.

Current FDA-approved drug classes for the treatment of hypertension are diuretics, renin-angiotensin-aldosterone system (RAAS) inhibitors, beta-blockers, calcium channel blockers, mineralocorticoid receptor antagonists (MRA), alpha-adrenergic receptor antagonists, central alpha-adrenergic receptor agonists, and direct vasodilators (Table 2). Drugs recommended for initial treatment include diuretics, ACEIs, ARBs, and calcium channel blockers.

| **Table 2: FDA-Approved Drug Classes to Treat hypertension** | |
|---|---|
| **Drug class** | **Mechanism of action** |
| Thiazide diuretics | Inhibition of luminal sodium reabsorption in the distal convoluted tubule of the nephron, thereby promoting natriuresis and diuresis |
| Angiotensin converting enzyme inhibitors | Inhibition of angiotensin converting enzyme resulting in decreased angiotensin II, vasoconstriction, and aldosterone secretion |
| Angiotensin receptor blockers | Selective inhibition of the angiotensin II subtype 1 receptor, thus preventing the vasoconstrictive effect of angiotensin |
| Beta blockers | Selective inhibition of the beta-adrenergic receptor decreases cardiac output, reduces renin release, and modulates the sympathetic nervous system |
| Calcium channel blockers | Inhibition of voltage-gated calcium channels in vascular smooth muscle resulting in peripheral vasodilation |
| Mineralocorticoid receptor antagonists | Inhibition of mineralocorticoid receptors in the late distal tubule and collecting duct of the nephron causing natriuresis and diuresis |
| Alpha-adrenergic receptor antagonists | Selective inhibition of the peripheral alphai-adrenergic receptor in vascular smooth muscle leading to reduced arteriolar resistance and increased venous capacitance |
| Central alpha-adrenergic receptor agonists | Stimulation of the alpha₂-adrenergic receptor in the brainstem, thereby reducing sympathetic outflow from the central nervous system |
| Direct Vasodilators | Direct relaxation of arteriolar smooth muscle |

### Aspects

Aspect 1. A method as described substantially herein.

Aspect 2. A compound that is: 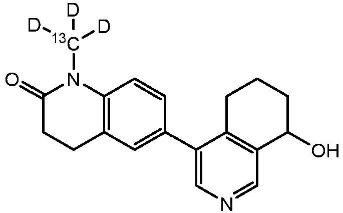 or a pharmaceutically acceptable salt thereof.

Aspect 3. A compound that is: 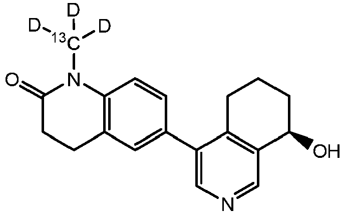 or a pharmaceutically acceptable salt thereof.

Aspect 4. A compound that is: 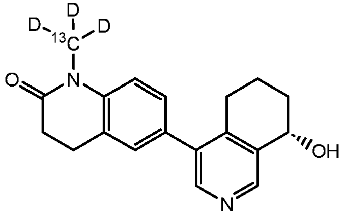 or a pharmaceutically acceptable salt thereof.

Aspect 5. A compound that is: 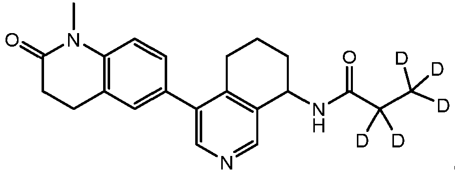 or a pharmaceutically acceptable salt thereof.

Aspect 6. A compound that is: 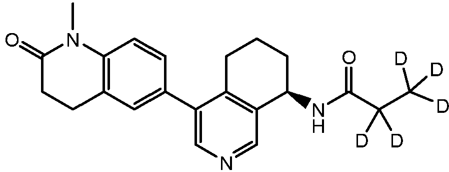 or a pharmaceutically acceptable salt thereof

Aspect 7. A compound that is: 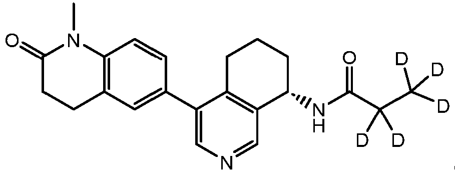 or a pharmaceutically acceptable salt thereof.

### ABBREVIATIONS AND DEFINITION OF TERMS

| **Abbreviation** | **Definition** |
|---|---|
| ABPM | Ambulatory blood pressure monitoring |
| ACE | Angiotensin converting enzyme |
| ACEI or ACEi | Angiotensin converting enzyme inhibitor |
| AE | Adverse event |
| A_{e (0-72)} | Total amount excreted in urine from time 0 to 72 hours after dose |
| AESI | Adverse event of special interest |
| ALT | Alanine Aminotransferase/Transaminase |
| ANCOVA | Analysis of covariance |
| AOBPM | Automated office blood pressure measurement |
| ARB | Angiotensin receptor blocker |
| AST | Aspartate Aminotransferase/Transaminase |
| AUC | Area under the curve |
| AUC₀₋₂₄ | AUC from time 0 to 24 hours post-dose |
| AUC_{0-inf} | AUC from time 0 to infinity |
| AUC₀₋ₜ | AUC until the last quantifiable plasma concentration |
| AUC_{%extrap} | Percent of area under the plasma concentration-time curve extrapolated |
| B | Baxdrostat |
| β-hCG | Human chorionic gonadotropin |
| BMI | Body mass index |
| BP | Blood pressure |
| CI | Confidence interval |
| CKD | Chronic kidney disease |
| CL/F | Apparent plasma clearance |
| CLR or CL_{R} | Renal clearance |
| cm | Centimeter(s) |
| COVID-19 | Coronavirus disease 2019 |
| CV | Coefficient of variation |
| CYP11B1 | 11-β hydroxylase |
| DAE | Discontinuation of IP due to AE |
| DBP | Diastolic blood pressure |
| DBP | Diastolic blood pressure |
| DOT | Direct observed therapy |
| e | Events |
| ECG | Electrocardiogram |
| eGFR | Estimated glomerular filtration rate |
| EOT | End of treatment |
| FAS | Full analysis set |
| FDA | Food and Drug Administration |
| FU | Follow-up |
| GLSM | Geometric least squares mean |
| GM | Geometric mean |
| GMR | Geometric mean ratio |
| h | Hour |
| HbA1c | Glycosylated hemoglobin |
| HBV | Hepatitis B Virus |
| HF | Heart failure |
| HTN | Hypertension |
| IMP | Investigational Medicinal Product |
| IRT | Interactive Response Technology |
| kg | Kilogram |
| K⁺ | Potassium |
| Ki | Inhibitory constant |
| L | Liter |
| λ_{z} | Terminal elimination rate constant |
| LC-MS/MS | Liquid chromatography tandem mass spectrometry |
| LS | Least squares |
| M | Metformin |
| m | Meter |
| MACE | Major adverse cardiovascular events |
| MAD | Multiple ascending dose |
| MATE | Multidrug efflux transporter |
| MedDRA | Medical Dictionary for Regulatory Activities |
| mEq | Milliequivalent |
| mITT | Modified intent to treat |
| mL | Milliliter |
| mmHg | Millimeters of mercury |
| mmol | Millimole |
| µg | Microgram |
| MMRM | Mixed model for repeated measures |
| MRA | Mineralocorticoid receptor antagonist |
| NA | Not available |
| N/A | Not applicable |
| NIMP | Non-investigational medicinal product |
| NSAID | Nonsteroidal anti-inflammatory drug |
| ng | Nanogram |
| nM | Nanomolar |
| PA | Primary aldosteronism |
| PD | Pharmacodynamics |
| PK | Pharmacokinetics |
| PRA | Plasma renin activity |
| QD | Once daily |
| R | Randomization |
| QD | Quaque die (one per day) |
| QTcF | QT interval corrected using Fridericia's formula |
| RAAS | Renin-angiotensin-aldosterone system |
| REML | Restricted maximum likelihood estimation |
| RTSM | Randomization and Trial Supply Management |
| RWD | Randomized withdrawal |
| rHTN | Treatment-resistant hypertension |
| SAD | Single ascending dose |
| SAE | Serious adverse events |
| SBP | Systolic blood pressure |
| SD | Standard deviation |
| SE | Standard error |
| siSBP | Sitting SBP |
| siDBP | Sitting DBP |
| SOC | Standard of Care |
| t_{½} | Terminal phase elimination half-life |
| T2DM | Type 2 diabetes mellitus |
| TBL | Total Bilirubin |
| TEAE | Treatment-emergent adverse events |
| Tmax | Time to maximum concentration |
| UACR | Urinary albumin to creatinine ratio |
| ULN | Upper limit of normal |
| uCi | Microcurie |
| uHTN | Uncontrolled hypertension |
| V/F | Volume of distribution (V/F) |

The following Examples are provided to illustrate some concepts described within this disclosure. While the Example is considered to provide specific individual embodiments of formulations, methods of preparation and use, the Examples should not be considered to limit the more general embodiments described herein. In the following Examples, efforts have been made to ensure accuracy with respect to numbers used (e.g., amounts, temperature, etc.) but some experimental error and deviation should be accounted for.

### Example 1: A single ascending dose study: safety, tolerability, pharmacokinetics, and pharmacodynamics of baxdrostat in healthy male volunteers

Eighty-eight healthy male volunteers completed the first-in-human study of baxdrostat. In part 1 of the trial, subjects received a single oral dose of baxdrostat ranging from 1 to 360 mg in order to evaluate safety, tolerability, PK, and pharmacodynamics (PD). Part 2 evaluated the PK of a single 3 mg intravenous dose of baxdrostat. Baxdrostat significantly reduced aldosterone levels in a dose-dependent manner under fasted, low salt, and normal salt diet conditions. Baxdrostat had no meaningful effect on cortisol levels with an adrenocorticotropin hormone challenge, demonstrating selectivity for aldosterone synthase inhibition. Plasma levels of baxdrostat increased in a dose-proportional manner over the entire range of doses tested with a mean half-life of 29 hours, supportive of once-daily dosing. There were no deaths, serious adverse events, or dose-limiting adverse events. Side effects were mild and included headache, nasopharyngitis, and diarrhea.

### Example 2: A randomized, double-blind study: safety, pharmacokinetics, and pharmacodynamics of baxdrostat following multiple ascending doses in healthy subjects

This randomized, double-blind, placebo-controlled phase 1 study evaluated the safety, pharmacokinetics, and pharmacodynamics of multiple ascending doses of baxdrostat in healthy volunteers. Fifty-six subjects were randomized to receive oral baxdrostat (0.5, 1.5, 2.5, or 5.0 mg) or placebo once daily for ten days and were placed on either a low salt or normal salt diet for the duration of the study. In particular, subjects were randomized into 5 cohorts to receive baxdrostat or placebo once daily for 10 days. Cohorts 1 and 2 were placed on a low salt diet to stimulate aldosterone production and were administered 2.5 or 5.0 mg oral baxdrostat, respectively. Cohorts 1 and 2 also underwent an adrenocorticotropic hormone (ACTH) challenge to increase aldosterone and cortisol levels to evaluate the specificity of baxdrostat for targeting aldosterone synthase. Cohorts 3, 4, and 5 were placed on a normal salt diet and were administered 1.5, 2.5, or 0.5 mg oral baxdrostat, respectively. Blood samples were collected prior to and after dosing on days 1 and 10 for measurement of plasma baxdrostat concentrations to characterize single-dose and steady-state pharmacokinetics. Pharmacodynamic measurements included plasma aldosterone, cortisol, and electrolytes. Safety assessments included physical examination, electrocardiograms, orthostatic vital signs, and clinical laboratory evaluations.

Fifty-four subjects completed the study. There were no deaths or serious adverse events, and all treatment-emergent adverse events in subjects receiving baxdrostat were mild in severity (e.g., headache, dizziness, and postural dizziness). Baxdrostat was rapidly absorbed. Plasma levels of baxdrostat increased proportionally with ascending doses, with peak concentrations observed within 4 hours after dosing and a mean half-life of 26 to 31 hours showing that the concentration of plasma baxdrostat declined in an apparent biphasic manner. A dose-dependent reduction of plasma aldosterone occurred with baxdrostat doses ≥1.5 mg, regardless of diet. Decreases in plasma aldosterone were sustained, with levels reduced by approximately 51-73% on Day 10. Baxdrostat had no meaningful impact on plasma cortisol and resulted in mild dose-dependent decreases in plasma sodium levels and increases in potassium levels, consistent with the observed reduction in aldosterone. Baxdrostat was safe and well-tolerated with a half-life that supports once-daily dosing. The dose-dependent reduction in plasma aldosterone and lack of effect on cortisol demonstrate the selective blockade of aldosterone synthase.

Oral administration of baxdrostat was safe and well tolerated in all subjects and resulted in dose-dependent increases in plasma baxdrostat with a half-life that supports once-daily dosing. The dose-dependent decrease in plasma aldosterone and lack of effect on cortisol demonstrate the selective blockade of aldosterone synthase.

### Example 3: A randomized, open label, crossover study: relative bioavailability of tablet formulation of baxdrostat as compared to oral solution; assess the effect of food on the tablet formulation of baxdrostat in healthy subjects

The relative bioavailability of a tablet formulation of baxdrostat was compared to the oral solution of baxdrostat that was administered in the SAD (Example 1) and MAD (Example 2) studies. A single 5mg dose of each formulation in fourteen healthy volunteers established that plasma levels of baxdrostat were equivalent whether administered using the oral solution or tablet. This study also assessed the effect of a standardized high-fat meal on the PK of baxdrostat. There was no noteworthy effect of the high-fat meal on the plasma levels of baxdrostat; there was a small effect on the rate of absorption, indicated by a shift in time to maximum concentration (Tₘₐₓ) of approximately 3 hours postdose in the fasted state to approximately 4 hours postdose in the fed state.

### Example 4: A phase 1, open-label, single dose, parallel group study: pharmacokinetics of baxdrostat in subjects with varying degrees of renal function

### A. Methods

### (i) Subjects

Subjects were included in the study if they were between the ages of 18 and 80 years, and in stable health based on medical and psychiatric history, physical examination, ECG, vital signs (seated and orthostatic), and routine laboratory tests (blood chemistry, hematology, coagulation, and urinalysis). For renally impaired subjects, their renal status must have been stable for a minimum of 3 months prior to screening. In addition, subjects must have been non-smokers or smoked <10 cigarettes/day and had a BMI ≥18 and ≤40 kg/m².

Subjects were excluded if they had:
1) a personal or family history of long QT syndrome, complex ventricular arrythmias, or family history of sudden death,
2) a personal history of, or current, clinically significant arrhythmias,
3) prolonged QTcF (>450 msec for males or >470 for females),
4) seated systolic blood pressure (BP) >160 mm Hg and/or diastolic BP >100 mm Hg, or systolic BP <90 mm HG and/or diastolic BP <50 mm Hg,
5) resting heart rate higher than 100 beats per minute (bpm) or lower than 50 bpm,
6) postural tachycardia or orthostatic hypotension (i.e., a fall in systolic BP ≥20 mmHg or diastolic BP ≥10 mmHg upon standing from a seated position),
7) clinically significant abnormal serum potassium greater than the ULN of the reference range,
8) clinically significant abnormal serum sodium less than the lower limit of normal of the reference range,
9) aspartate aminotransferase or alanine aminotransferase values greater than 1.5 times the ULN,
10) total bilirubin greater than 2 times the ULN (unless due to Gilbert's syndrome),
11) history of porphyria, myopathy, or active liver disease,
12) current treatment with weight loss medication or prior weight loss surgery, or
13) use of strong inducers of CYP3A4 (e.g., apalutamide, carbamazepine, enzalutamide, mitotane, phenytoin, rifampin, St. John's wort) within 28 days prior to the dose of study drug or corticosteroids within 3 months prior to study drug dosing.

### (ii) Study Design

This phase 1, open-label, single-dose, parallel-group study was designed in accordance with the FDA guidance for the design and analysis of studies that assess the influence of impaired renal function on PK. Subjects were enrolled into three renal function groups defined based on eGFR calculated using the Chronic Kidney Disease Epidemiology Collaboration equation as follows:
- Control (normal renal function or mild renal impairment: eGFR ≥60mL/min)
- Moderate to severe renal impairment (eGFR 15 to 59 mL/min)
- Kidney failure (eGFR <15mL/min, including subjects on dialysis)

All subjects received a single 10 mg dose of baxdrostat in an open-label manner. Each subject had a screening period of up to 28 days, followed by admission to the clinical site (Day -1) and baxdrostat administration (Day 1). All subjects were required to fast for a minimum of 10 hours prior to baxdrostat administration. They were then dosed at 8:00 AM (±2 hours) with two 5 mg tablets administered with approximately 240 mL of water. Subjects continued fasting for a minimum of 4 hours after dosing. No other medications were permitted within 2 hours prior to dosing and for 2 hours after dosing with baxdrostat. Subjects on dialysis received the study drug on a non-dialysis day. Blood and urine sample collection for PK analysis continued for 7 days and was proceeded by a follow-up phone call 3 ± 1 days after clinic discharge.

### (iii) Analytical Methods

Plasma and urine samples were analyzed to measure concentrations of baxdrostat and its primary metabolite (baxdrostat-M) using a validated LC-MS/MS method. "Baxdrostat-M" as used herein refers a compound having the following structure:

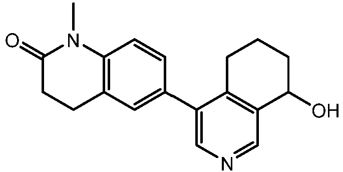

### (iv) Pharmacokinetic Analyses

| **Table 3: Baseline Demographics and Clinical Characteristics** | | | | |
|---|---|---|---|---|
| **Demographic characteristic** | | **Control eGFR ≥60 mL/min (n=10)** | **Moderate to severe renal impairment eGFR 15-59 mL/min (n=11)** | **Kidney failure eGFR <15 mL/min (n=12)** |
| Age (vears), mean (SD) | | 58.6 (8.9) | 71.5 (5.5) | 54.8 (11.0) |
| Race, n (%) | | | | |
| | White | 5 (50.0) | 10 (90.9) | 5 (41.7) |
| | Black or African American | 5 (50.0) | 1 (9.1) | 7 (58.3) |
| Ethnicity, n (%) | | | | |
| | Hispanic or Latino | 0 (0.0) | 1 (9.1) | 2 (16.7) |
| | Not Hispanic or Latino | 10 (100.0) | 10 (90.9) | 10 (83.3) |
| Sex, n (%) | | | | |
| | Female | 1 (10.0) | 4 (36.4) | 3 (25.0) |
| | Male | 9 (90.0) | 7 (63.6) | 9 (75.0) |
| Height (cm), mean (SD) | | 175.6 (5.1) | 170.5 (10.3) | 173.6 (8.2) |
| Body weight (kg), mean (SD) | | 90.8 (11.7) | 93.0 (15.4) | 85.8 (18.1) |
| BMI (kg/m²), mean (SD) | | 29.5 (3.7) | 32.0 (4.8) | 28.4 (5.6) |
| Body surface area (m²), mean (SD) | | 2.1 (0.2) | 2.1 (0.2) | 2.0 (0.2) |
| eGFR (mL/min), mean (SD) | | 91.9 (22.2) | 38.3 (12.0) | 16.2 (14.6) |

Blood and urine samples were collected prior to and after dosing for measurement of plasma and urine baxdrostat and baxdrostat-M concentrations to characterize maximum observed plasma concentration (Cmax), time to Cmax (Tₘₐₓ), AUC₀₋ₗₐₛₜ, and AUC_{0-inf} for baxdrostat and baxdrostat-M. The t_{1/2}, CL/F, and V/F were calculated for plasma baxdrostat only. Urine PK parameters included the cumulative amount of baxdrostat and baxdrostat-M excreted in urine (Aₑ), renal clearance (CL_{R}) of baxdrostat and baxdrostat-M, and fraction of dose excreted renally (Fₑ) for baxdrostat.

### (v) Safety Analyses

Safety was assessed throughout the study based on adverse events, physical examinations, ECG, seated and orthostatic vital signs, weight measurements, and clinical laboratory evaluations

### B. Results

### (i) Subjects

Baseline demographics and clinical characteristics were generally well matched among the renal function groups and are presented in Table 3.

Subjects were predominantly white (60.6%) males (75.8%) with an average age of 62 years and a mean BMI of 30 kg/m². The continuum of eGFR values were well represented by all subjects. All subjects in the kidney failure group were on hemodialysis.

### (ii) Pharmacokinetics

Plasma PK parameters of baxdrostat are presented in Table 4.

| **Table 4: Summary of Plasma and Urine PK Parameters of Baxdrostat** | | | |
|---|---|---|---|
| **PK Parameter** | **Control eGFR ≥60 mL/min (n=10)** | **Moderate to severe renal impairment eGFR 15-59 mL/min (n=11)** | **Kidney failure eGFR <15 mL/min (n=12)** |
| Plasma | | | |
| Cₘₐₓ (ng/mL), mean (SD) | 108.02 (25.5) | 110.06 (23.2) | 96.05 (26.8) |
| Tₘₐₓ (h), mean (SD) | 2.10 (1.6) | 1.82 (1.0) | 1.63 (0.8) |
| AUC₀₋ₗₐₛₜ (h·ng/mL), mean (SD) | 3377.10 (917.1) | 4012.53 (1192.5) | 2567.48 (1362.9) |
| AUC_{0-inf} (h·ng/mL), mean (SD) | 3530.92 (1032.8) | 4340.95 (1486.6) | 2661.74 (1461.8) |
| t_{1/2} (h), mean (SD) | 35.63 (12.1) | 42.14 (16.5) | 29.70 (10.5) |
| CL/F (L/h), mean (SD) | 3.06 (0.9) | 2.60 (1.0) | 5.07 (2.9) |
| Vz/F (L), mean (SD) | 146.41 (31.4) | 141.84 (30.9) | 200.34 (134.8) |

| Urine | | | |
|---|---|---|---|
| Fₑ (%), mean (CV%) | 12.53 (38.1) | 11.81 (26.0) | 0.91 (60.7)* |

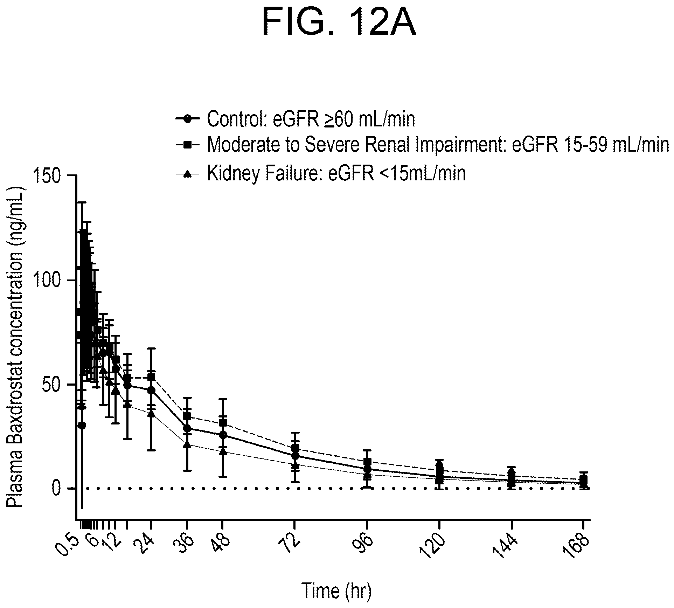

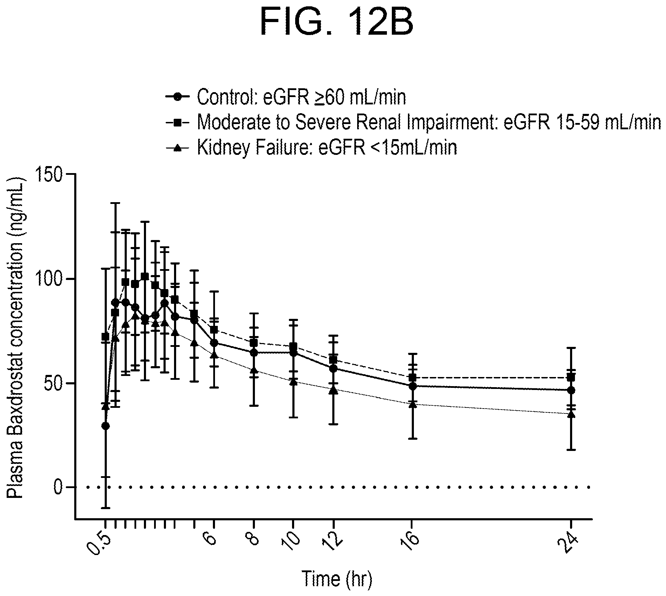

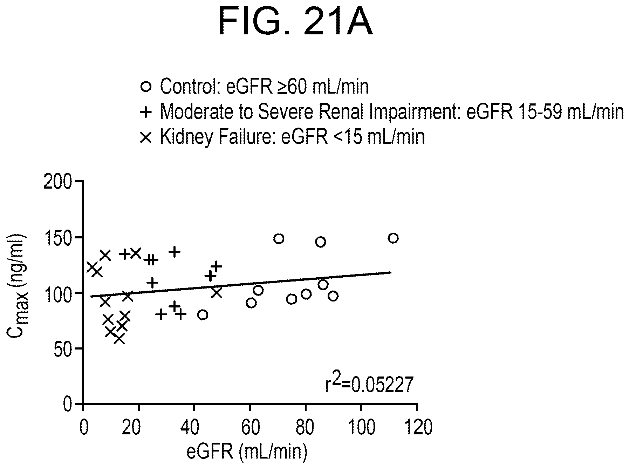

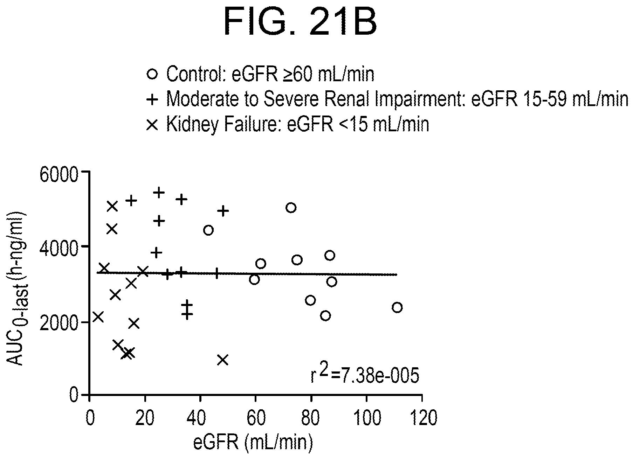

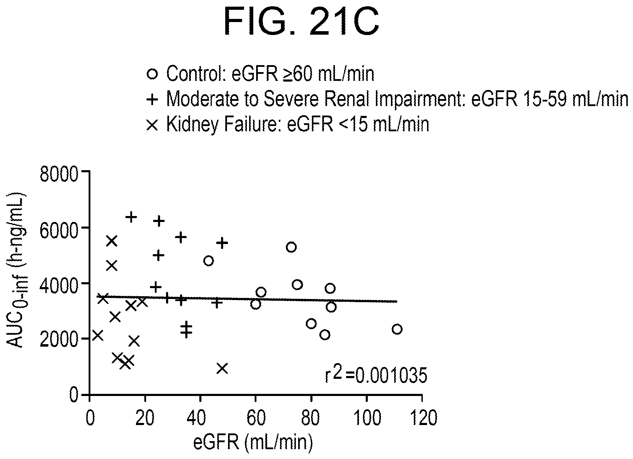

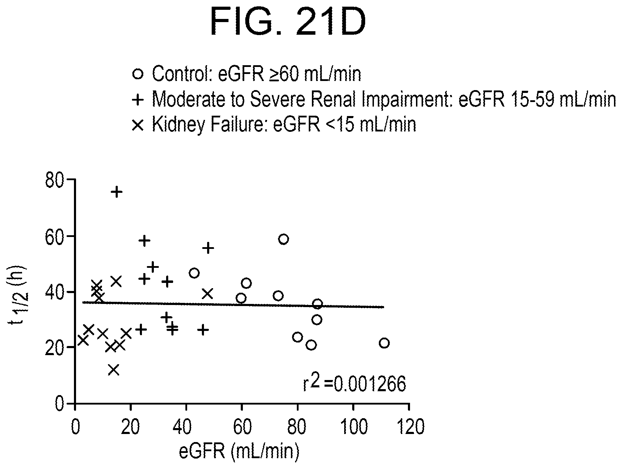

The absorption of baxdrostat was similar between renal function groups. It was rapidly absorbed with a median Tₘₐₓ observed within 1.5 hours of dosing for all groups (FIGs. 12A and 12Band an average half-life ranging from 30 to 42 hours. Pairwise comparisons of moderate to severe renal impairment versus control and kidney failure versus control showed that renal impairment had no meaningful effect on Cₘₐₓ, AUC₍₀₋ₗₐₛₜ₎, AUC_{(0-inf)}, half-life, apparent plasma clearance, or renal clearance. To understand the relationship between selected PK parameters and eGFR as a continuous variable, we analyzed Cₘₐₓ, AUC₀₋ₗₐₛₜ, and AUC_{0-inf} versus baseline eGFR for each subject. There was no indication of a strong linear or nonlinear relationship. See, FIGs. 21A-21D.

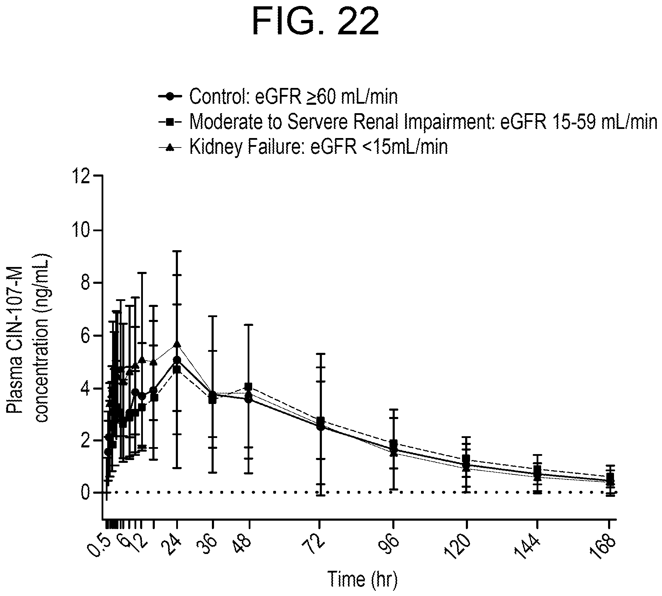

The plasma baxdrostat-M concentration-time curves for the moderate to severe renal impairment and kidney failure groups were qualitatively similar to the control group. See, FIG. 22. The Tₘₐₓ for baxdrostat-M was observed approximately 22 hours after dosing for all renal function groups.

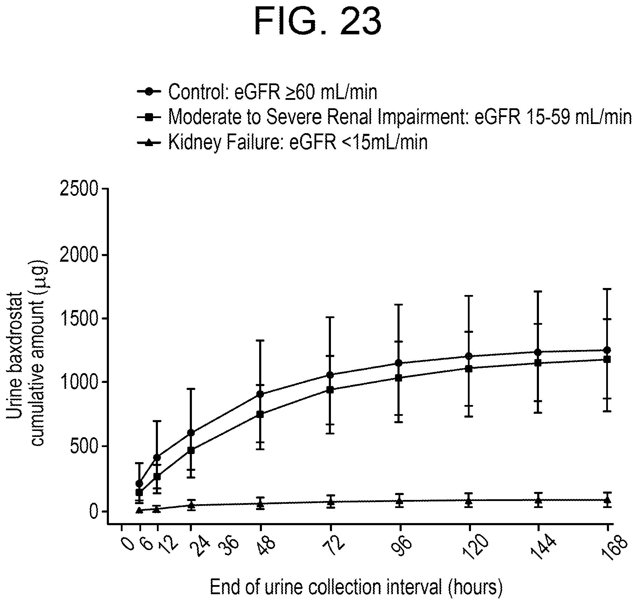

Urine PK parameters are also presented in Table 4. Approximately 12% of baxdrostat was recovered unchanged in the urine for the control and moderate to severe renal impairment groups. Inadequate urine production in the kidney failure group resulted in minimal renal excretion of baxdrostat in these subjects, as expected. See, FIG. 23.

### (iii) Safety

There were no deaths and only one mild drug-related treatment-emergent adverse event (diarrhea) occurred (Table 5).

| **Table 5: Treatment-emergent adverse events** | | | | |
|---|---|---|---|---|
| **Adverse events, n (%)** | **Control eGFR ≥60 mL/min (n=10)** | **Moderate to severe renal impairment eGFR 15-59 mL/min (n=11)** | **Kidney failure eGFR <15 mL/min (n=12)** | **Total (n=33)** |
| Subjects with any TEAEs | 1 (10.0) | 0 (0.0) | 1 (8.3) | 2 (6.1) |
| Diarrhea | 1 (10.0) | 0 (0.0) | 0 (0.0) | 1 (3.0) |
| **Metabolic encephalopathy*** | 0 (0.0) | 0 (0.0) | 1 (8.3) | 1 (3.0) |
| **Tremor*** | 0 (0.0) | 0 (0.0) | 1 (8.3) | 1 (3.0) |
| A TEAE was defined as an AE that occurred after the first dose of study drug. | | | | |
| *Considered severe and unrelated to the study drug. | | | | |

One subject in the kidney failure group experienced two TEAEs of tremor and metabolic encephalopathy that were determined to be unrelated to the study drug. The metabolic encephalopathy was considered severe and secondary to the concomitant medications of gabapentin, primidone, and baclofen and by study Day 11, the metabolic encephalopathy resolved, and the patient was considered to be recovered. There were no clinically meaningful changes in laboratory values, vital signs, physical examinations, or ECGs (data not shown).

### C. Discussion

Older MR antagonist, spironolactone and eplerenone, have been shown to induce changes in biomarkers associated with an improvement in the progression of CKD, presumably due to their anti-inflammatory and antifibrotic effects; however, a major limitation of using these drugs to treat CKD has been a high incidence of hyperkalemia. Finererone, a newer non-steroidal MR antagonist was recently approved for use in CKD patients to slow the progression of CKD despite a higher incidence of hyperkalemia. The effects of finerenone on blood pressure, however, were quite modest. The results of phase 1 studies of baxdrostat in healthy volunteers indicated the drug had minimal effect on serum potassium in individuals with normal renal function. Thus, exploring the utility of baxdrostat in individuals with CKD as both an anti-hypertensive agent as well as a potential therapy for the slowing of renal disease progressions appears warranted. To conduct such studies safely, it was important to first determine if the presence of impaired renal function would require a different dosing regimen of baxdrostat due to alterations in the pharmacokinetic disposition of the drug.

The results presented in this report indicate that renal impairment did not have a meaningful impact on any of the PK parameters measured. Plasma concentration-time curves and the systemic exposures to baxdrostat were as expected based on prior studies in healthy subjects. The half-life of baxdrostat was also comparable to previous studies. In addition, there were no trends in the PK parameters across the eGFR continuum and baxdrostat was safe and well-tolerated in patients with varying degrees of renal function.

Renal disease can alter drug clearance, resulting in increased exposure and potentially greater drug effects or toxicity. Therefore, it is common for dose adjustment to be necessary for patients with impaired kidney function. Previous observations from our single ascending dose study indicated that baxdrostat is primarily metabolized by the liver, consistent with the current finding that renal function did not significantly impact the PK or safety profile of baxdrostat.

As this study was only meant to evaluate PK in renally impaired subjects, additional longer-term studies are needed to fully evaluate the safety and efficacy of baxdrostat in the intended patient population (e.g., those with hypertension, primary aldosteronism, or CKD) who are also renally impaired. Additionally, this study did not include subjects in the kidney failure group that were not on dialysis.

### D. Conclusions

Our results demonstrate that a single dose of baxdrostat was safe and well-tolerated in subjects with varying degrees of renal function, including those with kidney failure and that renal impairment did not significantly impact systemic exposure or clearance of baxdrostat. These findings suggest that dose adjustment of baxdrostat due to altered PK properties in patients with impaired renal function does not appear necessary, even in patients with end-stage renal disease.

### E. Summary

This phase 1 open-label study assessed the safety and pharmacokinetics (PK) of a single oral dose of baxdrostat in subjects with varying degrees of renal function. Thirty-three subjects enrolled into renal function groups based on estimated glomerular filtration rate (eGFR). The groups were control (eGFR ≥60 mL/min), moderate to severe renal impairment (eGFR 15 to 59 mL/min), and kidney failure (eGFR <15 mL/min, including subjects on dialysis). Control subjects were matched by age, gender, race, body mass index (BMI) and smoking status. A single 10 mg baxdrostat dose was given, followed by 7 days of blood and urine sampling for PK analysis. Safety was assessed based on adverse events, clinical laboratory evaluations, vital signs, ECGs, physical examinations, and weight measurements.

Thirty-two subjects completed the study. There were no deaths, and only one mild drug-related adverse event (diarrhea) occurred. There were no clinically meaningful changes in laboratory values, vital signs, physical examinations, or electrocardiograms (ECG). The plasma concentration-time curves of baxdrostat were qualitatively similar in all groups. See, FIG. 12. The urine PK parameters in the moderate to severe renal impairment group were similar to control (12% excreted); inadequate urine production in the kidney failure group resulted in an expected negligible excretion of baxdrostat.

A single dose of baxdrostat was well tolerated in all subjects, including those with kidney failure. Renal impairment did not significantly impact systemic exposure or clearance of baxdrostat, suggesting that dose adjustment due to PK differences in these patients is unnecessary, even in cases of advanced kidney disease.

### Example 5: A phase 1, open-label, single dose, parallel group study: pharmacokinetics of baxdrostat in subjects with varying degrees of hepatic function

### Methods

Subjects with moderate hepatic impairment (Child-Pugh Category B, n=10) and healthy controls (n=10) were each given a single 10 mg oral dose of baxdrostat. Subjects were matched by age, sex, body mass index, race, and smoking status. Serial blood and urine samples were collected to determine plasma and urine concentrations of baxdrostat for 7 days postdose. Safety was assessed based on adverse events, clinical laboratory evaluations, vital signs, electrocardiography, and physical examinations.

### Results

All 20 subjects completed the study. There were no deaths, serious treatment-emergent adverse events (TEAE), or study discontinuations. One TEAE of headache with moderate severity in the normal hepatic function group was deemed related to baxdrostat. One TEAE of hyperkalemia and one of hypoglycemia occurred in the moderate hepatic impairment group; both were deemed unrelated to baxdrostatNo clinically significant changes after baxdrostat dosing were noted for clinical laboratory evaluations, vital signs, electrocardiograms, or physical examinations in both groups. Plasma (FIG. 12A and 12B) and urine pharmacokinetic parameters of baxdrostat were similar between the two hepatic function groups as demonstrated in Table 6.

| **Table 6: Summary of Baxdrostat Pharmacokinetic Parameters** | | | |
|---|---|---|---|
| | **Normal Hepatic Function (n=10)** | **Moderate Hepatic Impairment (n=10)** | **Ratio of GLSM (90% CI)** |
| **Cₘₐₓ (ng/mL)** | 104 (21.8) | 119 (19.3) | 1.1 (0.97, 1.32) |
| **Tₘₐₓ* (hr)** | 2.55 (0.99) | 2.15 (1.11) | |
| **AUC_{(0-inf)} (hr·ng/mL)** | 3780 (24.7) | 3570 (30.1) | 0.95 (0.73, 1.23) |
| **CL/F (L/hr)** | 2.64 (24.7) | 2.80 (30.1) | |
| **CL_{R} (L/hr)** | 0.361 (57.6) | 0.299 (55.9) | |
| Results are geometric mean (% coefficient of variation) unless otherwise indicated. | | | |
| * Arithmetic mean (SD). Abbreviations: Cₘₐₓ; maximum observed plasma concentration; Tₘₐₓ, time to maximum observed plasma concentration. | | | |

In summary, approximately ten subjects were enrolled into each of 2 hepatic function groups based on Child-Pugh classification as either moderately impaired (Category B) or healthy control subjects. Control subjects were selected to be comparable to impaired subjects in terms of age, gender, race, BMI and smoking status. No adjustment in baxdrostat dose is necessary in subjects with moderate hepatic impairment.

### Example 6: A randomized, open label, two-period, crossover study: the effect of baxdrostat on the pharmacokinetics metformin in healthy subjects

Due to the high comorbidity of type 2 diabetes mellitus (T2DM) and hypertension, many patients will require concomitant treatment with the common T2DM drug metformin and antihypertensives. To evaluate the potential drug-drug interaction of baxdrostat and metformin, 27 healthy volunteers were given either 1000 mg metformin alone or 1000 mg metformin following a 10 mg dose of baxdrostat. There was no shift or change in shape of the PK curves for plasma metformin in the presence or absence of baxdrostat. Baxdrostat did not significantly affect the renal clearance of metformin. There were no deaths, serious adverse events (SAEs), discontinuations due to treatment-emergent adverse events (TEAEs), or noteworthy increases in adverse events (AEs) with either treatment. All reported TEAEs were mild in severity; no subjects experienced moderate or severe TEAEs. The most common TEAEs were gastrointestinal disorders. No clinically meaningful changes were observed in physical examinations, vital signs, ECGs (including no QT prolongation), or clinical laboratory results. Metformin and baxdrostat were safe and well tolerated when coadministered.

### Example 6A: Randomized, Open-Label, Crossover Study Evaluating the Effect of the Aldosterone Synthase Inhibitor Baxdrostat on the Pharmacokinetics of Metformin in Healthy Human Subjects

### A. Subjects

To meet the inclusion criteria of this study, men and women between the ages of 18 and 55 years must have been in good health based on medical and psychiatric history, physical examination, ECG, vital signs, and routine laboratory tests (serum chemistry, hematology, and urinalysis). Subjects had a BMI ≥18 and ≤30 kg/m², adequate renal function defined as estimated glomerular filtration rate ≥85 mL/min/1.73 m², and no recent nicotine use.

Subjects were excluded from participation in this study if they had a personal or family history of long QT syndrome, complex ventricular arrythmias, current or past history of clinically significant arrhythmias, family history of sudden death, prolonged QTcF (>450 msec), seated BP >140/90 or <90/50 mm Hg, a resting heart rate >100 or <50 beats per minute, sinus node dysfunction, clinically significant heart block, postural tachycardia, or orthostatic hypotension. Other exclusionary criteria included any clinical laboratory values meaningfully outside of normal limits; a history of clinically significant or multiple drug allergies; any prior episode of lactic acidosis; a radiologic scan with contrast within 14 days prior to the first dose of study drug; or a positive test for HIV antibody, hepatitis C virus antibody, hepatitis B surface antigen, or SARS-CoV-2 RNA. Subjects were excluded if they reported any recent illicit drug use; drank more than 14 alcoholic beverages a week; used any prescription medications including topicals, herbal/dietary supplements, nutraceuticals, or over-the-counter medications (other than occasional use of acetaminophen or nonsteroidal anti-inflammatory drugs) within the longer of 14 days or 5 half-lives before the first dose of study drug and throughout the inpatient and discharge periods.

### B. Study Design

This study was designed as a phase 1, randomized, open-label, 2-period, crossover trial. Each subject participated in a screening period of up to 26 days followed by 2 inpatient treatment periods and a follow-up phone call. Subjects were randomized in a 1:1 ratio to treatment sequence AB or sequence BA for the 2 inpatient treatment periods. Treatment A was a single 1000-mg dose of metformin, and treatment B was a single 10-mg dose of baxdrostat followed by a single 1000-mg dose of metformin 2 hours later.

There was a minimum 10-day washout period between administration of the 2 treatments. Blood and urine sample collection for PK analysis began prior to dosing in each period and continued for 3 days after dosing in each period. Samples were collected 2, 1.5, 1, 0.5, and 0 hours before dosing and 0.5, 1, 1.5, 2, 2.5, 3, 3.5, 4, 5, 6, 8, 10, 12, 14, 16, 22, 24, 36, 48, and 72 hours after dosing. Subjects received a follow-up phone call 3±1 days after discharge from the clinic in the second dosing period.

### (i) Bioanalytical Methods

Plasma and urine samples were analyzed to measure concentrations of metformin, baxdrostat, and its primary metabolite, baxdrostat-M, using validated LC-MS/MS methods.

The quantifiable range for metformin in plasma was 0.5 to 500 ng/mL, using metformin-d₆ as the internal standard. Plasma samples were extracted by protein precipitation with methanol, followed by analysis by LC-MS/MS with electrospray ionization in positive mode (ESI(+)) and MRM. Reversed-phase chromatographic separation with a mobile phase gradient of 15% to 90% was utilized, and total run time was approximately 5 minutes. Precursor to product ion transitions for metformin and internal standard were 130.1 to 71.1 and 135.4 to 77.0, respectively. Similarly, the quantifiable ranges for metformin in urine were 1.00 to 500 ng/mL (low range) and 2.00 to 1250 ng/mL (high range), with similar extraction and LC-MS/MS conditions. Between-day precision (%CV) values for metformin in plasma and urine were within 5% and 10%, respectively.

The quantifiable ranges for both baxdrostat and its metabolite baxdrostat-M in plasma were 0.05 to 50 ng/mL (low range) and 5.00 to 2500 ng/mL (high range), using baxdrostat-d₅ and baxdrostat-M-d₃, respectively, as the internal standards.

"Baxdrostat-ds" as used herein refers to a compound having the following structure:

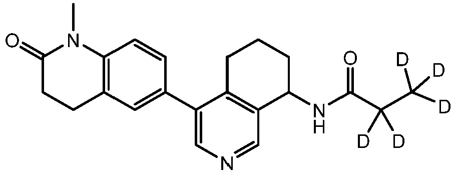

"Baxdrostat-M-d₃" as used herein refers to a compound having the following structure:

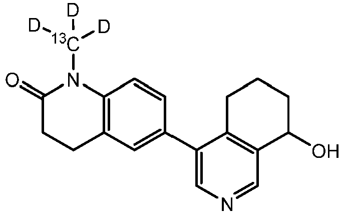

Plasma samples were extracted by protein precipitation with methanol, followed by analysis by LC-MS/MS with ESI(+) and MRM. Reversed-phase chromatographic separation with a mobile phase gradient of 20% to 90% was utilized, and total run time was approximately 5 minutes. Precursors to product transition for baxdrostat and its internal standard were 364.2 to 291.2 and 369.2 to 291.2, respectively. The transitions for baxdrostat-M and its internal standard were 309.2 to 291.2 and 313.2 to 295.2, respectively. Between-day precision (%CV) values for baxdrostat and baxdrostat-M in plasma were within 3% and 4%, respectively.

### (ii) PK Analyses

PK parameters were calculated using noncompartmental methods with SAS software, version 9.4 (SAS Institute). Plasma PK parameters calculated for metformin, baxdrostat, and baxdrostat-M included maximum observed plasma concentration (Cₘₐₓ); Tₘₐₓ; AUC₀₋₂₄; AUC₀₋ₜ; AUC_{0-inf}; AUC_{0-inf} extrapolated; λ_{z}; and t_{1/2}. Plasma AUC₀₋₇₂ was assessed only for baxdrostat and baxdrostat-M. Cₘₐₓ, AUC_{0-inf}, and AUC₀₋ₜ were the 3 plasma PK parameters analyzed statistically to detect any interaction between baxdrostat and metformin. Urine PK parameters included Aₑ, renal clearance (calculated as Aₑ/AUC), and the fraction of dose excreted renally.

### (iii) Safety Analyses

Safety assessments included monitoring of AEs, physical examinations, ECGs, orthostatic vital signs, and clinical laboratory evaluations.

### C. Results

### (i) Subjects

Baseline demographics and clinical characteristics are presented in Table 7.

| **Table 7: Baseline Demographics and Clinical Characteristics** | | | |
|---|---|---|---|
| **Demographic characteristic** | | **Sequence AB (n=14)** | **Sequence BA (n=13)** |
| Age, y, mean (SD) | | 33.0 (6.2) | 40.7 (9.5) |
| Race, No. (%) | | | |
| | White | 6 (42.9) | 9 (69.2) |
| | Black or African American | 8 (57.1) | 4 (30.8) |
| | Asian | 0(0) | 0 (0) |
| | Other | 0(0) | 0 (0) |
| Ethnicity, No. (%) | | | |
| | Hispanic or Latino | 1 (7.1) | 3 (23.1) |
| | Not Hispanic or Latino | 13 (92.9) | 10 (76.9) |
| Sex, No. (%) | | | |
| | Female | 6 (42.9) | 2 (15.4) |
| | Male | 8 (57.1) | 11 (84.6) |
| Height, cm, mean (SD) | | 170.3 (7.5) | 174.7 (10.3) |
| Body weight, kg, mean (SD) | | 73.8 (10.0) | 78.8 (10.2) |
| BMI, kg/m², mean (SD) | | 25.4 (2.5) | 25.8 (2.6) |
| In sequence AB, subjects received metformin alone on their first visit followed by metformin and baxdrostat on their second visit. Subjects in Sequence BA received metformin and baxdrostat on their first visit followed by metformin alone on their second visit. | | | |

Twenty-seven subjects were randomized into 2 cohorts who received study drugs in treatment sequence AB or BA. Most study participants were white (56%) men (70%) who were not Hispanic or Latino. The average age was 37 years and mean BMI was 25.6 kg/m². Demographic and baseline characteristics were generally well matched in sex, race, and ethnicity between both treatment sequences.

### (ii) Pharmacokinetics

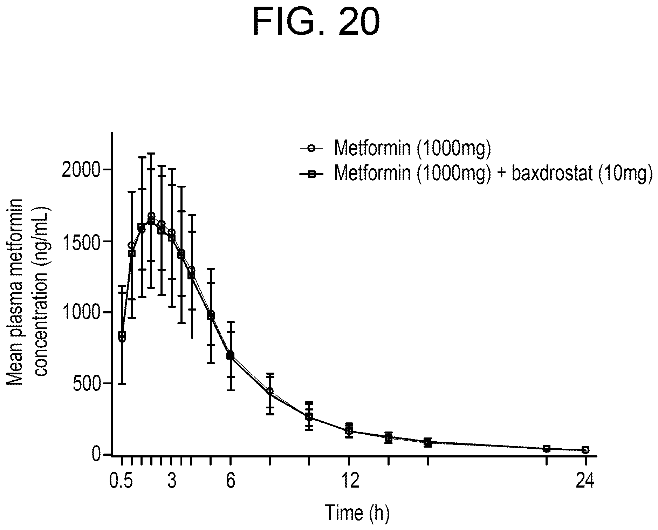

The PK profile of metformin, when administered alone in the current study, was consistent with what would be expected based on the package insert for immediate-release metformin. Furthermore, when administered after a dose of baxdrostat, metformin's plasma PK profile was both qualitatively and quantitatively similar to that of metformin alone (FIG. 20).

After confirming a lack of effect of treatment sequence, PK data for treatment A from all subjects were summarized, and PK data for treatment B from all subjects were summarized. Plasma and urine PK parameters after a dose of metformin alone or in the presence of baxdrostat are reported in Table 7. The 90% CIs of the geometric mean ratios for the plasma Cₘₐₓ and AUC values for metformin in the presence of baxdrostat vs metformin alone fell entirely within the boundaries to claim bioequivalence (80%-125%), demonstrating the absence of a PK drug-drug interaction.

| **Table 7: Plasma and Urine PK Parameters of Metformin in the Presence or Absence of Baxdrostat** | | | | | | | | |
|---|---|---|---|---|---|---|---|---|
| **PK parameter of metformin (plasma or urine)** | **Treatment** | | | | | | | |
| | **N** | **Mean (SD)** | **GM (CV%)a** | **N** | **Mean (SD)** | **GM (CV%)a** | **N** | **GMLS ratio (90% CI),b %** |
| Cₘₐₓ (ng/mL) (plasma) | 26 | 1765.39 (344.41) | 1732.62 (20.1) | 27 | 1793.11 (503.88) | 1723.37 (30.0) | 26 | 98.84 (91.3-107.0) |
| Tₘₐₓ (h) (plasma) | 26 | 2.14 (0.77) | 2.00 (40.0) | 27 | 2.04 (0.69) | 1.92 (36.7) | NA | NA |
| t_{1/2} (h) (plasma) | 26 | 5.52 (1.03) | 5.44 (18.2) | 27 | 6.20 (1.36) | 6.07 (20.9) | NA | NA |
| AUC₀₋₂₄ (h•ng/mL) (plasma) | 26 | 10,552.25 (1769.98) | 10,414.63 (16.6) | 27 | 10,388.94 (2912.76) | 10,014.18 (28.1) | NA | NA |
| AUC₀₋ₜ (h•ng/mL) (plasma) | 26 | 11,158.75 (1810.54) | 11,024.21 (15.9) | 27 | 11,043.59 (2898.80) | 10,688.87 (26.5) | 26 | 96.77 (90.7-103.2) |
| AUC_{0-inf} (h•ng/mL) (plasma) | 25 | 11,224.76 (1849.81) | 11,087.78 (15.9) | 26 | 11,319.24 (2905.12) | 10,965.41 (26.3) | 24 | 100.16 (94.4-106.3) |
| CLR (L/hr) (urine) | 26 | 27.99 (4.64) | 27.62 (16.8) | 27 | 26.48 (5.61) | 25.77 (25.9) | NA | NA |
| A_{e (0-72)} (µg) (urine) | 26 | 312,255.23 (75,636.13) | 304,520.62 (22.6) | 27 | 287,165.62 (83,016.05) | 275,459.85 (30.5) | NA | NA |
| a Geometric CV% = 100(exp(SD2)-1)0.5, where SD is the standard deviation of the log-transformed data. b A 90% CI within the boundary of 80% to 125% is the requirement to claim no effect of baxdrostat on metformin PK parameters. Abbreviations: Cₘₐₓ, maximum observed plasma concentration; Tₘₐₓ, time to maximum observed plasma concentration. | | | | | | | | |

The urine PK parameters for metformin alone and in the presence of baxdrostat are presented in Table 8, and the Aₑ of metformin excreted following administration of metformin in the presence or absence of baxdrostat was measured and the results confirmed that the renal excretion of metformin was not significantly affected by baxdrostat.

| **Table 8: Cumulative Fraction of Unchanged Metformin Excreted in Urine of Subjects Receiving Metformin in the Presence or Absence of Baxdrostat** | | |
|---|---|---|
| | **Cumulative fraction of unchanged metformin excreted, value (%)** | |
| | **Metformin (n=26)** | **Metformin + baxdrostat (n=27)** |
| 0-3 h | 11.76 (33.0) | 10.47 (36.6) |
| 0-6 h | 21.50 (27.2) | 19.06 (32.4) |
| 0-9 h | 25.40 (28.1) | 23.11 (33.8) |
| 0-12 h | 27.42 (26.2) | 25.07 (32.5) |
| 0-18 h | 29.11 (25.6) | 26.52 (31.3) |
| 0-24 h | 29.82 (25.1) | 27.21 (30.6) |
| 0-30 h | 30.21 (24.9) | 27.62 (30.3) |
| 0-36 h | 30.50 (24.7) | 27.87 (30.0) |
| 0-48 h | 30.90 (24.5) | 28.25 (29.4) |
| 0-72 h | 31.23 (24.2) | 28.72 (28.9) |

The PK parameters of baxdrostat and its primary metabolite (Table 9) were similar to previously published data, thereby demonstrating study validity.

| **Table 9: Plasma Pharmacokinetic Parameters of Baxdrostat and Its Primary Metabolite Baxdrostat-M** | | | | | | |
|---|---|---|---|---|---|---|
| **Plasma PK Parameter** | **Baxdrostat** | | | **Baxdrostat-M** | | |
| | **n** | **Mean (SD)** | **GM (CV%)^{a}** | **n** | **Mean (SD)** | **GM (CV%)^{a}** |
| Cₘₐₓ (ng/mL) | 27 | 118.94 (25.57) | 116.508 (20.6) | 27 | 6.72 (3.21) | 6.159 (42.1) |
| Tₘₐₓ (h) | 27 | 1.68 (0.77) | 1.545 (40.9) | 27 | 15.74 (9.86) | 12.396 (84.7) |
| λ_{z} (1/h) | 20 | 0.033 (0.0079) | 0.032 (23.4) | 12 | 0.030 (0.0043) | 0.030 (13.8) |
| AUC₀₋₂₄ (h•ng/mL) | 27 | 1663.02 (303.45) | 1637.461 (18.0) | 27 | 110.71 (48.42) | 103.013 (37.7) |
| AUC₀₋₇₂ (h•ng/mL) | 27 | 2872.71 (707.04) | 2788.93 (25.5) | 27 | 263.52 (111.57) | 247.77 (34.3) |
| AUC_{0-inf} (h•ng/mL) | 20 | 3025.04 (811.35) | 2916.93 (28.8) | NA | NA | NA |
| AUC_{%extrap} (h•ng/mL) | 20 | 9.87 (5.18) | 8.36 (71.0) | NA | NA | NA |
| ^{a} Geometric CV% = 100(exp(SD²)-1)^{0.5}, where SD is the standard deviation of the log-transformed data. Abbreviations: Cₘₐₓ, maximum observed plasma concentration; Tₘₐₓ, time to maximum observed plasma concentration. | | | | | | |

### (iii) Safety

There were no deaths, serious AEs, or discontinuations due to treatment-emergent adverse events (TEAEs), and the incidence and severity of AEs were similar when metformin was administered in the presence or absence of baxdrostat (Table 10). Overall, 7 subjects experienced a total of 15 TEAEs: 5 (19.2%) subjects experienced a total of 6 TEAEs following administration of metformin alone and 6 (22.2%) subjects experienced a total of 9 TEAEs following dosing with metformin and baxdrostat. Four of these subjects experienced TEAEs following both treatments (metformin alone and metformin following a dose of baxdrostat).

| **Table 10: Summary of Adverse Events** | | | |
|---|---|---|---|
| **Adverse events, No. (%)** | | **Metformin (n=26) No. (%) e** | **Metformin + baxdrostat (n=27), No. (%) e** |
| Any TEAE | | 5(19.2) 6 | 6 (22.2) 9 |
| Any baxdrostat-related TEAE | | 0 (0.0) 0 | 0 (0.0) 0 |
| Any metformin-related TEAE | | 4 (15.4) 5 | 6 (22.2) 7 |
| Any TEAEs leading to withdrawal of study drug | | 0 (0.0) 0 | 0 (0.0) 0 |
| Any TEAEs leading to death | | 0 (0.0) 0 | 0 (0.0) 0 |
| TEAEs by MedDRA class | | | |
| Gastrointestinal disorders | | 4 (15.4) | 5 (18.5) |
| | Diarrhea | 3 (11.5) | 4 (14.8) |
| | Abdominal pain upper | 1 (3.8) | 1 (3.7) |
| | Flatulence | 0 (0.0) | 1 (3.7) |
| | Nausea | 1 (3.8) | 0 (0.0) |
| Nervous system disorders | | 0 (0.0) | 2 (7.4) |
| | Dizziness postural | 0 (0.0) | 1 (3.7) |
| | Presyncope | 0 (0.0) | 1 (3.7) |
| General disorders and administration site conditions | | 0 (0.0) | 1 (3.7) |
| | Vessel puncture site pain | 0 (0.0) | 1 (3.7) |
| Injury, poisoning, and procedural complications | | 1 (3.8) | 0 (0.0) |
| | Burns first degree | 1 (3.8) | 0 (0.0) |
| TEAEs are defined as any adverse event, regardless of relationship to the study drug, that began after the first dose was administered. | | | |

All reported TEAEs were mild; no subjects experienced moderate or severe TEAEs. The most common TEAEs were gastrointestinal disorders. Five (18.5%) subjects reported diarrhea, an AE commonly associated with metformin treatment: 3 (11.5%) subjects following administration of metformin alone, 4 (14.8%) subjects following dosing of both metformin and baxdrostat, and 2 subjects following both treatments. No clinically meaningful changes were observed in physical examinations, vital signs, ECGs (including no QT prolongation), or clinical laboratory results.

### D. Conclusions

The results of the current study demonstrated that single doses of metformin and baxdrostat were well tolerated when co-administered to healthy subjects with mostly mild gastrointestinal AEs observed, as was expected with a large dose of metformin. Baxdrostat did not significantly affect plasma concentrations or renal clearance of metformin. Therefore, diabetic patients with hypertension receiving metformin and baxdrostat are unlikely to require a dose adjustment of metformin due to a PK interaction.

### Example 7: A Phase 1, open-label study of the absorption, metabolism, and excretion of [¹⁴C]-baxdrostat following a single oral dose in healthy male subjects

Eight male subjects each received a single oral 10 mg (containing approximately 100 uCi) dose of baxdrostat. Blood, urine and fecal samples were collected to determine the metabolic and excretion pathways of baxdrostat. Preliminary findings indicate that the mean excretion of radioactivity was 69.4% and 15.3%, of the dose in the urine and feces, respectively. Unchanged baxdrostat was the major component in urine and feces, constituting 16.8% and 6.1% of the dose, respectively. In plasma, baxdrostat was the main circulating species, constituting 71% of total systemic exposure by the AUC curve to infinite time (AUC_{0-inf}). Mean blood:plasma concentration ratios indicated low association of radioactivity with blood cells.

### Example 8: Study in Patients with Resistant Hypertension

This study was a randomized, double-blind, placebo-controlled study to evaluate the efficacy and safety of baxdrostat in patients with rHTN. Patients with rHTN were defined as being on a stable regimen of ≥ 3 antihypertensive agents, one of which is a diuretic with a mean seated blood pressure (BP) ≥130/80 mmHg. The primary objective was to demonstrate that at least one dose strength of baxdrostat is superior to placebo in mean change from baseline in seated SBP after 12 weeks of treatment. The secondary objectives were to evaluate the change from baseline in mean seated DBP with each of the selected dose strengths of baxdrostat compared to placebo and to evaluate the percentage of patients achieving a seated BP response < 130/80 mmHg with each of the dose strengths of baxdrostat compared to placebo after 12 weeks of treatment. Patients who remained on a stable regimen of ≥3 antihypertensive agents, including a non-potassium sparing diuretic for at least two weeks, had a mean seated BP ≥ 130/80 mmHg were eligible to participate in the study and were randomly assigned to 1 of the 4 treatment groups (baxdrostat regimen of 0.5 mg, 1 mg, 2 mg or placebo once daily) in equal distribution. The study was planned to enroll 348 patients.

The primary endpoint was the placebo-adjusted change from baseline to week 12 of the mean office SBP. The secondary endpoints included the placebo-adjusted change from baseline to week 12 in seated office DBP, and the percentage of patients achieving a seated BP response < 130/80 mmHg with each of the selected dose strengths of baxdrostat compared to placebo after 12 weeks of treatment for rHTN.

The point estimates for the main treatment effect and for the secondary endpoints were generated by mixed model analyses of covariance that included responder status as a fixed effect. The primary efficacy analysis was to compare the change in mean seated SBP from baseline to Day 85 between each dose strength of baxdrostat and placebo based on the modified intent to treat (mITT) Population. The change from baseline in mean seated SBP will be analyzed using a mixed model for repeated measures (MMRM) model. The analysis includes fixed effects for treatment, visit, and treatment-by-visit interaction, along with covariates of the baseline mean seated SBP and baseline glomerular filtration rate. The restricted maximum likelihood estimation (REML) approach was used with an unstructured covariance matrix.

Safety was evaluated by comparing the frequencies, type, distribution, and severity of adverse events, by analysis of the results of clinical chemistry, hematology and urinalysis testing, by shift tables comparing the number of participants experiencing a change from normal to abnormal values (low or high) from baseline to week 12, and by detailed evaluation of the circumstances surrounding serious adverse events and events leading to treatment discontinuation.

### Results

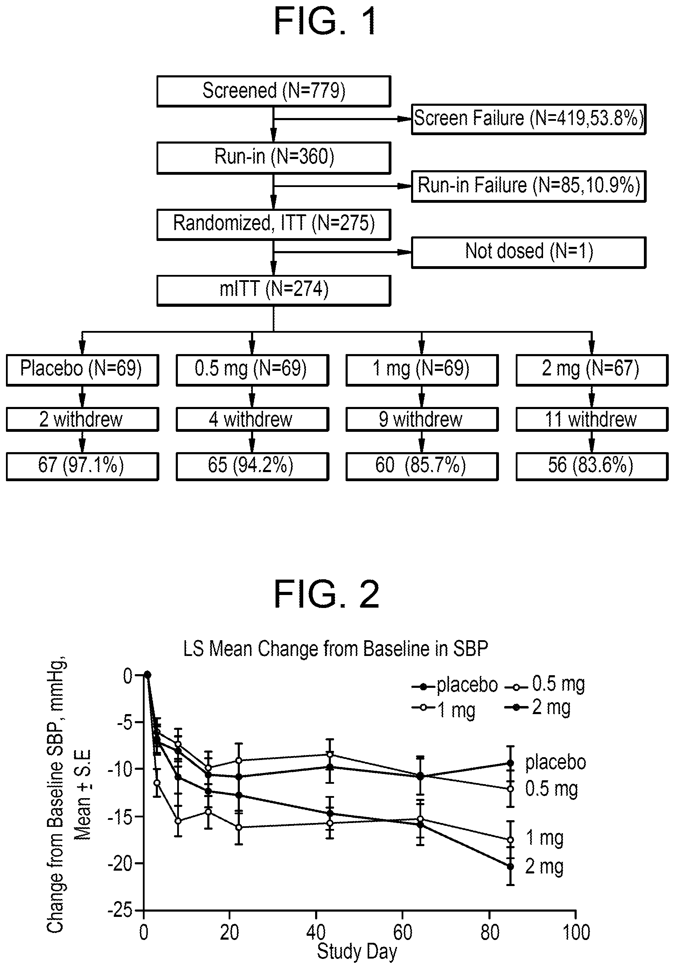

A total of 779 candidates consented to study participation and 275 were eligible and randomly assigned to one of the four treatment groups (0.5mg, 1 mg, 2 mg, baxdrostat or placebo once daily) and 248 patients completed the study (FIG. 1). Fifteen of the 27 discontinued patients withdrew due to lost to follow-up (n=8) or consent withdrawal (n=7), with most occurring when the Coronavirus Disease 2019 (COVID-19) pandemic situation may have interfered with patient retention. When the study was half complete, there was a single discontinuation for lost to follow up in each of the placebo, 1 mg, and 2 mg dose groups, and 3 patients were lost to follow up in the lowest dose 0.5 mg group. This rate of discontinuation in the latter half of the study is not dose dependent. The COVID-19 pandemic likely influenced the discontinuation rate in the first half of the study.

One patient discontinued study participation due to adverse events of intervertebral disc degeneration (n=1) and one patient experienced 3 SAEs of hyperglycemia, hyponatremia and hyperkalemia that led to study withdrawal. (Table 11).

| **Table 11: Reasons for Early Discontinuation** | | | | | | |
|---|---|---|---|---|---|---|
| | | **Placebo (N=69)** | **0.5 mg baxdrostat (N=69)** | **1 mg baxdrostat (N=70)** | **2 mg baxdrostat (N=67)** | **Total (N=275)** |
| **Completed the Study (%)** | | 67 (97.1) | 65 (94.2) | 60 (85.7) | 56 (83.6) | 248 (90.2) |
| **Withdrew Early from Study (%)** | | 2 (2.9) | 4 (5.8) | 10 (14.3) | 11 (16.4) | 27 (9.8) |
| | Lost to Follow-Up | 1 (1.4) | 3 (4.3) | 1 (1.4) | 3 (4.5) | 8 (2.9) |
| Withdrawal by Subject | | 0 (0.0) | 0 (0.0) | 3 (4.3) | 4 (6.0) | 7 (2.5) |
| | Physician Decision | 0 (0.0) | 1 (1.4) | 2 (2.9) | 3 (4.5) | 6 (2.2) |
| | Adverse Event | 0 (0.0) | 0 (0.0) | 1 (1.4) | 1 (1.5) | 2 (0.7) |
| | Protocol Deviation | 0 (0.0) | 0 (0.0) | 2 (2.9) | 0 (0.0) | 2 (0.7) |
| | Other | 1 (1.4) | 0 (0.0) | 1 (1.4) | 0 (0.0) | 2 (0.7) |

The demographics and baseline characteristics of patients in the 4 study arms were well balanced (Table 12). Most of the participants were older, overweight, or obese and had normal or mildly impaired renal functions. All but one patient were taking at least 3 antihypertensive medications including a diuretic. In addition, over 90% of them took an antihypertensive agent in the RAAS class (Table 13). Overall, the population represents typical patients with rHTN.

| **Table 12: Demographics and Baseline Characteristics of Study 121 Patients** | | | | | | |
|---|---|---|---|---|---|---|
| **Characteristic** | | **Placebo (N=69)** | **0.5 mg baxdrostat (N=69)** | **1 mg baxdrostat (N=70)** | **2 mg baxdrostat (N=67)** | **Total (N=275)** |
| **Category/Statistics** | | | | | | |
| Age (years) | | | | | | |
| | Mean | 63.8 | 61.5 | 62.7 | 61.2 | 62.3 |
| | SD | 10.78 | 10.28 | 10.14 | 10.77 | 10.48 |
| Age group, n (%) | | | | | | |
| | <65 years | 32 (46.4) | 39 (56.5) | 39 (55.7) | 41 (61.2) | 151 (54.9) |
| | ≥65 years | 37 (53.6) | 30 (43.5) | 31 (44.3) | 26 (38.8) | 124 (45.1) |
| Sex, n (%) | | | | | | |
| | Male | 42 (60.9) | 36 (52.2) | 37 (52.9) | 38 (56.7) | 153 (55.6) |
| | Female | 27 (39.1) | 33 (47.8) | 33 (47.1) | 29 (43.3) | 122 (44.4) |
| Race, n (%) | | | | | | |
| | White | 51 (73.9) | 45 (65.2) | 48 (68.6) | 47 (70.1) | 191 (69.5) |
| Black or African American | | 16 (23.2) | 22 (31.9) | 20 (28.6) | 19 (28.4) | 77 (28.0) |
| | Asian | 2 (2.9) | 1 (1.4) | 2 (2.9) | 1 (1.5) | 6 (2.2) |
| | Other | 0 (0.0) | 1 (1.4) | 0 (0.0) | 0 (0.0) | 1 (0.4) |
| Ethnicity, n (%) | | | | | | |
| | Hispanic or Latino | 30 (43.5) | 33 (47.8) | 23 (32.9) | 32 (47.8) | 118 (42.9) |
| Not Hispanic or Latino | | 39 (56.5) | 36 (52.2) | 47 (67.1) | 35 (52.2) | 157 (57.1) |
| Body Mass Index (kg/m²) | | | | | | |
| | Mean | 32.1 | 33.2 | 31.9 | 33.3 | 32.6 |
| | SD | 5.26 | 5.26 | 5.21 | 5.05 | 5.21 |
| Seated SBP (mmHg) | | | | | | |
| | Mean | 148.9 | 147.6 | 147.7 | 147.3 | 147.9 |
| | SD | 12.38 | 12.49 | 13.05 | 11.82 | 12.40 |
| Glomerular Filtration Rate (mL/min/1.73m²) | | | | | | |
| | Mean | 85.5 | 81.0 | 83.2 | 85.2 | 83.7 |
| | SD | 17.46 | 20.41 | 20.58 | 19.42 | 19.49 |

| **Table 13: Background Antihypertensive Medications Used** | | | | | | |
|---|---|---|---|---|---|---|
| **Characteristic** | | **Placebo (N=69)** | **0.5 mg baxdrostat (N=69)** | **1 mg baxdrostat (N=70)** | **2 mg baxdrostat (N=67)** | **Total (N=275)** |
| **Category/Statistics** | | | | | | |
| Background Diuretic | | | | | | |
| | Yes | 69 (100.0) | 69 (100.0) | 70 (100.0) | 67 (100.0) | 275 (100.0) |
| | No | 0 (0.0) | 0 (0.0) | 0 (0.0) | 0 (0.0) | 0 (0.0) |
| Background Beta Blocker | | | | | | |
| | Yes | 47 (68.1) | 44 (63.8) | 41 (58.6) | 35 (52.2) | 167 (60.7) |
| | No | 22 (31.9) | 25 (36.2) | 29 (41.4) | 32 (47.8) | 108 (39.3) |
| Background Calcium Channel Blocker | | | | | | |
| | Yes | 47 (68.1) | 44 (63.8) | 49 (70.0) | 47 (70.1) | 187 (68.0) |
| | No | 22 (31.9) | 25 (36.2) | 21 (30.0) | 20 (29.9) | 88 (32.0) |
| Background Agent Acting on the RAAS | | | | | | |
| | Yes | 63 (91.3) | 64 (92.8) | 65 (92.9) | 64 (95.5) | 256 (93.1) |
| | No | 6 (8.7) | 5 (7.2) | 5 (7.1) | 3 (4.5) | 19 (6.9) |
| Background General Antihypertensive | | | | | | |
| | Yes | 9 (13.0) | 8 (11.6) | 11 (15.7) | 8 (11.9) | 36 (13.1) |
| | No | 60 (87.0) | 61 (88.4) | 59 (84.3) | 59 (88.1) | 239 (86.9) |

### Efficacy

Study results demonstrated that baxdrostat at 1 mg and 2 mg were effective in lowering SBP in patients with rHTN. Treatment with baxdrostat at 2 mg produced a 20.3 mmHg reduction in SBP compared to the placebo response of a 9.4 mmHg reduction, yielding a model adjusted, placebo-corrected decline of 11.0 mmHg (95% Confidence Interval [CI] -16.4 mmHg, -5.5 mmHg) (p value =0.0001) thus the null hypothesis was rejected and the effectiveness of baxdrostat 2 mg was established. The next dose of baxdrostat at 1 mg was tested following the planned hierarchical sequence. The 1 mg dose also produced a significant placebo-adjusted SBP decline of 8.1 mmHg (p value=0.003). The treatment effect of 0.5 mg dose was modestly greater than that of placebo, but this difference did not reach statistical significance. Improvement in DBP was also observed. After 12 weeks of treatment, the DBP decreased by 14.3 mmHg, 11.8 mmHg and 8.6 mmHg in the 2 mg, 1 mg and 0.5 mg dose groups, respectively, compared to the 9.2 mmHg decrease in the placebo group (Table 14).

| **Table 14: Baxdrostat Treatment Effects on Blood Pressure** | | | | | | |
|---|---|---|---|---|---|---|
| **Characteristic** | | | **Placebo (N=69)** | **0.5 mg baxdrostat (N=69)** | **1 mg baxdrostat (N=70)** | **2 mg baxdrostat (N=67)** |
| | **Category/Statistics** | | | | | |
| Systolic Blood Pressure (mmHg) | | | | | | |
| | Baseline Mean | | 148.9 | 147.6 | 148.3 | 147.3 |
| | | Standard Deviation | 12.38 | 12.49 | 12.17 | 11.82 |
| | | LS mean change (SE) | -9.4 (1.88) | -12.1 (1.91) | -17.5 (1.95) | -20.3 (2.05) |
| | | LS mean difference (SE) | | -2.7 (2.68) | -8.1 (2.72) | -11.0 (2.78) |
| | | 95% CI | | (-8.0, 2.6) | (-13.5, -2.8) | (-16.4, -5.5) |
| | | *p* value | | *0.3110* | *0.0030* | *0.0001* |
| Diastolic Blood Pressure (mmHg) | | | | | | |
| | Baseline Mean | | 88.2 | 87.6 | 87.7 | 88.2 |
| | | Standard Deviation | 6.13 | 7.71 | 5.97 | 7.13 |
| | | LS mean change (SE) | -9.2 (1.22) | -8.6 (1.23) | -11.8 (1.26) | -14.3 (1.31) |
| | | LS mean difference (SE) | | 0.5 (1.74) | -2.7 (1.75) | -5.2 (1.79) |
| | | 95% CI | | (-2.9, 4.0) | (-6.1, 0.8) | (-8.7, -1.6) |
| | | *p* value | | *0.7536* | *0.1298* | *0.0042* |
| Baseline is defined as the measurement at Randomization (Day 1/Visit 4). If missing, baseline is the last measurement prior to the first dose of double-blind study drug. Glomerular Filtration Rate is estimated using the EPI-CKD formulation. | | | | | | |
| The LS means, CIs, and *p* values are from an MMRM model with change from baseline as the dependent variable with randomized treatment, visit, and treatment-by-visit interaction as fixed categorical effects; and baseline mean seated systolic blood pressure and baseline glomerular filtration rate as continuous covariates. The restricted maximum likelihood estimation (REML) approach was used with an unstructured covariance matrix and the Kenward-Roger approximation for degrees of freedom. | | | | | | |

The SBP decrease was rapid after treatment started and sustained throughout the 12 weeks of treatment as shown in FIG. 2.

Prospectively planned sensitivity analyses were performed using the intent to treat (ITT) or the per protocol analysis sets and showed nearly identical model adjusted and placebo-corrected changes from baseline in the 2 mg group of -11 mmHg (95% CI -16.4 mmHg to -5.5 mmHg) or -11.1 mmHg (95% CI -16.8 mmHg to -5.4 mmHg), respectively. These results supported the primary endpoint analysis.

To further elucidate the treatment effects on patients with various baseline characteristics, the same primary analysis model was used to compare the change in mean seated SBP from baseline to Day 85 between each dose strength of baxdrostat and placebo by subgroups. Subgroup analyses were performed for sex, race, baseline SBP (<145 mmHg or ≥145 mmHg), baseline glomerular filtration rate (GFR) (<60 mL/min/1.73 m² or ≥60 mL/min/1.73 m²), baseline serum aldosterone concentration (<6 ng/dL or ≥6 ng/dL) and background antihypertension medication use. Patients dosed with baxdrostat experienced a greater reduction in SBP if they were male, white, not Hispanic or Latino, higher eGFR, and baseline SBP value of ≥145 mmHg (Table 15A and Table 15B). The blood pressure response by patients with a baseline plasma aldosterone greater or lower than 6 ng/dL showed similar blood pressure reduction. This may reflect the wide variability of a single plasma aldosterone measurement during the study visits. Total aldosterone in the 24-hour urine collection demonstrated substantial treatment related reduction in patients assigned to the active groups.

Substantial difference in treatment effect among subgroups appeared to be driven by the large reduction of SBP in the placebo arms. For example, female participants allocated to the placebo arm showed an SBP decrease of 15.3 mmHg; Hispanic/Latino participants in the placebo arm showed an SBP decrease of 11.3 mmHg. Female Hispanic subjects (N=14) randomized to placebo had a decrease of SBP of 18.1 mmHg compared to a decrease of 7.1 mmHg (N=55) among subjects who were not female and Hispanic. Even though placebo effect is commonly observed in clinical trials, the mean change of -18.1 mmHg is quite unusual (Table 15A and Table 15B).

| **Table 15A: Subgroup Analysis of Sex and Ethnicity** | | | | | | |
|---|---|---|---|---|---|---|
| **Characteristic Category/Statistics^{*}** | | | **Placebo (N=69)** | **0.5 mg baxdrostat (N=69)** | **1 mg baxdrostat (N=70)** | **2 mg baxdrostat (N=67)** |
| **Sex** | | | | | | |
| | Male, n | | 42 | 36 | 37 | 38 |
| | | LS mean change (SE) | -5.7 (2.39) | -13.7 (2.59) | -17.4 (2.76) | -22.4 (2.73) |
| | | LS mean difference (SE) | | -8.0 (3.53) | -11.7 (3.66) | -16.4 (3.63) |
| | | 95% CI | | (-14.9, -1.1) | (-18.9, -4.5) | (-23.8, -9.5) |
| | | *p* value | | *0.0241* | *0.0016* | *<0.0001* |
| | Female, n | | 27 | 33 | 33 | 29 |
| | | LS mean change (SE) | -15.3 (3.04) | -10.2 (2.77) | -17.8 (2.74) | -18.2 (3.01) |
| | | LS mean difference (SE) | | 5.1 (4.12) | -2.5 (4.09) | -2.9 (4.27) |
| | | 95% CI | | (-3.0, 13.2) | (-10.5, 5.6) | (-11.3, 5.5) |
| | | *p* value | | *0.2129* | *0.5437* | *0.4995* |

| Ethnicity | | | | | | |
|---|---|---|---|---|---|---|
| | Hispanic/Latino, n | | 30 | 33 | 23 | 32 |
| | | LS mean change (SE) | -11.3 (2.85) | -13.3 (2.72) | -16.7 (3.43) | -21.8 (2.95) |
| | | LS mean difference (SE) | - | -1.9 (3.94) | -5.4 (4.46) | -10.5 (4.10) |
| | | p value | | *0.6234* | *0.2286* | *0.0109* |
| | Not Hispanic/Latino, n | | 39 | 36 | 47 | 35 |
| | | LS mean change (SE) | -7.8 (2.57) | -10.9 (2.72) | -17.9 (2.41) | -19.2 (2.86) |
| | | LS mean difference (SE) | | -3.1 (3.75) | -10.0 (3.52) | -11.4 (3.84) |
| | | *p* value | | *0.4141* | *0.0047* | *0.0033* |

| Sex & Ethnicity | | | | | | |
|---|---|---|---|---|---|---|
| | Hispanic/Latino Female, n | | 14 | 13 | 11 | 19 |
| | | LS mean change (SE) | -18.1 (4.12) | -15.9 (4.27) | -11.5 (4.80) | -19.4 (3.59) |
| | | LS mean difference (SE) | - | 2.2 (5.93) | 6.6 (6.33) | -1.3 (5.46) |
| | | *p* value | - | *0.7117* | *0.2953* | *0.8078* |
| | Other, n | | 55 | 56 | 58 | 48 |
| | | LS mean change (SE) | -7.1 (2.11) | -11.1 (2.11) | -18.7 (2.13) | -20.9 (2.45) |
| | | LS mean difference (SE) | - | -4.0 (2.99) | -11.6 (3.00) | -13.8 (3.24) |
| | | *p* value | - | *0.1779* | *0.0001* | *<0.0001* |
| * For subgroup analyses, nominal *p* values are presented. | | | | | | |

| **Table 15B: Baxdrostat Treatment effects in Subgroups** | | | | | |
|---|---|---|---|---|---|
| **Sex** | **Statistic** | **Placebo** | **0.5 mg Baxdrostat** | **1 mg Baxdrostat** | **2 mg Baxdrostat** |
| Male | N | 42 | 35 | 29 | 29 |
| | LSM (SE) | -5.7 (2.37) | -13.7 (2.59) | -17.4 (2.76) | -22.1 (2.76) |
| | LSM difference (SE) | - | -8.0 (3.51) | -11.7 (3.64) | -16.4 (3.64) |
| | 95% CI | - | (-14.9, -1.1) | (-18.8, -4.5) | (-23.6, -9.2) |
| | *P* value | - | 0.0229 | 0.0015 | <0.0001 |
| Female | N | 25 | 30 | 31 | 25 |
| | LSM (SE) | -15.3 (3.03) | -10.2 (2.77) | -17.8 (2.73) | -18.2 (3.00) |
| | LSM difference (SE) | - | 5.1 (4.11) | -2.5 (4.08) | -2.9 (4.27) |
| | 95% CI | - | (-3.0, 13.2) | (-10.5, 5.6) | (-11.3, 5.5) |
| | *P* value | - | 0.2126 | 0.5439 | 0.5000 |

### Pharmacodynamic Assessment

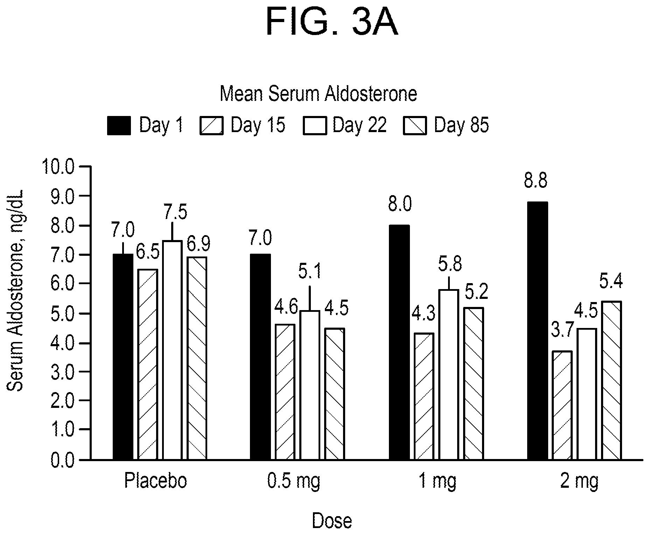

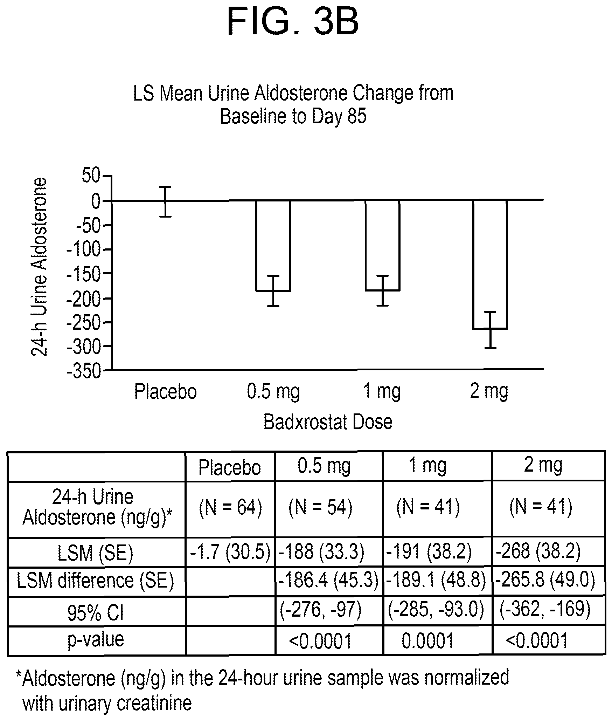

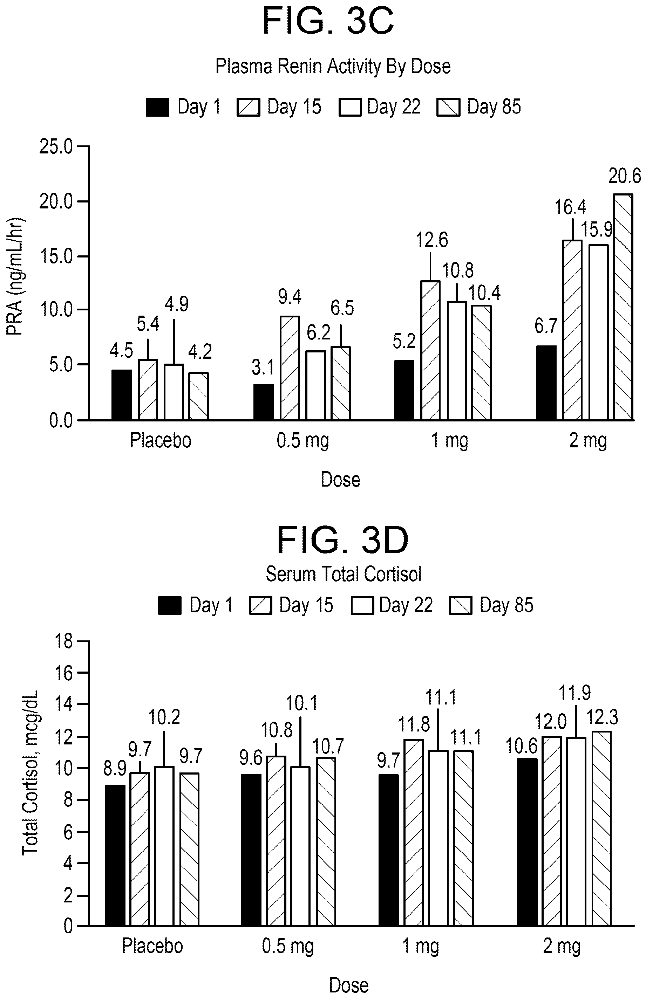

Blood and urine samples were collected for determination of the pharmacodynamic parameters related to baxdrostat mechanism of aldosterone suppression. Serum concentrations of aldosterone, aldosterone excreted in 24-hour urine collections, plasma renin activity (PRA) and serum cortisol are summarized by dose and by study day as shown in FIGs. 3A-3D. The pharmacodynamic results demonstrated a baxdrostat treatment related decrease in serum aldosterone concentration (FIG. 3A), decrease in aldosterone excretion in the 24-hour urine samples (FIG. 3B), increase of the PRA (FIG. 3C) without significantly affecting the total cortisol concentration (FIG. 3D). These data demonstrated that treatment with baxdrostat resulted in an average 50% to 60% reduction of serum aldosterone, significantly reduced urinary aldosterone excretion and increased PRA several-fold yet with no reduction in total serum cortisol. These hormonal data provide strong biochemical evidence to support the mechanism by which baxdrostat's inhibition of aldosterone synthesis produces a significant impact on blood pressure levels without lowering serum cortisol levels.

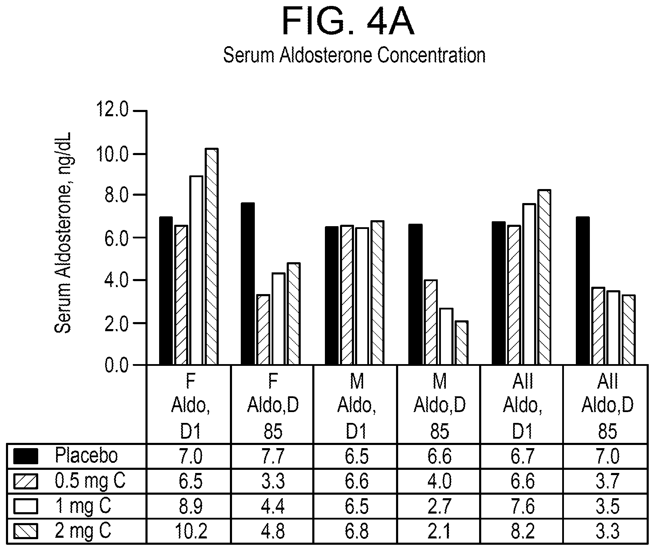

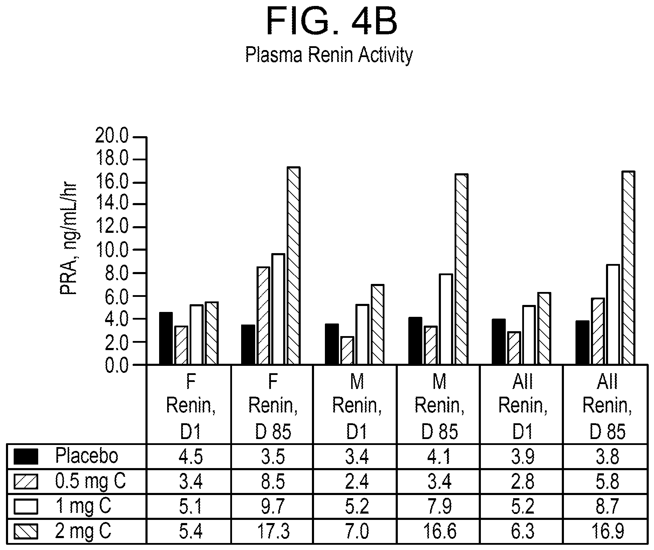

Since a substantial difference in treatment effect on SBP among male and female patients was observed, the potential difference in response to baxdrostat treatment was examined by comparison of serum aldosterone concentration and PRA. FIGs. 4A and 4B show that the suppression of aldosterone and augmentation of renin activity were comparable between male and female patients, suggesting extrinsic factors may have contributed to the unusually high placebo response in the female population, particularly those of Hispanic ethnicity.

### Treatment Emergent AEs and SAEs

Administration of baxdrostat once daily for 12 weeks was well tolerated. 120 of the 274 patients (43.8%) experienced a total of 232 TEAEs (Table 16). A higher percentage of patients in the 1 mg (52.2%) and 2 mg (47.8%) dose groups experienced TEAEs compared to that in the 0.5 mg (34.8%) or placebo (40.6%) group. The most frequently occurring TEAEs experienced by 5% or more patients in any treatment group were urinary tract infection, hyperkalemia, dizziness, headache and fatigue. Most of the TEAEs were mild (62.5%) in severity and deemed not related (89.2%) to the study drug by the investigators.

| **Table 16: Overview of Adverse Events in the Safety Population** | | | | | |
|---|---|---|---|---|---|
| | **Placebo (N=69)** | **0.5 mg baxdrostat (N=69)** | **1 mg baxdrostat (N=70)** | **2 mg baxdrostat (N=67)** | **Total (N=275)** |
| | n (%) e | | | | |
| Any treatment-emergent AEs (TEAEs) | 28 (40.6) 50 | 24 (34.8) 38 | 36 (52.2) 77 | 32 (47.8) 67 | 120 (43.8) 232 |
| Any study drug-related TEAEs | 1 (1.4) 1 | 7(10.1)7 | 9 (13.0) 14 | 3 (4.5) 3 | 20 (7.3) 25 |
| Any TEAEs of special interest | 0 (0.0) 0 | 1 (1.4) 1 | 5 (7.2) 6 | 2 (3.0) 3 | 8 (2.9) 10 |
| Any serious AEs (SAEs) | 2 (2.9) 3 | 0 (0.0) 0 | 2 (2.9) 3 | 6 (9.0) 12 | 10 (3.6) 18 |
| Any serious TEAEs (TESAEs) | 2 (2.9) 3 | 0 (0.0) 0 | 2 (2.9) 3 | 6 (9.0) 12 | 10 (3.6) 18 |
| Any TEAEs leading to discontinuation of study drug | 0 (0.0) 0 | 2 (2.9) 2 | 4 (5.8) 5 | 2 (3.0) 5 | 8 (2.9) 12 |
| Any TEAEs leading to discontinuation of study | 0 (0.0) 0 | 0 (0.0) 0 | 1 (1.4) 1 | 1 (1.5) 3 | 2 (0.7) 4 |
| Any AEs leading to death | 0 (0.0) 0 | 0 (0.0) 0 | 0 (0.0) 0 | 0 (0.0) 0 | 0(0.0) 0 |

TEAEs that occurred more frequently by a rate of 2% or more in the 2 mg or 1 mg group compared to the placebo group are shown in Table 17. These events may be related to the decline in blood pressure (e.g., dizziness, syncope, palpitation) or the mechanism of action of the study drug (i.e., hyperkalemia, hyponatremia, renal impairment). Although there were more patients who experienced urinary tract infection and COVID-19 in the baxdrostat 1 mg or 2 mg group, no causal relationship was identified.

| **Table 17: TEAEs Occurred More Frequently in the High Dose Groups** | | | | | |
|---|---|---|---|---|---|
| | | **Placebo (N=69)** | **0.5 mg baxdrostat (N=69)** | **1 mg baxdrostat (N=70)** | **2 mg baxdrostat (N=67)** |
| | | **n (%) e** | | | |
| Any treatment-emergent AEs (TEAEs) | | 28 (40.6) 50 | 24 (34.8) 38 | 36 (52.2) 77 | 32 (47.8) 67 |

| Infections and infestations | | | | | |
|---|---|---|---|---|---|
| | Urinary tract infection | 3 (4.3) 3 | 3 (4.3) 3 | 7 (10.1) 9 | 7 (10.4) 7 |
| | COVID-19 | 1 (1.4) 1 | 1 (1.4) 1 | 1 (1.4) 1 | 3 (4.5) 3 |

| Nervous system disorders | | | | | |
|---|---|---|---|---|---|
| | Dizziness | 0 (0.0) 0 | 3 (4.3) 3 | 3 (4.3) 3 | 2 (3.0) 3 |
| | Headache | 2 (2.9) 2 | 1 (1.4) 1 | 5 (7.2) 5 | 0 (0.0) 0 |
| | Syncope | 0 (0.0) 0 | 0 (0.0) 0 | 0 (0.0) 0 | 2 (3.0) 2 |

| Metabolism and nutrition disorders | | | | | |
|---|---|---|---|---|---|
| | Hyperkalaemia | 1 (1.4) 1 | 2 (2.9) 2 | 5 (7.2) 5 | 3 (4.5) 3 |
| | Hyponatraemia | 0 (0.0) 0 | 0 (0.0) 0 | 3 (4.3) 3 | 1 (1.5) 1 |

| General disorders and administration site conditions | | | | | |
|---|---|---|---|---|---|
| | Fatigue | 1 (1.4) 2 | 0 (0.0) 0 | 4 (5.8) 4 | 0 (0.0) 0 |
| | Pain | 0 (0.0) 0 | 0 (0.0) 0 | 2 (2.9) 2 | 0 (0.0) 0 |

| Gastrointestinal disorders | | | | | |
|---|---|---|---|---|---|
| | Nausea | 1 (1.4) 1 | 0 (0.0) 0 | 2 (2.9) 2 | 1 (1.5) 1 |
| | Abdominal pain | 0 (0.0) 0 | 0 (0.0) 0 | 2 (2.9) 2 | 0 (0.0) 0 |

| Musculoskeletal and connective tissue disorders | | | | | |
|---|---|---|---|---|---|
| | Arthralgia | 0 (0.0) 0 | 1 (1.4) 1 | 1 (1.4) 1 | 2 (3.0) 3 |
| | Muscle spasms | 0 (0.0) 0 | 0 (0.0) 0 | 0 (0.0) 0 | 2 (3.0) 2 |

| Injury, poisoning and procedural complications | | | | | |
|---|---|---|---|---|---|
| | Fall | 0 (0.0) 0 | 2 (2.9) 2 | 0 (0.0) 0 | 1 (1.5) 1 |
| | Muscle strain | 0 (0.0) 0 | 0 (0.0) 0 | 0 (0.0) 0 | 2 (3.0) 2 |

| Renal and urinary disorders | | | | | |
|---|---|---|---|---|---|
| | Renal impairment | 0 (0.0) 0 | 0 (0.0) 0 | 3 (4.3) 5 | 1 (1.5) 1 |

| Cardiac disorders | | | | | |
|---|---|---|---|---|---|
| | Palpitations | 0 (0.0) 0 | 0 (0.0) 0 | 0 (0.0) 0 | 3 (4.5) 3 |
| Abbreviation: e, number of events. | | | | | |

Eighteen treatment-emergent serious adverse events (TESAEs) were experienced by 10 patients (Table 18). There were no deaths in the study and all SAEs were assessed by the investigators to be unrelated to baxdrostat. A 72-year-old white male subject with suspected urosepsis in the 2 mg dose group experienced 6 SAEs of acute kidney injury, urinary tract infection, dehydration, hyperglycemia, hyperkalemia and hyponatremia on day 15 in the study. The events were assessed to be unrelated to baxdrostat and the patient withdrew from participation in the study due to these metabolic derangements. There were 3 patients who experienced 2 SAEs each: a patient in the 1 mg dose group with pyelonephritis and nephrolithiasis, a patient in the 2 mg dose group with respiratory failure and cellulitis, and a third patient receiving placebo with pyelonephritis and pneumonia. All other SAEs were experienced by a single patient.

| **Table 18: Treatment-Emergent Serious Adverse Events** | | | | | | |
|---|---|---|---|---|---|---|
| | | **Placebo (N=69)** | **0.5 mg baxdrostat (N=69)** | **1 mg baxdrostat (N=70)** | **2 mg baxdrostat (N=67)** | **Total (N=275)** |
| Number of Subjects (Number of Events) | | 2 (3) | 0 (0) | 2 (3) | 6 (12) | 10 (18) |
| **General disorders and administration site conditions** | | 1 (1) | 0 | | | 1 (1) |
| | Non-cardiac chest pain | 1 (1) | | | | 1 (1) |
| **Infections and infestations** | | 1 (2) | 0 | 1 (1) | 2 (2) | 4 (5) |
| | Cellulitis | | | | 1 (1) | 1 (1) |
| | Pneumonia | 1 (1) | | | | 1 (1) |
| | Pyelonephritis | 1 (1) | | 1 (1) | | 2 (2) |
| | Urinary tract infection | | | | 1 (1) | 1 (1) |
| **Metabolism and nutrition disorders** | | 0 | 0 | | 1 (4) | 1 (4) |
| | Dehydration | | | | 1 (1) | 1 (1) |
| Hyperglycaemia | | | | | 1 (1) | 1 (1) |
| Hyperkalaemia | | | | | 1 (1) | 1 (1) |
| Hyponatraemia | | | | | 1 (1) | 1 (1) |
| **Musculoskeletal and connective tissue disorders** | | | 0 | 1 (1) | 1 (1) | 2 (2) |
| | Arthralgia | | | | 1 (1) | 1 (1) |
| | Intervertebral disc degeneration | | | 1 (1) | | 1 (1) |
| **Nervous system disorders** | | | 0 | | 2 (2) | 2 (2) |
| | Dizziness | | | | 1 (1) | 1 (1) |
| | Syncope | | | | 1 (1) | 1 (1) |
| **Renal and urinary disorders** | | | 0 | 1 (1) | 1 (1) | 2 (2) |
| | Acute kidney injury | | | | 1 (1) | 1 (1) |
| | Nephrolithiasis | | | 1 (1) | | 1 (1) |
| **Respiratory, thoracic and mediastinal disorders** | | | 0 | | 2 (2) | 2 (2) |
| | Acute respiratory failure | | | | 1 (1) | 1 (1) |
| | Respiratory failure | | | | 1 (1) | 1 (1) |

### Adverse Event of Special Interest (AESI)

In this study, adverse events of special interest (AESIs) were events of hypotension, low sodium levels or elevated potassium levels that required clinical intervention. There were a total of 10 AESIs distributed among the treatment arms in 8 patients: placebo (0), 0.5 mg (1), 1 mg arm (5), and 2 mg arm (2). In cases of hyperkalemia or hyponatremia, close follow up was mandated in the protocol, and recommendations for dietary or hydration interventions were made by the study team to the investigators upon recognition of the laboratory abnormality.

The sole AESI of hypotension occurred in a patient in the 1 mg baxdrostat dose group who discontinued the study drug with resulting resolution of the hypotension. There were three AESIs of hyponatremia or low sodium. One patient was in the 2 mg dose group with suspected urosepsis (noted above) who experienced 6 SAEs of acute kidney injury, urinary tract infection, dehydration, hyperglycemia, hyperkalemia and hyponatremia, and discontinued the study due to adverse events. Another patient randomized to the 1 mg baxdrostat group had an AESI of hyponatremia that was moderate in severity, related to study drug, and their study drug and diuretic were permanently discontinued. They recovered. A final, third, patient who received 1 mg baxdrostat had hyponatremia of 129 mEq/L noted at nadir; study drug was interrupted and then resumed. Their AESI was moderate in severity, study drug related, and resolved.

Of 6 AESIs of hyperkalemia, 2 are discussed above. None were associated with ECG changes or hospitalization. All patients with hyperkalemia were on other medications known to be associated with hyperkalemia (e.g., beta blockers or ACEIs), and no patient discontinued from the study specifically for hyperkalemia.

### Serum Electrolytes

Aldosterone plays a direct role in the pathogenesis of hypertension via increasing renal absorption of sodium and water while promoting excretion of potassium. There is the potential therefore for an aldosterone synthesis inhibitor to cause the electrolyte abnormalities of hyperkalemia or hyponatremia. Moreover, the decrease in SBP expected with aldosterone inhibitors will reduce blood flow through the afferent arterioles in the kidney, leading to an apparent decline in determination of eGFR. These potential perturbations in kidney physiology were closely monitored throughout the study via the measurement of serum electrolytes and eGFR.

The overall change in serum potassium from baseline to end of treatment was a mean (standard deviation) of -0.08 (0.429) mEq/L in the placebo group, and it rose in the baxdrostat groups by 0.19 (0.474) mEq/L in the 0.5 mg dose group, 0.36 (0.481) mEq/L in the 1 mg dose group and 0.29 mEq/L (0.380) in the 2 mg dose group (FIG. 5).

There were 3 patients with moderate hyperkalemia (potassium >6.0 mEq/L) in the 2 highest dose groups for an overall rate of 2.2%. The rate for spironolactone in the PATHWAY-2 study was 2.1%. Hyperkalemia typically represented isolated values which resolved after dietary advice and without modification of study drug dosing.

Hypokalemia is a known complication of therapy with diuretics which was required for study entry. Hypokalemia is defined as mild (potassium 3.5 to 3.1 mEq/L), moderate (potassium 3.0 to 2.5 mEq/L) and severe (less than 2.5 mEq/L). There were two patients in the placebo group with moderate hypokalemia; there were no cases in any baxdrostat dose groups. The absence of moderate hypokalemia in any baxdrostat treated patients suggests that the use of baxdrostat may offer protection from potential thiazide diuretic-induced hypokalemia.

Similarly, baxdrostat treatment was associated with a slight decrease in serum sodium concentration. The range of decline in sodium was from baseline to end of treatment was a mean (SD) of -1.2 (2.73) mEq/L in the 0.5 mg dose group, -1.1 (3.03) mEq/L in the 1 mg dose group to -2.4 mEq/L (2.94) in the 2 mg dose group which posed no significant clinical concerns (FIG. 6). Ten patients developed mild hyponatremia defined as a serum sodium of < 130 mEq/L with 1 in the placebo group, 1 in the 0.5 mg baxdrostat group, 7 in the 1 mg baxdrostat group and 1 in the 2 mg group. Four discontinued the study early including one of the patients in the placebo group. Over the course of the study, investigators recognized that the sodium was also easily corrected with fluid management advice and no discontinuations from a low serum sodium occurred after the midpoint of the trial.

During the study, baxdrostat treatment was associated with a mild dose-dependent decrease in mean eGFR changes appeared related to the baxdrostat dose (FIG. 7). Clinical outcomes revealed 3 AEs of renal impairment in the 1 mg baxdrostat dose group, 1 in the 2 mg dose group and no cases in the 0.5 mg dose group. All patients recovered. Overall, the modest decline in eGFR seen in the figure is consistent with the renal hemodynamic effect observed with all classes of RAAS inhibitors, particularly aldosterone antagonists such as spironolactone, which is entirely reversible upon discontinuation. There was no apparent relationship between baxdrostat dose and serum calcium, serum phosphorus, or serum phosphorous levels.

### Summary of Safety Profile

Study 121 demonstrated that baxdrostat at doses of 0.5 mg, 1 mg, and 2 mg is safe and well tolerated. There were no deaths in the study. The majority of TEAEs were mild and over 89% were deemed not related to study drug. Of the 18 SAEs in 10 patients, only two patients discontinued due to SAEs, and all SAEs were considered unrelated to study drug by the investigators.

Initially, there were several study discontinuations due to lost to follow up or patient decision in the higher dose baxdrostat groups and none in the placebo group, but this imbalance between treatment groups resolved later in the study suggesting discontinuations were not related to baxdrostat and more likely due to external factors.

In terms of electrolytes and renal function, the rate of hyperkalemia and hyponatremia were expected based on the mechanism of action of baxdrostat. Baxdrostat treatment was associated with a decrease in mean eGFR that was expected given the experience with aldosterone inhibitors and the mechanism of action of baxdrostat.

### Conclusion

Results from study 121 demonstrated that the 1 mg and 2 mg doses of baxdrostat were effective in lowering SBP in patients with rHTN. The placebo corrected changes (standard error) of -11.0 (2.78) mmHg and -8.1 (2.7) mmHg after 12 weeks of treatment in the 2 mg and 1 mg dose group, respectively, were statistically significant and clinically meaningful in this hard-to-treat population. Treatment with baxdrostat at 2 mg significantly lowered the DBP by 5.2 mmHg compared to the placebo group.

Data from the 121 study demonstrate that baxdrostat is able to lower aldosterone levels in a resistant hypertensive population resulting in substantial SBP reductions at both the 1 mg/d and 2 mg/d doses with good overall safety and tolerability. The study further demonstrated that their mean serum cortisol levels were not lowered by baxdrostat treatment, and there were no reported cases of adrenal insufficiency during the trial. As non-selective inhibition of CYP11B1, the enzyme responsible for cortisol synthesis, has been the major barrier to prior efforts in developing an aldosterone synthase inhibitor for the treatment of hypertension, these results support drug development.

### Example 9: Study in Patients with Uncontrolled Hypertension

Study 124 is a double-blind, placebo-controlled, dose range finding, multicenter study conducted in patients with uHTN receiving background antihypertensive agent(s) that are either an ACEI or an ARB, an ACEI/ARB plus a thiazide diuretic, or an ACEI/ARB plus a calcium channel blocker. The study consists of two parts; Part 1 consists of an 8-week treatment period in which the patients are to be assigned to one of 4 dose groups in equal ratio testing 0.5 mg, 1 mg, and 2mg baxdrostat effects compared to a placebo. Patients are to administer double-blind baxdrostat tablets once per day while they continue taking the background antihypertensive medications. After 8 weeks of double-blind treatment, patients were to enter part 2 of the study unless the patient was assigned to the 2 mg group and the SBP remains ≥ 130 mmHg. In part 2 of the study, all patients are to cease dosing with background antihypertensive medication and take only baxdrostat 2 mg dose as a monotherapy for 4 weeks.

Patient Demographics and baseline characteristics are shown in Tables 19-21.

| **Table 19: Patient Demographics** | | | | |
|---|---|---|---|---|
| | | | **Mean** | **SD** |
| Age (Years) | | | 60.2 | 10.96 |
| | | | n | % |
| | | < 65 years | 155 | 62.2 |
| | | ≥ 65 years | 94 | 37.8 |
| | Gender | | | |
| | | Male | 132 | 53.0 |
| | Female | 117 | 47.0 | |
| Race | | | | |
| | White | 181 | 72.7 | |
| | Black | 60 | 24.1 | |
| | Asian | 6 | 2.4 | |
| | Other | 2 | 0.8 | |
| Ethnicity | | | | |
| | Hispanic or Latino | 133 | 53.4 | |
| | Not Hispanic or Latino | 116 | 46.6 | |

| **Table 20: Background Therapy** | | |
|---|---|---|
| | **n** | **%** |
| Background ACEi/ARB | 104 | 41.8 |
| Background ACEi/ARB and Calcium Channel Blocker | 57 | 22.9 |
| Background ACEi/ARB and Thiazide Diuretic | 81 | 32.5 |
| Background Calcium Channel Blocker | 3 | 1.2 |
| Background Thiazide Diuretic | 4 | 1.6 |

| **Table 21: Baseline Characteristics** | | |
|---|---|---|
| | Mean | SD |
| Body Mass Index (kg/m²) | 32.2 | 5.09 |
| Seated SBP (mmHg) | 146.9 | 8.71 |
| Glomerular Filtration Rate (mL/min/1.73m²) | 88.3 | 18.42 |

Key objectives generally include (i) does Baxdrostat work in uHTN patients, (ii) what doses produce robust BP lowering, and (ii) are there sub-groups of patients who respond better than others, and if yes (a) was response defined by background BP meds affect?, (b) did levels of aldosterone or renin predict response? And (c) Did demographic or race/ethnic characteristics contribute to response?

The primary objective of the trial is to demonstrate that at least one dose strength of baxdrostat is superior to placebo for the change from baseline in mean seated SBP after 8 weeks of treatment in these patients (Part 1 of the study).

The secondary objectives are to evaluate the change from baseline in mean seated DBP with each of the selected dose strengths of baxdrostat compared to placebo after 8 weeks of treatment (Part 1), the change from baseline in 24-hour urine aldosterone and serum aldosterone levels with each of the selected dose strengths of baxdrostat compared to placebo after 8 weeks of treatment (Part 1), the percentage of patients achieving a mean seated SBP < 130 mmHg ("responders") with each of the selected dose strengths of baxdrostat compared to placebo after 8 weeks; and the change from baseline in 24-hour urine renin and serum renin levels with baxdrostat compared to placebo after 8 weeks of treatment. Patients who have completed the Part 2 study through Week 12 or who were considered withdrawn at the end of Part 1 may be eligible to enter a separate open label extension study (Study 130). Part 2 is an exploratory phase of the trial designed to evaluate the effect of 4 weeks of monotherapy with 2 mg baxdrostat in this hypertensive population, but the major goal of the Part 2 period is to transition patients to a long-term open label safety extension study of baxdrostat at the highest dose likely to be used in the hypertension population.

The safety objectives for both Parts 1 and 2 are to evaluate vital signs, standing blood pressure, heart rate, physical examinations, ECG, body weight, and clinical laboratory assessments, including standard safety chemistry panel, hematology, coagulation, and urinalysis.

Key inclusion criteria include that patients are on a stable regimen of anti-hypertensive agents for at least 8 weeks; and have a seated BP ≥ 140 mmHg. Key exclusion criteria include a seated SBP ≥ 180 mmHg and estimated glomerular filtration rate (eGFR) < 30 mL/min/1.73m².

### A. Initial Results

A total of 249 patients have enrolled in the study, 91 have completed 12 weeks of assessment and 16 have withdrawn their study participation early. The reasons for the early withdrawals included consent withdrawal (n=7), lost to follow up (n=1), high blood pressure (n=1), requirement for a prohibited medication (n=1), failure to meet criteria for participating in part 2 (n=3), failure to meeting eligibility criteria (n=1) and adverse events (n=2). Ninety patients have consented to continue the open-label extension study 130.

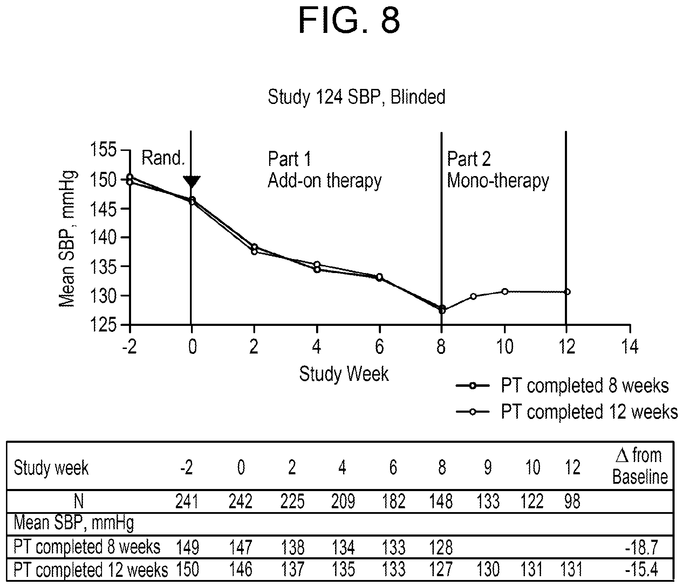

The mean blood pressure, an average of 3 office blood pressure measurements, was 147 mmHg at baseline while patients were on stable regimens of up to two antihypertensive agents. The aggregate blinded SBP, including data from placebo and active baxdrostat groups, exhibited a progressive decline of SBP over 8 weeks during the Part 1 treatment period from a baseline of 147 mmHg to 128 mmHg at week 8 (FIG. 8). The lowering of SBP of 18 mmHg was similar to the aggregate change of approximately 16 mmHg observed in study 121. Interestingly, the average blood pressure did not increase substantially in part 2 of the study when patients ceased dosing of their background antihypertensive medications when baxdrostat was the only study drug taken.

### B. Interim Results

### (i) Part 1

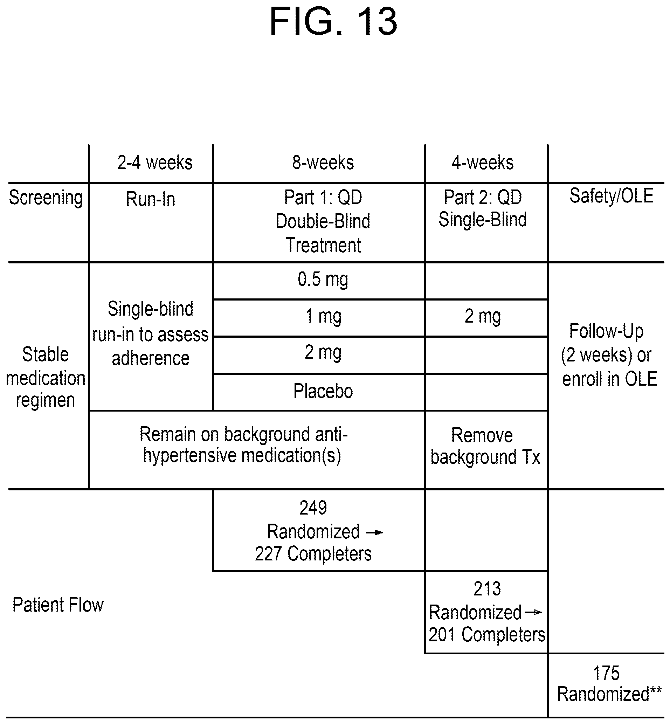

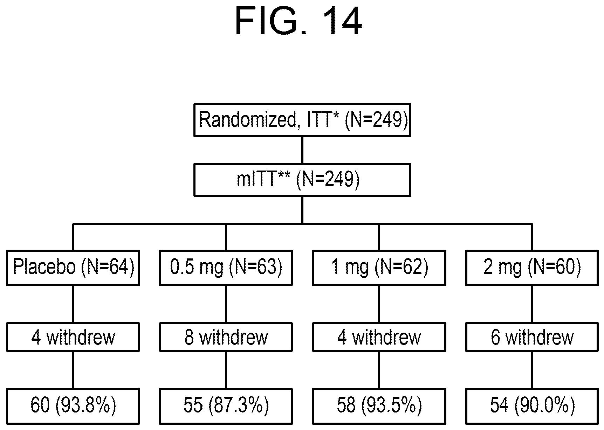

To date, 249 patients were randomized, 227 completed 8-weeks of assessment, 201 completed 12 weeks of assessment and 175 completed the 12 weeks of assessments and the 2 week follow-up period. See, FIGs. 13 and 14. The reasons for withdrawal are shown in Table 22.

| **Table 22: Patient Withdrawal** | | | | | |
|---|---|---|---|---|---|
| **Trial Withdrawals** | **Placebo** | **Baxdrostat** | | | **Total** |
| | | 0.5 mg | 1 mg | 2 mg | |
| Completed the trial (%) | 60 (93.8) | 55 (87.3) | 58 (93.5) | 54 (90.0) | 227 (91.2) |
| Withdrew Early from Trial (%) | 4 (6.3) | 8 (12.7) | 4 (6.5) | 6 (10.0) | 22 (8.8) |
| Lost To Follow-Up | 1 (1.6) | 1 (1.6) | 2 (3.2) | 0 (0.0) | 4 (1.6) |
| Withdrawal By Patient | 3 (4.7) | 3 (4.8) | 2 (3.2) | 4 (6.7) | 12 (4.8) |
| Physician Decision | 0 (0.0) | 3 (4.8) | 0 (0.0) | 0 (0.0) | 3 (1.2) |
| Adverse Event | 0 (0.0) | 1 (1.6) | 0 (0.0) | 1 (1.7) | 2 (0.8) |
| Other (Prohibited Conmed Required) | 0 (0.0)) | 0 (0.0) | 0 (0.0) | 1 (1.7) | 1 (0.4) |
| *ITT: Intention-to-Treat; **mITT: Modified intention-to-treat | | | | | |

The primary efficacy endpoint results are shown in Table 23.

| **Table 23: Summary and Analysis of Change from Baseline in SBP: MMRM mITT Population** | | | | | |
|---|---|---|---|---|---|
| Visit | | Placebo | 0.5 mg Baxdrostat | 1 mg Baxdrostat | 2 mg Baxdrostat |
| | Statistic | (N=64) | (N=63) | (N=62) | (N=60) |
| Week 8 | | | | | |
| | n | 61 | 57 | 58 | 55 |
| | Mean | 130.5 | 129.1 | 130.8 | 126.6 |
| | Standard Deviation | 11.04 | 10.76 | 13.81 | 13.87 |
| | Median | 128.0 | 127.0 | 128.0 | 125.0 |
| | Minimum | 103 | 107 | 102 | 97 |
| | Maximum | 155 | 151 | 192 | 167 |
| Change from Baseline to Week 8 | | | | | |
| | n' [1] | 61 | 57 | 58 | 55 |
| | Mean | -17.8 | -17.0 | -16.6 | -19.3 |
| | Standard Deviation | 12.39 | 10.95 | 14.77 | 13.57 |
| | Median | -17.0 | -17.0 | -16.0 | -18.0 |
| | Minimum | -51 | -42 | -54 | -49 |
| | Maximum | 3 | 6 | 38 | 14 |
| ------------ MMRM [2] ---------- | | | | | |
| | LS mean (SE) | -16.6 (1.58) | -17.0 (1.63) | -16.0 (1.62) | -19.8 (1.67) |
| | LS mean difference (SE) | | -0.5 (2.21) | 0.6 (2.20) | -3.2 (2.23) |
| | 95% CI of LS mean difference | | (-4.8, 3.9) | (-3.8, 4.9) | (-7.6, 1.2) |
| | p-value | | 0.8303 | 0.7915 | 0.1530 |

In this table, CI = confidence interval, DBP = diastolic blood pressure, LS = least squares, SBP = systolic blood pressure, SE = standard error. The LS means, CIs, and p-values are from an MMRM model with change from baseline as the dependent variable with randomized treatment, visit, and treatment-by-visit interaction as fixed categorical effects; along with covariates of the baseline value, race (African American versus non-African American), and the type of background antihypertensive regimen. The restricted maximum likelihood estimation (REML) approach will be used with an unstructured covariance matrix and the Kenward-Roger approximation for degrees of freedom.

The LS mean change from baseline in SBP is based on week of treatment is shown in Table 24.

| **Table 24: LS Mean Change from Baseline (SE) in SBP (mm Hg)** | | | | |
|---|---|---|---|---|
| **Dose Group** | **Wk 2** | **Wk 4** | **Wk 6** | **Wk 8** |
| Placebo | -7.3 (1.58) | -10.3 (1.56) | -9.5 (1.82) | -16.6 (1.58) |
| 0.5 mg | -10.3 (1.61) | -14.2 (1.61) | -13.5 (1.88) | -17 (1.63) |
| 1 mg | -7.7 (1.6) | -9.7 (1.59) | -13.5 (1.88) | -16 (1.62) |
| 2 mg | -8.8 (1.65) | -13.6 (1.65) | -15.7 (1.93) | -19.8 (1.67) |

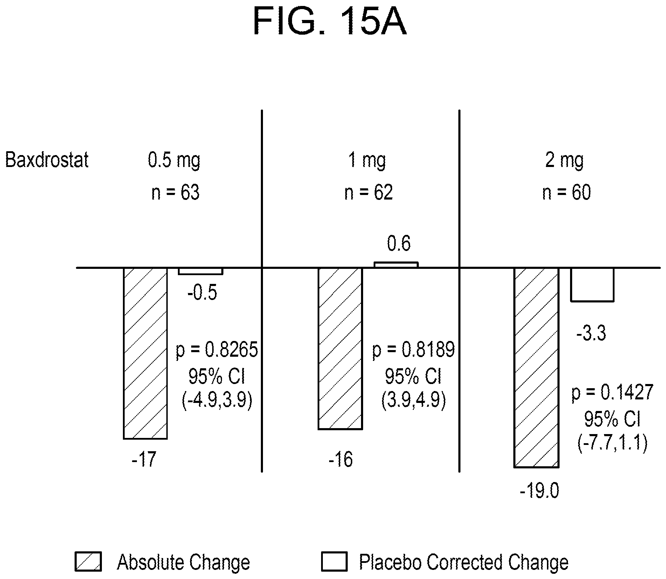

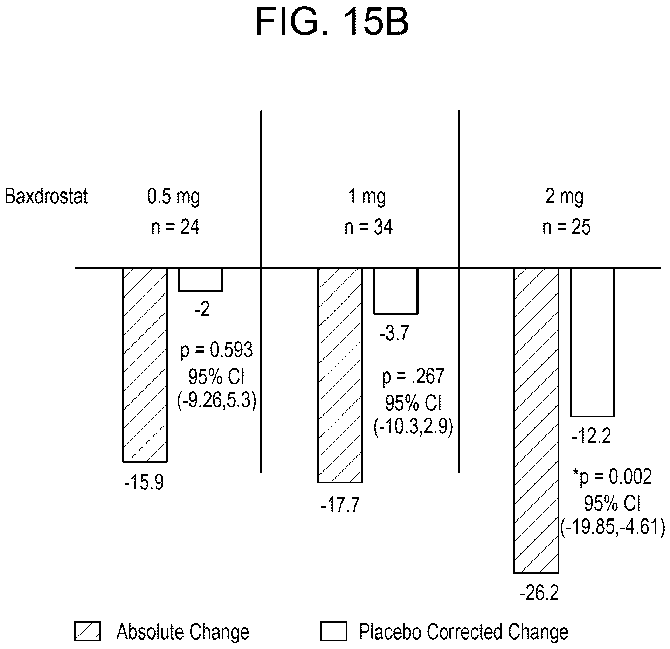

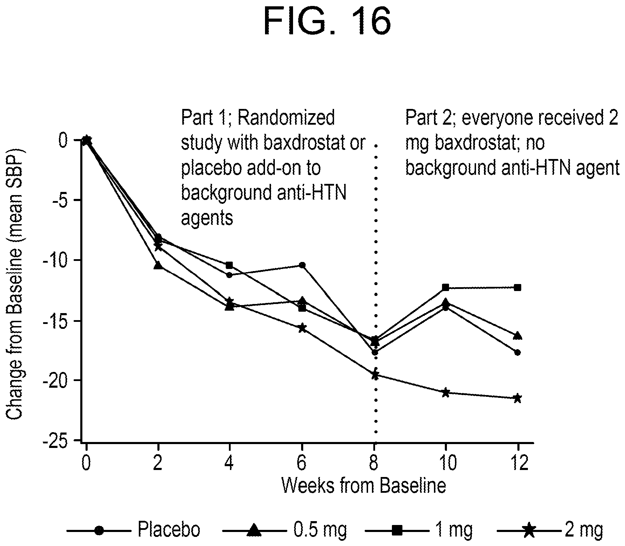

Data also showed that patients in the placebo arm had a large decrease in post-randomization SBP; particularly between week 6 and week 8. See, FIG. 16. Although the primary endpoint was not met (FIG. 14), there was a substantial drop of SBP in the non-Hispanic/Latino population. See, FIG. 15.

### Hispanic/Latino Patient Subgroup

Post unblinding, it was confirmed placebo patients did not receive active drug. It was also noted that for the two mg dose (n = 57), 19/35 Hispanic patients and 1 non-Hispanic patient was determined non-adherent based on PK. For the one mg dosage (n= 57), 7/28 Hispanic patients and two non-Hispanic patients were determined non-adherent based on PK. Tables 25A and 25B show the subgroup analysis.

| **Table 25A: Summary of Model-Based Least-Square Mean Change from Baseline Over Time; All Intent-To-Treat Population for Hispanic or Latino** | | | | | | |
|---|---|---|---|---|---|---|
| Visit | Placebo (N=31) | | | 0.5 mg (N=37) | | |
| | n | LSMEANS | (S.E)¹ | n | LSMEANS | (S.E)¹ |
| Week 2 | 30 | -6.3 | (2.25) | 37 | -6.9 | (1.98) |
| Week 4 | 31 | -10.5 | (2.27) | 36 | -13.0 | (2.04) |
| Week 6 | 31 | -9.2 | (2.64) | 36 | -13.3 | (2.42) |
| Week 8 | 29 | -18.2 | (2.25) | 33 | -17.1 | (2.03) |
| Comp w/ Placebo at Week 6 (Diff (95% CI) p-value) | | | | -4.1 (-10.84, 2.69) 0.235 | | |
| Comp w/ Placebo at Week 8 (Diff (95% CI) p-value) | | | | -1.1 (-4.54, 6.66) 0.709 | | |

| **Table 25B: Summary of Model-Based Least-Square Mean Change from Baseline Over Time; All Intent-To-Treat Population for Hispanic or Latino** | | | | | | |
|---|---|---|---|---|---|---|
| Visit | 1 mg (N=31) | | | 2 mg (N=31) | | |
| | n | LSMEANS | (S.E)¹ | n | LSMEANS | (S.E)¹ |
| Week 2 | 28 | -7.3 | (2.22) | 35 | -5.0 | (2.05) |
| Week 4 | 27 | -8.1 | (2.30) | 35 | -10.1 | (2.11) |
| Week 6 | 26 | -10.6 | (2.76) | 35 | -11.8 | (2.46) |
| Week 8 | 26 | -14.1 | (2.26) | 35 | -16.3 | (2.03) |
| Comp w/ Placebo at Week 6 (Diff (95% CI) p-value) | -1.4 (-8.71, 5.92) 0.707 | | | -2.5 (-9.31, 4.24) 0.460 | | |
| Comp w/ Placebo at Week 8 (Diff (95% CI) p-value) | 4.0 (-2.00, 10.06) 0.188 | | | 1.9 (-3.69, 7.44) 0.506 | | |

The results also confirmed the lack of Hispanic female response. See, Tables 26A-26B and 27A-27B.

| **Table 26A: Summary of Model-Based Least-Squares Mean Change from Baseline Over Time; mITT Population for Female Hispanic/Latino Patients** | | | | | | |
|---|---|---|---|---|---|---|
| Visit | Placebo (N=10) | | | 0.5 mg (N=22) | | |
| | n | LSMEANS | (S.E)¹ | n | LSMEANS | (S.E)¹ |
| Week 2 | 9 | -6.7 | (4.37) | 22 | -6.2 | (2.75) |
| Week 4 | 10 | -8.3 | (4.25) | 21 | -13.4 | (2.84) |
| Week 6 | 10 | -6.4 | (5.05) | 21 | -11.3 | (3.50) |
| Week 8 | 9 | -19.1 | (4.53) | 20 | -16.1 | (2.99) |
| Comp w/ Placebo at Week 6 (Diff (95% CI) p-value) | | | | -4.9 (-16.80, 6.97) 0.412 | | |
| Comp w/ Placebo at Week 8 (Diff (95% CI) p-value) | | | | 3.0 (-7.45, 13.46) 0.567 | | |

| **Table 26B: Summary of Model-Based Least-Squares Mean Change from Baseline Over Time; mITT Population for Female Hispanic/Latino Patients** | | | | | | |
|---|---|---|---|---|---|---|
| Visit | 1 mg (N=14) | | | 2 mg (N=19) | | |
| | n | LSMEANS | (S.E)¹ | n | LSMEANS | (S.E)¹ |
| Week 2 | 14 | -7.8 | (3.41) | 19 | -6.0 | (2.98) |
| Week 4 | 13 | -10.3 | (3.58) | 19 | -7.1 | (3.09) |
| Week 6 | 12 | -12.6 | (4.52) | 19 | -8.0 | (3.67) |
| Week 8 | 12 | -12.7 | (3.86) | 19 | -12.6 | (3.15) |
| Comp w/ Placebo at Week 6 (Diff (95% CI) p-value) | -6.2 (-19.55, 7.07) 0.353 | | | -1.5 (-13.53, 10.44) 0.797 | | |
| Comp w/ Placebo at Week 8 (Diff (95% CI) p-value) | 6.4 (-5.38, 18.10) 0.283 | | | **6.5 (-4.02, 16.98) 0.221** | | |

| **Table 27A: Summary of Model-Based Least-Squares Mean Change from Baseline Over Time; mITT Population for Female Non-Hispanic Female Patients** | | | | | | |
|---|---|---|---|---|---|---|
| Visit | Placebo (N=15) | | | 0.5 mg (N=11) | | |
| | n | LSMEANS | (S.E)¹ | n | LSMEANS | (S.E)¹ |
| Week 2 | 15 | -7.7 | (3.98) | 11 | -12.5 | (4.72) |
| Week 4 | 15 | -10.7 | (3.79) | 10 | -12.2 | (4.65) |
| Week 6 | 15 | -10.3 | (3.90) | 10 | -11.8 | (4.82) |
| Week 8 | 15 | -13.0 | (2.97) | 10 | -16.7 | (3.69) |
| Comp w/ Placebo at Week 6 (Diff (95% CI) p-value) | | | | -1.5 (-13.92, 10.84) 0.803 | | |
| Comp w/ Placebo at Week 8 (Diff (95% CI) p-value) | | | | -3.7 (-13.14, 5.67) 0.425 | | |

| **Table 27B: Summary of Model-Based Least-Squares Mean Change from Baseline Over Time; mITT Population for Female Non-Hispanic Female Patients** | | | | | | |
|---|---|---|---|---|---|---|
| Visit | 1 mg (N=11) | | | 2 mg (N=9) | | |
| | n | LSMEANS | (S.E)¹ | n | LSMEANS | (S.E)¹ |
| Week 2 | 11 | -11.8 | (4.69) | 9 | -15.6 | (5.33) |
| Week 4 | 11 | -13.5 | (4.47) | 9 | -23.8 | (5.08) |
| Week 6 | 11 | -20.3 | (4.60) | 8 | -26.1 | (5.42) |
| Week 8 | 11 | -23.8 | (3.52) | 9 | -32.8 | (4.15) |
| Comp w/ Placebo at Week 6 (Diff (95% CI) p-value) | -10.0 (-22.06, 2.15) 0.104 | | | **-15.8 (-29.06, -2.47) 0.021** | | |
| Comp w/ Placebo at Week 8 (Diff (95% CI) p-value) | -10.8 (-20.05, -1.63) 0.022 | | | -19.8 (-29.87, -9.73) <.001 | | |

Hispanic male response was less robust than the non-Hispanic male response. See, Tables 28A-28B and 29A-29B.

| **Table 28A: Summary of Model-Based Least-Squares Mean Change from Baseline Over Time; mITT Population for Hispanic Male Patients** | | | | | | |
|---|---|---|---|---|---|---|
| Visit | Placebo (N=21) | | | 0.5 mg (N=15) | | |
| | n | LSMEANS | (S.E)¹ | n | LSMEANS | (S.E)¹ |
| Week 2 | 21 | -6.7 | (2.78) | 15 | -8.3 | (3.09) |
| Week 4 | 21 | -12.2 | (2.69) | 15 | -12.6 | (2.99) |
| Week 6 | 21 | -11.3 | (2.92) | 15 | -16.0 | (3.28) |
| Week 8 | 20 | -18.7 | (2.41) | 13 | -18.6 | (2.76) |
| Comp w/ Placebo at Week 6 (Diff (95% CI) p-value) | | | | -4.7 (-12.73, 3.30) 0.244 | | |
| Comp w/ Placebo at Week 8 (Diff (95% CI) p-value) | | | | 0.1 (-6.20, 6.41) 0.974 | | |

| **Table 28B: Summary of Model-Based Least-Squares Mean Change from Baseline Over Time; mITT Population for Hispanic Male Patients** | | | | | | |
|---|---|---|---|---|---|---|
| Visit | 1 mg (N=14) | | | 2 mg (N=16) | | |
| | n | LSMEANS | (S.E)¹ | n | LSMEANS | (S.E)¹ |
| Week 2 | 14 | -7.0 | (3.18) | 16 | -5.1 | (3.15) |
| Week 4 | 14 | -6.4 | (3.07) | 16 | -14.6 | (3.05) |
| Week 6 | 14 | -9.2 | (3.37) | 16 | -17.5 | (3.32) |
| Week 8 | 14 | -15.8 | (2.67) | 16 | -21.9 | (2.70) |
| Comp w/ Placebo at Week 6 (Diff (95% CI) p-value) | 2.1 (-6.16, 10.29) 0.618 | | | -6.2 (-14.14, 1.67) 0.120 | | |
| Comp w/ Placebo at Week 8 (Diff (95% CI) p-value) | 2.9 (-3.41, 9.25) 0.359 | | | **-3.2 (-9.28, 2.87) 0.296** | | |

| **Table 29A: Summary of Model-Based Least-Squares Mean Change from Baseline Over Time; mITT Population for Non-Hispanic Male Patients** | | | | | | |
|---|---|---|---|---|---|---|
| Visit | Placebo (N=16) | | | 0.5 mg (N=12) | | |
| | n | LSMEANS | (S.E)¹ | n | LSMEANS | (S.E)¹ |
| Week 2 | 16 | -8.9 | (2.71) | 12 | -19.9 | (3.28) |
| Week 4 | 16 | -10.0 | (2.66) | 11 | -20.5 | (3.33) |
| Week 6 | 16 | -9.9 | (3.45) | 11 | -16.3 | (4.24) |
| Week 8 | 16 | -14.9 | (3.37) | 12 | -17.2 | (3.93) |
| Comp w/ Placebo at Week 6 (Diff (95% CI) p-value) | | | | -6.4 (-17.23, 4.48) 0.244 | | |
| Comp w/ Placebo at Week 8 (Diff (95% CI) p-value) | | | | -2.2 (-12.46, 8.04) 0.688 | | |

| **Table 29B: Summary of Model-Based Least-Squares Mean Change from Baseline Over Time; mITT Population for Non-Hispanic Male Patients** | | | | | | |
|---|---|---|---|---|---|---|
| Visit | 1 mg (N=22) | | | 2 mg (N=13) | | |
| | n | LSMEANS | (S.E)¹ | n | LSMEANS | (S.E)¹ |
| Week 2 | 22 | -6.8 | (2.42) | 13 | -16.2 | (3.16) |
| Week 4 | 22 | -10.3 | (2.38) | 12 | -17.4 | (3.19) |
| Week 6 | 21 | -14.2 | (3.12) | 11 | -21.5 | (4.21) |
| Week 8 | 21 | -14.7 | (3.03) | 10 | -22.3 | (4.35) |
| Comp w/ Placebo at Week 6 (Diff (95% CI) p-value) | -4.3 (-13.56, 5.00) 0.359 | | | -11.6 (-22.42, -0.83) 0.35 | | |
| Comp w/ Placebo at Week 8 (Diff (95% CI) p-value) | 0.2 (-8.82, 9.26) 0.961 | | | **-7.4 (-18.32, 3.53) 0.180** | | |

The results were further analyzed to determine drug effectiveness in the adherent Hispanic patients. See, Table 30.

| **Table 30: mITT Population for Hispanic Compliant Patients** | | | | | | |
|---|---|---|---|---|---|---|
| | Placebo (N=62) | 2 mg (N=16) | | | | |
| Visit | n | LSMEANS | (S.E.)¹ | n | LSMEANS | (S.E.)¹ |
| Week 2 | 61 | -7.2 | (1.64) | 16 | -4.6 | (3.15) |
| Week 4 | 62 | -10.2 | (1.55) | 16 | -13.9 | (2.98) |
| Week 6 | 62 | -9.5 | (1.87) | 16 | -15.4 | (3.64) |
| Week 8 | 60 | -16.2 | (1.56) | 16 | -22.8 | (2.97) |
| Comp w Placebo at Week 6 (Diff (95% CI) p-value) | | | | -6.0 (-13.91, 2.00) 0.141 | | |
| Comp w Placebo at Week 8 (Diff (95% CI) p-value) | | | | **-6.6** (-13.07, -0.13) **0.046** | | |

The results showed that baxdrostat (2 mg) was effective in adherent Hispanic/Latino, but efficacy response was substantially lower.

### Black/African American Subgroup

The Black/African American subgroup also was analyzed and it was found that 2 mg of Baxdrostat was effective. See, Tables 31A and 31B.

| **Table 31A: Summary of Model-Based Least-Square Mean Change from Baseline over Time; mITT Population for Compliance for Black Patients** | | | | | | |
|---|---|---|---|---|---|---|
| Visit | Placebo (N=14) | | | 0.5 mg (N=11) | | |
| | n | LSMEANS | (S.E)¹ | n | LSMEANS | (S.E)¹ |
| Week 2 | 14 | -7.4 | (3.53) | 11 | -9.0 | (3.98) |
| Week 4 | 14 | -10.8 | (3.31) | 10 | -11.6 | (3.90) |
| Week 6 | 14 | -10.3 | (3.90) | 10 | -13.0 | (4.64) |
| Week 8 | 14 | -14.2 | (3.30) | 9 | -17.2 | (4.03) |
| Comp w/ Placebo at Week 6 (Diff (95% CI) p-value) | | | | -2.6 (-14.94, 9.64) 0.665 | | |
| Comp w/ Placebo at Week 8 (Diff (95% CI) p-value) | | | | -3.0 (-13.58, 7.49) 0.562 | | |

| **Table 31B: Summary of Model-Based Least-Square Mean Change from Baseline over Time; mITT Population for Compliance for Black Patients** | | | | | | |
|---|---|---|---|---|---|---|
| Visit | 1 mg (N=13) | | | Placebo (N=8) | | |
| | n | LSMEANS | (S.E)¹ | n | LSMEANS | (S.E)¹ |
| Week 2 | 13 | -8.9 | (3.69) | 8 | -7.7 | (4.79) |
| Week 4 | 13 | -11.8 | (3.47) | 8 | -18.3 | (4.51) |
| Week 6 | 13 | -13.5 | (4.08) | 7 | -20.6 | (5.53) |
| Week 8 | 13 | -17.4 | (3.47) | 7 | -27.6 | (4.71) |
| Comp w/ Placebo at Week 6 (Diff (95% CI) p-value) | -3.1 (-14.59, 8.31) 0.582 | | | -10.3 (-24.04, 3.43) 0.137 | | |
| Comp w/ Placebo at Week 8 (Diff (95% CI) p-value) | -3.2 (-12.88, 6.52) 0.511 | | | -13.4 (-25.04, -1.69) 0.026 | | |

### Aldosterone Changes by Ethnicity

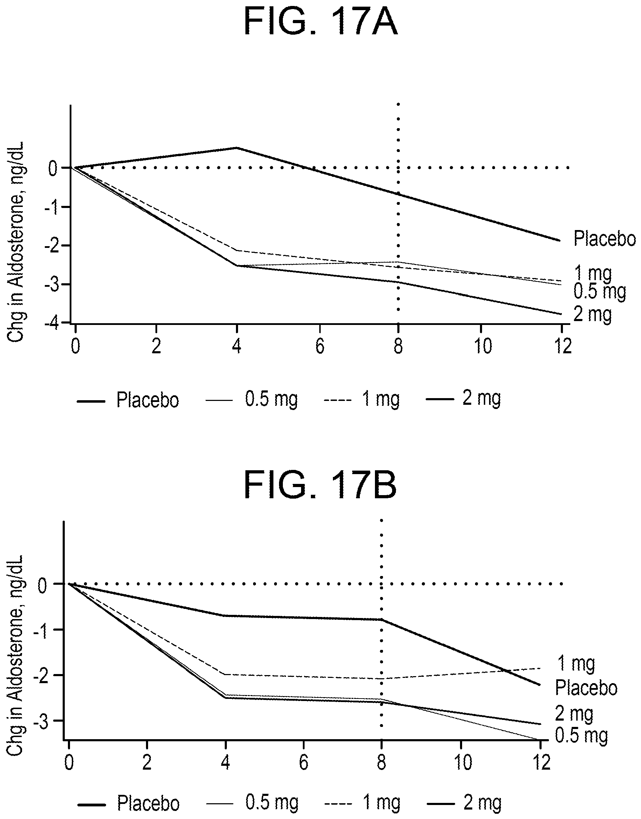

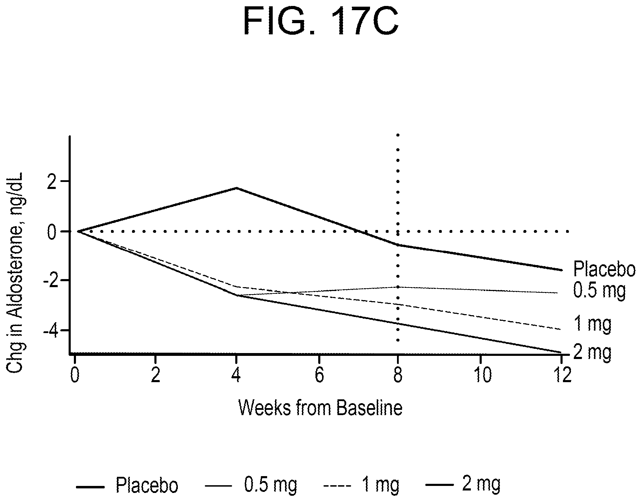

The effect of ethnicity on aldosterone changes was analyzed. Baseline values are shown in Table 32. See, also, FIG. 17.

| **Table 32: Baseline Values** | | | | |
|---|---|---|---|---|
| | | **Amount of Baxdrostat (mg)** | | |
| **Aldosterone, ng/dL** | **Placebo** | **0.5** | **1** | **2** |
| **Total** | 6.5 (5.4) | 6.8 (4.4) | 7.0 (6.8) | 6.9 (5.6) |
| **Hispanic** | 7 (6.3) | 7.9 (4.6) | 8.4 (8.4) | 7.6 (5.9) |
| **non-Hispanic** | 6.1 (4.3) | 5.1 (3.4) | 5.7 (4.6) | 6.9 (5.6) |

### Plasma Renin Activity by Ethnicity

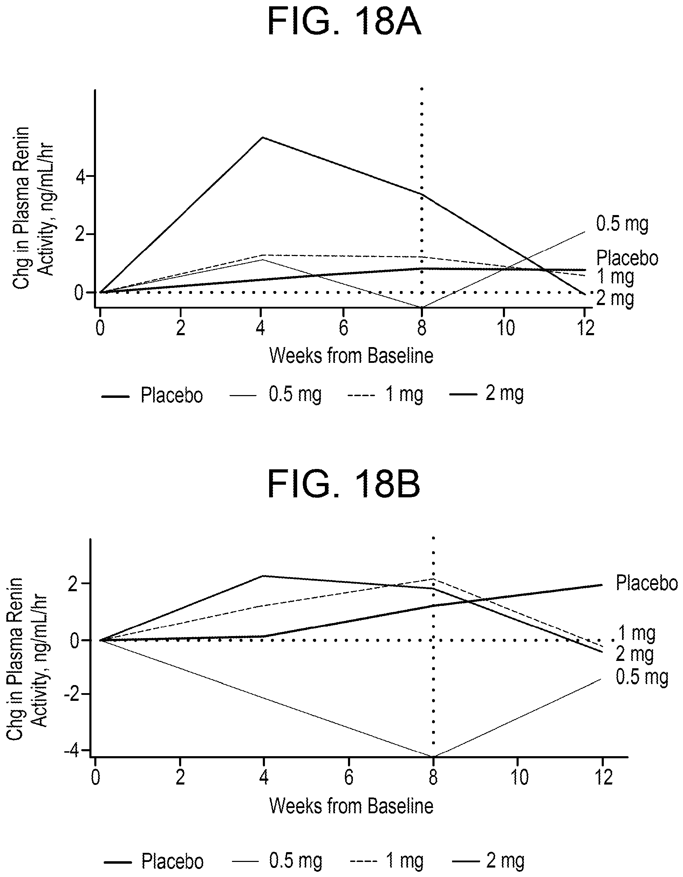

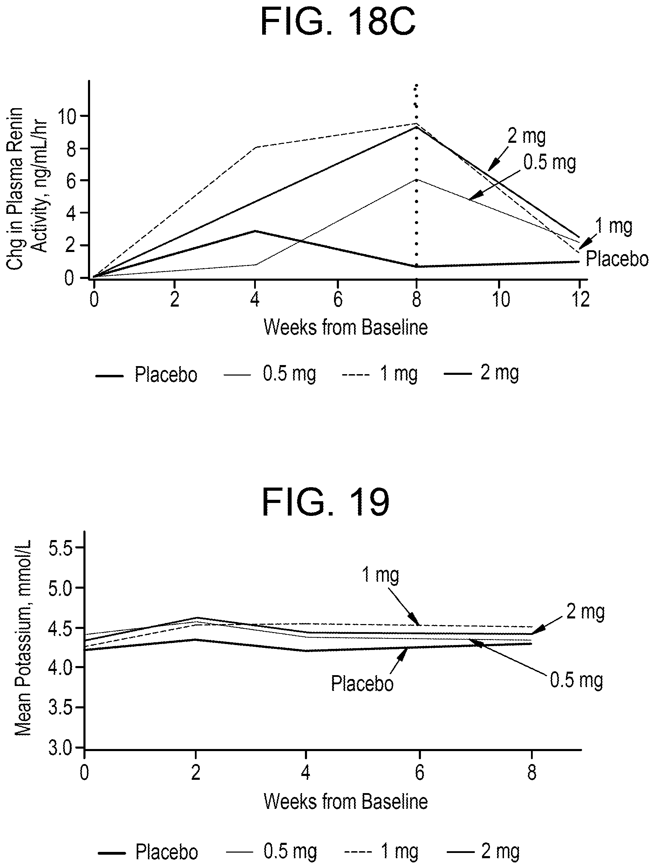

The effect of ethnicity on plasma renin activity (PRA) was analyzed. Baseline values are shown in Table 33. See, also FIG. 18.

| **Table 33: Baseline Values** | | | | |
|---|---|---|---|---|
| | | **Amount of Baxdrostat (mg)** | | |
| **PRA, µg/L/h** | **Placebo** | **0.5** | **1** | **2** |
| **Total** | 5.5 (8.7) | 8.0 (13.6) | 4.4 (6.0) | 5.6 (9.5) |
| **Hispanic** | 5.3 (8.4) | 9 (15.5) | 4.4 (6.1) | 5.8 (10.6) |
| **non-Hispanic** | 5.2 (8.8) | 6.4 (9.8) | 4.2 (6) | 5.1 (7.7) |

### Safety Results

The safety results shows that baxdrostat delivers a compelling safety profile. There were two treatment emergent SAEs (2 patients) and neither were deemed related to baxdrostat. An 81 year old man died from and unrelated SAE of acute respiratory failure following a diagnosis of COVID-19 30 days after his last dose of baxdrostat. See, Table 34.

| **Table 34: Serious Adverse Events and Treatment Emergent Adverse Events** | | | | | | | | | | | | | | | |
|---|---|---|---|---|---|---|---|---|---|---|---|---|---|---|---|
| | **Placebo n = 64** | | | **0.5 mg baxdrostat n = 63** | | | **1 mg baxdrostat n = 62** | | | **2 mg baxdrostat n = 60** | | | **Total n = 249** | | |
| | **%** | **n** | **e** | **%** | **n** | **e** | **%** | **n** | **e** | **%** | **n** | **e** | **%** | **n** | **e** |
| **SAEs** | **0.0** | **0** | **0** | **0.0** | **0** | **0** | **1.6** | **1** | **1** | **1.7** | **1** | **1** | **0.8** | **2** | **2** |
| TEAEs | 21.9 | 14 | 26 | 28.6 | 18 | 30 | 30.6 | 19 | 42 | 23.3 | 14 | 27 | 26.1 | 65 | 115 |
| Drug-Related | 6.3 | 4 | 4 | 6.3 | 4 | 6 | 9.7 | 6 | 10 | 10.0 | 6 | 8 | 8.0 | 20 | 28 |
| AESI | 1.6 | 1 | 1 | 1.6 | 1 | 1 | 1.6 | 1 | 1 | 6.7 | 4 | 4 | 2.8 | 7 | 7 |
| Leading to dosing discontinuation | 1.6 | 1 | 1 | 1.6 | 1 | 1 | 3.2 | 2 | 2 | 3.3 | 2 | 2 | 2.4 | 6 | 6 |
| Leading to study discontinuation | 1.6 | 1 | 1 | 1.6 | 1 | 1 | 0.0 | 0 | 0 | 1.7 | 1 | 1 | 1.2 | 3 | 3 |
| Leading to death | 0.0 | 0 | 0 | 0.0 | 0 | 0 | 0.0 | 0 | 0 | 1.7 | 1 | 1 | 0.4 | 1 | 1 |
| TEAEs - Treatment emergent adverse events; AESI - Adverse events of special interest; SAEs - Serious adverse events; n - Number of subjects; e - Number of events | | | | | | | | | | | | | | | |

There also were no instances of hyperkalemia in a baxdrostat recipient led to study discontinuation and no off-target side effects.

It also was found that the rates of hyperkalemia are low and isolated. There was an overall modest increase in mean potassium of 0.1 mEq/L at the 2 mg dose. See, Tables 35-36 and FIG. 19.

| **Table 35: Mean Change in Serum Potassium From Baseline at Week 8** | | | | |
|---|---|---|---|---|
| | | **Amount of Baxdrostat (mg)** | | |
| | **Placebo** | **0.5** | **1** | **2** |
| Serum Potassium mmol/L, (SD) | 0.08 (0.45) | -0.07 (0.37) | 0.26 (0.47) | 0.12 (0.48) |

| **Table 36: Serum Potassium Levels** | | | | |
|---|---|---|---|---|
| | | **Amount of Baxdrostat (mg)** | | |
| | **Placebo n = 64** | **0.5 n = 63** | **1 n =62** | **2 n =60** |
| # value ≥6.0 | 1 | 0 | **1** | **2** |
| # patients with values ≥6.0 | 1 | 0 | **1** | **2** |
| % patients | 1.6% | 0.0% | 1.6% | 3.3% |

Three patients receiving baxdrostat (~1.6% of the patients receiving baxdrostat) experienced isolated instances of moderately elevated potassium >6 mEq/L. This rate of moderate hyperkalemia was identical to the PBO rate of 1.6%; and compares favorably with previous results of 2.1%. The mean potassium rise of 0.1 mEq/L in the 2 mg dose group was consistent with the small potassium rise observed in the 2 mg group observed earlier. No potassium elevations >6.2 mEq/L were recorded in patients on Baxdrostat, and no patients completed the trial with moderate hyperkalemia. No instances of hyperkalemia led to trial discontinuation among patients in the active Baxdrostat groups.

### (ii) Part 2 Results

Part 2 showed that Baxdrostat (2 mg) was safe and well-tolerated in the 213 patients who entered Part 2. Fourteen patients completing Part 1 did not enter Part 2 - 12 of which did not qualify based on BP <130 mmHg.

In Part 2, background antihypertensive therapy was discontinued following Part 1, and patients took two mg Baxdrostat for four additional weeks. SBP remained stable when compared between the start and the end of the four weeks following withdrawal of background medication and only on 2 mg Baxdrostat. There were two cases of moderate hyperkalemia with K+ >/= 6.0 mEq/L in the 213 patients in Part 2; both patients had stopped diuretic at the start of Part 2 and developed transient moderate hyperkalemia within 2 weeks.

### Safety Profile

All Part 2 patients were presumed to be on 2 mg of baxdrostat. Part 2 results reinforce that baxdrostat at a 2 mg dose delivers a compelling safety profile. See, Table 37.

| **Table 37** | | | | | | | | | | | | | | | | |
|---|---|---|---|---|---|---|---|---|---|---|---|---|---|---|---|---|
| | | **Placebo to 2mg** | | | **0.5 mg to 2 mg** | | | **1 mg to 2 mg** | | | **2 mg** | | | **Total** | | |
| | | **n = 64** | | | **n = 63** | | | **n = 62** | | | **n= 60** | | | **n = 249** | | |
| | | % | n | e | % | n | e | % | n | e | % | n | e | % | n | e |
| **SAEs** | | **3.3** | **2** | **2** | **0.0** | **0** | **0** | **1.8** | **1** | **2** | **0** | **0** | **0** | **1.4** | **3** | **4** |
| TEAEs | | 21.7 | 13 | 24 | 16.7 | 9 | 15 | 21.1 | 12 | 26 | 16.7 | 7 | 9 | 19.2 | 41 | 74 |
| | Drug-Related | 5.0 | 3 | 5 | 5.6 | 3 | 4 | 8.8 | 5 | 6 | 0 | 0 | 0 | 5.2 | 11 | 15 |
| | AESI | 1.7 | 1 | 2 | 0 | 0 | 0 | 1.8 | 1 | 1 | 2.4 | 1 | 1 | 1.4 | 3 | 4 |
| | Leading to dosing discontinuation | 0 | 0 | 0 | 0 | 0 | 0 | 3.5 | 2 | 3 | 0 | 0 | 0 | 0.9 | 2 | 3 |
| | Leading to study discontinuation | 0.0 | 0 | 0 | 0.0 | 0 | 0 | 0.0 | 0 | 0 | 0.0 | 0 | 0 | 0.0 | 0 | 0 |
| | Leading to death | 0.0 | 0 | 0 | 0.0 | 0 | 0 | 0.0 | 0 | 0 | 0.0 | 0 | 0 | 0.0 | 0 | 0 |
| TEAEs - Treatment emergent adverse events; AESI - Adverse events of special interest; SAEs - Serious adverse events; n - Number of subjects; e - Number of events | | | | | | | | | | | | | | | | |

Treatment Emergent SAEs: 4 events/ 3 patients with none deemed related to baxdrostat. There were no instances of hyperkalemia in a Baxdrostat recipient led to study discontinuation. There also were no off-target side effects.

### C. Summary of Results

In summary, the top-line efficacy primary end-point was not met due to differential response of Hispanic/Latino cohort. The Hispanic/Latino cohort represented >50% of the study population. However, pre-specified non-Hispanic analysis resulted in ability to salvage the study/data.

The results were promising in other sub-groups, including the Black/African American patients and non-Hispanic Women.

The overall safety data was good and there was a low hyperkalemia rate.

### Example 10: Study in Patients with Uncontrolled Hypertension and Chronic Kidney Disease

Study 123 is a randomized, double-blind, placebo-controlled, multicenter, parallel group, dose-ranging study to evaluate baxdrostat for the treatment of patients with uHTN and CKD. The primary objective of the study is to evaluate the treatment effect of baxdrostat on SBP compared to placebo at week 26. The secondary objectives are to evaluate the baxdrostat effect on SBP compared to placebo by high or low dosing strategy, to determine the percentage of patients achieving SBP < 130 mmHg, to evaluate the change from baseline in urinary albumin-to-creatinine ratio (UACR), to evaluate the change from baseline in DBP with each dosing strategy and to evaluate the change from baseline in eGFR after 26 weeks of treatment. The safety objectives are to evaluate the vital signs, standing BP and heart rate, physical examinations, electrocardiography, weight measurement, and clinical laboratory evaluations, including standard safety chemistry panel, hematology, coagulation, and urinalysis. Approximately 300 patients are planned to be enrolled in 70 clinical sites in the U.S.

Male and female adult patients with uHTN and mild to severe CKD are to be recruited in the study. Patients with uHTN are defined as patients taking a stable dose of antihypertensive medication ACEI/ARB and have seated office SBP of ≥ 140 mmHg or ≥ 130 mmHg if diabetic. Patients with a mild-to-severe CKD are defined as having an eGFR (based on the CKD-EPI equation) of 25 to 75 mL/min/1.73 m² and a UACR ≥200 mg/g (≥22.6 mg/mmol) in at least 2 out of 3 measurements based on first urine collected in the morning on consecutive days during the Screening Period. Patients taking an MRA must agree to cease dosing of the MRA to be eligible to start a 2-week run-in period in which patients will take once daily single-blind placebo tablets while continue the background antihypertensive medication. Patients who remain eligible are to be randomized to one of three treatment arms of placebo, low dose strategy and high dose strategy and start a 26-week treatment period.

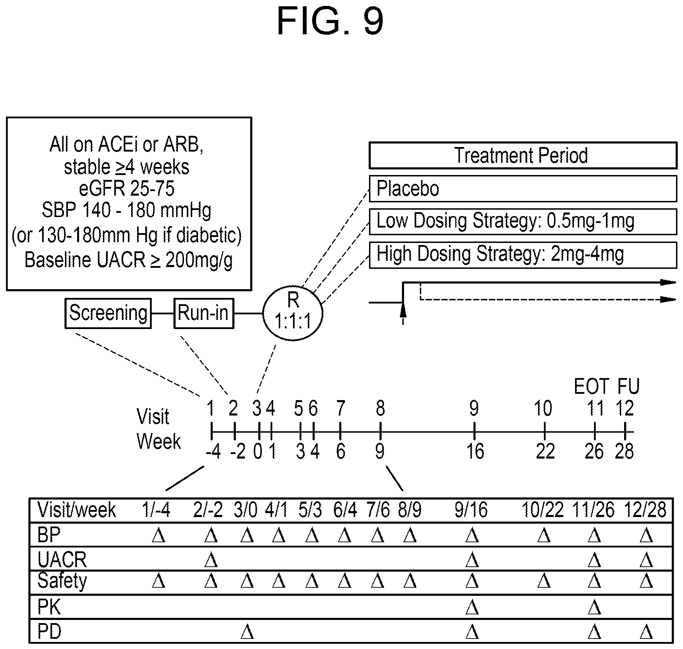

Patients assigned to the low dose strategy arm are provided with baxdrostat 0.5 mg for once daily dosing. The dose level may be up-titrated to 1 mg at week 3 if a patient does not experience hyperkalemia, hyponatremia, or a significant decline in renal function based on laboratory testing of samples drawn at week 1 and if the average seated SBP is ≥ 130 mmHg at week 3. The dose may be down-titrated at 6 weeks after randomization if a patient experience hyperkalemia, hyponatremia, or declining renal function based on laboratory testing of sample drawn at week 5. No further dose titration is permitted after 6 weeks. The study design is illustrated in FIG. 9.

### Example 11

### A. Study Design

### (i) Overall Design

This is a Phase III, multicentre, randomised, double-blinded, placebo-controlled, parallel group study to evaluate the safety, tolerability and effect of 1 or 2 mg baxdrostat versus placebo, administered QD orally, on the reduction of SBP in approximately 720 participants aged ≥ 18 years with HTN, despite a stable regimen of 2 antihypertensive agents at baseline, one of which is a diuretic (uHTN); or ≥ 3 antihypertensive agents at baseline, one of which is a diuretic (rHTN).

Consenting participants will be screened within 4 weeks and will subsequently enter a 2-week single-blind run-in period with placebo. Thereafter, participants will be sequentially distributed across 2 cohorts (Cohort 1 and Cohort 2) and randomised in a 1:1:1 ratio to receive 1 of the following 3 treatments QD, during a 12-week double-blind period:
- 2 mg baxdrostat
- 1 mg baxdrostat
- Placebo

The randomisation will be stratified by HTN at baseline (uHTN, rHTN) and by siSBP at baseline (< 145 mmHg, ≥ 145 mmHg).

After participants in both cohorts complete the 12-week double-blind period:
- Participants who complete treatment with 2 mg baxdrostat will be randomized in a 1:0 ratio to receive 2 mg baxdrostat QD during a 12-week open-label period.
- Participants who complete treatment with 1 mg baxdrostat will be randomized in a 4:1 ratio to receive either 2 mg baxdrostat QD during a 12-week open-label period (from Week 12 to Week 24) or to receive standard-of-care. The standard-of-care period will last 40 weeks (from Week 12 to Week 52) for participants in Cohort 1, and 12 weeks (from Week 12 to Week 24) for participants in Cohort 2.
- Participants who received placebo will be randomized in a 1:4 ratio to receive either 2 mg baxdrostat QD during a 12-week open-label period (from Week 12 to Week 24) or to receive standard-of-care.

After participants complete the 2 mg baxdrostat 12-week open-label period:
- Participants in Cohort 1 (who complete treatment with baxdrostat 2 mg) will be randomised for a third time, in a 2:1 ratio, to either continue receiving 2 mg baxdrostat QD or to receive placebo QD for an 8-week double-blind RWD period. The randomisation will be stratified by baseline HTN (uHTN, rHTN) and by siSBP at RWD randomisation (< 130 mmHg, ≥ 130 mmHg). Participants who complete the RWD period will then enter an open-label period where they will receive 2 mg baxdrostat QD for 20 weeks, followed by a 2-week safety follow-up period.
- Participants in Cohort 2 will start the 2-week safety follow-up period.

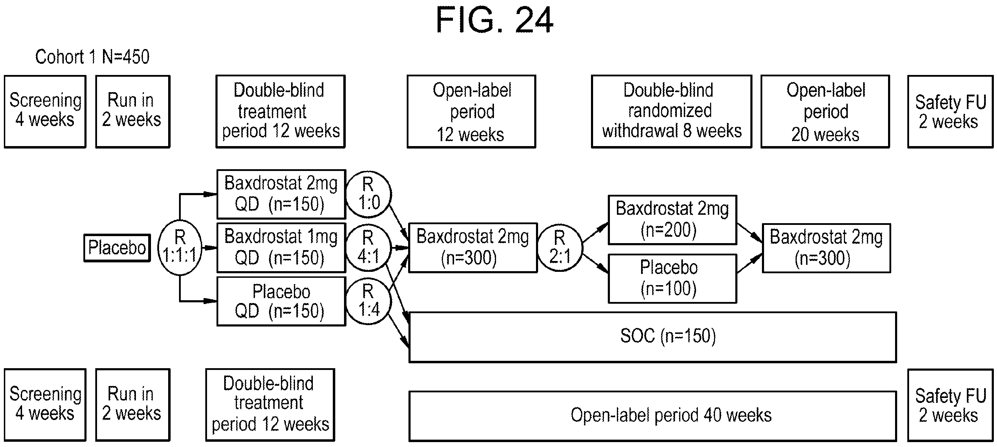

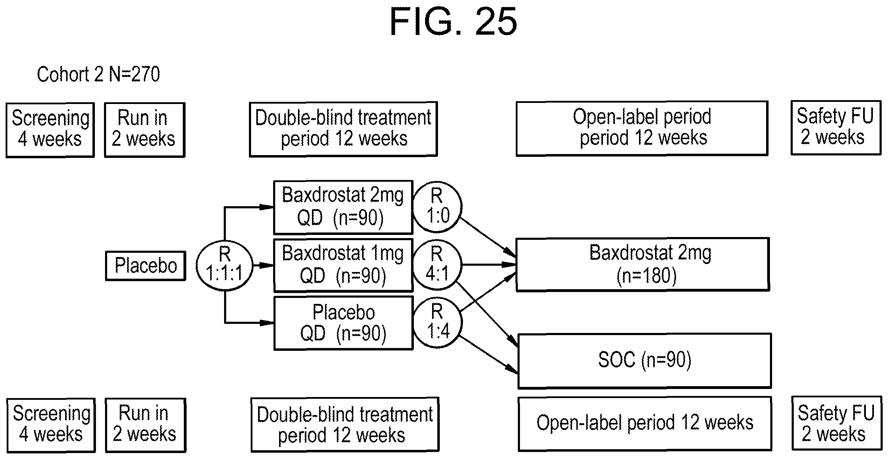

A study diagram for both cohorts is presented in FIGs. 24 and 25. If at any time during the study a participant reports K⁺ levels > 5.5 mEq/L, this laboratory analysis should be repeated within 72 hours. The study is planned to be conducted globally.

### (ii) Screening

All participants that consent to be involved in the study will be screened within 4 weeks, prior to start the run-in period.

### (iii) Placebo Single-Blind Run-in Period

Eligible participants who complete the Screening Visit procedures, will enter a 2-week single-blind run-in period and receive placebo in addition to any existing background antihypertensive mediation.

The objective of this period is to decrease the placebo effect after baseline and to ensure that the participants have a stable background antihypertensive medication.

### (iv) Double-Blind Placebo-Controlled Treatment Period

Approximately 720 participants, who meet the study Inclusion Criteria, will be sequentially distributed across 2 cohorts (approximately 450 participants in Cohort 1 and 270 in Cohort 2). Participants in each cohort will be randomised in a 1:1:1 ratio to receive one of the following 3 study treatments QD: 2 mg baxdrostat, 1 mg baxdrostat or placebo. Participants will receive this treatment for 12 weeks (from Week 0 to Week 12).

During this period, participants should remain on their background antihypertensive medication regimen and dose. Doses of background antihypertensive medication should not be changed during this period unless participants experience SBP < 100 mmHg with symptoms of hypotension.

### (v) Open-Label Treatment Period

All participants in Cohort 1 and in Cohort 2 who complete the 12-week double-blind treatment period with 2 mg baxdrostat QD will be randomised at the start of the open-label period in a 1:0 ratio, to continue receiving 2 mg baxdrostat QD for 12 weeks (from Week 12 up to Week 24).

Participants in Cohorts 1 and 2 who received 1 mg baxdrostat QD or placebo QD during the 12-week double-blind period, will be randomised in a 4:1 or 1:4 ratio, respectively, to receive either 2 mg baxdrostat or standard-of-care.

### (vi) Baxdrostat

All participants randomised to 2 mg baxdrostat at Week 12 will receive 2 mg baxdrostat in an open-label manner for 12 weeks (from Week 12 to Week 24), in addition to any existing background antihypertensive medication, which will be subject to change during this open-label period (except for the addition of MRAs and potassium-sparing diuretics, which are not permitted).

Approximately 300 participants in Cohort 1 and 180 in Cohort 2 will receive 2 mg baxdrostat during this period.

Participants with hyperkalaemia will also follow instructions as per treatment discontinuation criteria and will follow the local standard-of-care.

### (vii) Standard-of-Care

Approximately 150 participants in Cohort 1 and 90 in Cohort 2 will be randomised to receive standard-of-care in an open-label manner. Participants in Cohort 1 will receive this treatment for 40 weeks (from Week 12 to Week 52; see FIG. 24.) and participants in Cohort 2 will receive this treatment for 12 weeks (from Week 12 up to Week 24; FIG. 25). Upon completing this period of standard-of-care treatment, and prior to completing the overall study, participants will enter a 2-week safety follow-up period.

### (viii) Randomized Withdrawal Double-Blind Period - Only Participants in Cohort 1

All participants, in Cohort 1, who have completed the 12-week open-label treatment period on 2 mg baxdrostat will be randomised again at Week 24, in a 2:1 ratio, to either continue receiving 2 mg baxdrostat QD or to receive placebo QD for an 8-week double-blind withdrawal period (from Week 24 to Week 32), and stratified by baseline HTN (uHTN, rHTN) and SBP at RWD randomisation (≤ 130 mmHg, > 130 mmHg). Approximately 200 participants will be randomised to 2 mg baxdrostat and 100 participants to placebo.

During this period, participants should remain on the same background antihypertensive medication regimen and dose they receive at the start of this period. Doses of background antihypertensive medication should not be changed unless participants experience SBP < 100 mmHg with symptoms of hypotension.

Mineralocorticoid receptor antagonists and potassium-sparing diuretics are prohibited during this period.

### (ix) Second Open-Label Period in Cohort 1

Participants in Cohort 1 who complete the 8-week RWD double-blind period will enter this 20-week open-label period (Week 32 to Week 52) to receive 2 mg baxdrostat QD, in addition to any existing background antihypertensive medication. During this period, participant's background therapy can be changed, but MRAs or potassium-sparing diuretics are not allowed.

### (x) Safety Follow-up

All study participants will complete a 2-week safety follow-up period prior to finalising the overall study. Participants in Cohort 1 will enter this last study period after finalising the 20-week open-label period, or the 40-week standard-of-care period, and participants in Cohort 2 will enter this period after finalising the 12-week open-label period.

### (xi) End-of-study Definition

For the purpose of Clinical Trial Transparency, the definition of the end of the study differs under FDA and EU regulatory requirements:
- European Union requirements define study completion as the last visit of the last subject for any protocol related activity.
- Food and Drug Administration requirements defines two completion dates:
   - Primary Completion Date - the date that the final participant is examined or receives an intervention for the purposes of final collection of data for the primary outcome measure, whether the clinical study concluded according to the pre-specified protocol or was terminated. In the case of clinical studies with more than one primary outcome measure with different completion dates, this term refers to the date on which data collection is completed for all the primary outcomes.
   - Study Completion Date - the date the final participant is examined or receives an intervention for purposes of final collection of data for the primary and secondary outcome measures and AEs (for example, last participant's last visit), whether the clinical study concludes according to the pre-specified protocol or is terminated.

A participant is considered to have completed the study if he/she has completed all phases of the study including the last visit or the last scheduled procedure. The end of the study is defined as the date of the last visit of the last participant in the study or last scheduled procedure for the last participant in the study globally.

### B. Study Population

### (i) Inclusion Criteria

Participants are eligible to be included in the study only if all the following criteria apply:

### Age

1. Male or female participants must be ≥ 18 years old, at the time of signing the informed consent.

### Type of Participant and Disease Characteristics

2. Mean siSBP on AOBPM ≥ 140 mmHg and < 170 mmHg at Screening.

3. Fulfil at least 1 of the following 2 criteria:
(a) Participants in the uHTN subpopulation: have a stable regimen of 2 antihypertensive medications, from different therapeutic classes (at least one should be a diuretic), at maximum tolerated dose, for at least 4 weeks prior to Screening. Beta blockers used to treat other conditions (i.e., migraine, HF, coronary artery disease) should not be counted as an antihypertensive medication.
(b) Participants in the rHTN subpopulation: have a stable regimen of ≥ 3 antihypertensive medications, from different therapeutic classes (at least one should be a diuretic), at maximum tolerated dose, for at least 4 weeks prior to Screening. Beta blockers used ions to treat other conditions (i.e., migraine, HF, coronary artery disease) should not be counted as an antihypertensive medication.

4. Demonstrated good adherence to the prescribed antihypertensive medications by performing DOT during Visit 2.

5. Estimated glomerular filtration rate ≥ 45 mL/min/1.73m² at Screening

6. Serum potassium (K⁺) level ≥ 3.5 and < 5.0 mmol/L at Screening.

7. Morning cortisol levels (measured at 08:00 am ± 2 hours) > 3 µg/dL.

### (ii) Randomization Criteria

These randomisation criteria only apply to the initial randomisation of the study. Participants are eligible to be randomised to a treatment group only if all the following criteria apply: 1. Mean siSBP on attended office AOBPM of ≥ 135 mmHg at baseline (end of the run-in period.

2. Have an 80 to 120% adherence to placebo during the run-in period, based on pill counts on the morning of randomization.

3. Have no change in background therapy regimen and dose consisting of either 2 antihypertensive medications (at least one should be a diuretic) for participants in the uHTN subpopulation, or ≥ 3 antihypertensive medications (at least one should be a diuretic) for participants in the rHTN subpopulation, for at least 4 weeks prior to randomization. Beta blockers used to treat other conditions (i.e., migraine, HF, coronary artery disease) should not be counted as an antihypertensive medication.

4. Demonstrated good adherence to the prescribed antihypertensive medications by performing DOT during Visit 2.

### (iii) Exclusion Criteria

Participants are excluded from the study if any of the following criteria apply:

### Medical Conditions

1. Mean siSBP ¹ on attended office AOBPM ≥ 170 mmHg at randomization.
2. Mean seated DBP ²on attended office AOBPM ≥ 110 mmHg at randomization.
3. Current or prior treatment (within the 4 weeks before Screening) with angiotensin-receptor blockers and ACEIs (both taken simultaneously).
4. Serum sodium level < 135 mmol/L at Screening.
5. Has the following known secondary causes of hypertension: renal artery stenosis, uncontrolled or untreated hyperthyroidism, uncontrolled or untreated hypothyroidism, pheochromocytoma, Cushing's syndrome, aortic coarctation.
6. New York Heart Association functional HF class IV at Screening.
7. Medical history of stroke, acute coronary syndrome, hypertensive encephalopathy, or hospitalization for HF within 6 months prior to Screening.
   1 siSBP should be taken after > 5 min in a seated position (in a chair feet on floor, back supported).
   2 siDBP should be taken after > 5 min in a seated position (in a chair feet on floor, back supported).
8. Planned percutaneous coronary intervention/coronary artery bypass grafting or percutaneous coronary intervention/coronary artery bypass grafting done within 6 months prior to Screening.
9. Known current severe left ventricular outflow obstruction, such as obstructive hypertrophic cardiomyopathy and/or severe aortic valvular disease.
10. Left bundle branch block and any cardiac arrhythmia requiring treatment.
11. Persistent atrial fibrillation.
12. Known severe hepatic impairment, defined as Child-Pugh Class C, based on records that confirm documented medical history.
13. Uncontrolled diabetes with HbA1c > 10.0% (86 mmol/mol) at Screening.
14. Baseline QTcF > 470 msec.
15. Family history of Long QT syndrome.
16. Heart rate < 45 or > 110 beats/min in a resting position.
17. Participants suspected to have severe cardiac hypertrophy.
18. Participants who are pregnant or breastfeeding.
19. Participants with a diagnosis of adrenal insufficiency.
20. Any of the following related to COVID-19 infection:
   (a) Suspected or confirmed COVID-19 infection within the last 4 weeks prior to Screening or at randomization.
   (b) Hospitalization for COVID-19 within the last 12 weeks prior to Screening.

### Prior/Concomitant Therapy

21. Prior medical treatment with any MRAs, antiarrhythmic medications, or potassium-sparing diuretic used within 4 weeks prior to Screening.

22. Treatment with potassium binders within 2 months prior to Screening.

23. Is expected to receive or is receiving any of the exclusionary drugs such as strong inducers of cytochrome P450 (CYP) 3A, chronic (taken more than 3 times a week for more than 3 months) use of NSAIDs, MRAs and/or chronic use of systemic steroids.

24. Drugs that prolong QT should be avoided, if possible, and if there are other alternatives without the QT liability, these should be preferred.

25. Current or prior treatment within 6 months prior to Screening with cytotoxic therapy.

26. Treatment with K⁺ supplements are not prohibited but should be continuously assessed and monitored throughout the trial.

### Prior/Concurrent Clinical Study Experience

27. Known hypersensitivity to baxdrostat or drugs of the same class or any of its excipients.

28. Participation in another clinical study with an investigational product administered in the 3 months prior to randomization in this study.

### Other

29. Participants working shifts (i.e., shifts that comprise working hours at different times on different days).

Participants can take the study intervention with or without food.

### C. Study Intervention and Concomitant Therapy

### (i) Study Intervention Administered

Study treatments to be administered and investigated in this study are described in Table 38.

| **Table 38: Study Intervention** | | | |
|---|---|---|---|
| **Treatment name** | **2 mg Baxdrostat Treatment** | **1 mg Baxdrostat Treatment** | **Placebo Treatment** |
| **Intervention name** | Baxdrostat | Baxdrostat | Placebo |
| **Tvpe** | Drug | Drug | Drug |
| **Dose formulation** | Tablet | Tablet | Tablet |
| **Unit dose strengths** | 2 mg per tablet | 1 mg per tablet | N/A |
| **Dosage levels** | 2 mg baxdrostat once daily | 1 mg baxdrostat once daily | Placebo once daily |
| **Route of administration** | Oral | Oral | Oral |
| **Regimen** | One tablet per day, preferably in the morning | One tablet per day, preferably in the morning | One tablet per day, preferably in the morning |
| **Packaging and labelling** | Study Intervention will be provided in blister packs containing IMP tablets. Each blister pack will be labelled as required per country requirement | Study Intervention will be provided in blister packs containing IMP tablets. Each blister pack will be labelled as required per country requirement | Study Intervention will be provided in blister packs containing IMP tablets. Each blister pack will be labelled as required per country requirement |
| The external label and appearance of all tablets, for all study treatments, will be the same in order to keep the blind. | | | |

### (ii) Background Antihypertensive Medication

The stable regimen of background antihypertensive medication that participants receive when they are enrolled in this study and which they will continue receiving after they are randomised to study treatment. The background antihypertensive medication can be changed during the open-label periods, except for the use of MRAs and potassium-sparing diuretics.

### (iii) Standard-of-Care Treatment

The standard-of-care treatment received by participants who are randomised into this study period). The standard-of-care treatment can be changed during the standard-of-care period. Assignment to Study Intervention

All participants who meet all the eligibility criteria will be distributed sequentially in both cohorts (Cohort 1 and Cohort 2) and will be centrally assigned to randomised study intervention using an automated IRT/RTSM system that will allocate participants into 1 of 3 different treatment groups. There will be a total of 3 randomisation stages during the study that will be performed using the IRT/RTSM system (see FIG. 24):
- A first randomization will be performed after the run-in period to allocate approximately 720 participants (450 in Cohort 1 and 270 in Cohort 2) into receiving either 2 mg baxdrostat QD, 1 mg baxdrostat QD or placebo QD, using a 1:1:1 ratio. The randomization will be stratified by baseline HTN (uHTN, rHTN) and baseline siSBP (< 145 mmHg, ≥ 145 mmHg).
- The second randomization will be performed after the initial 12-week double-blind period. Approximately 480 participants (300 in Cohort 1 and 180 in Cohort 2) will be randomized to 2 mg baxdrostat and 240 participants (150 in Cohort 1 and 90 in Cohort 2) will be randomized to standard-of-care. The randomization is as follows:
   - Participants who received 2 mg baxdrostat during the first 12 weeks of treatment will be randomized in a 1:0 ratio to continue receiving 2 mg baxdrostat QD.
   - Participants who received 1 mg baxdrostat or placebo during the first 12 weeks of treatment will be randomized in a 4:1 or 1:4 ratio, respectively, to receive either 2 mg baxdrostat QD or standard-of-care.
- Only participants in Cohort 1: after participants complete the 12-week open-label period with 2 mg baxdrostat QD (approximately 300 participants in Cohort 1), participants will be randomized for a third time, in a 2: 1 ratio, to either continue receiving 2 mg baxdrostat QD or to receive placebo QD. These participants will be stratified by baseline HTN (uHTN, rHTN) and by siSBP at RWD randomization (≤ 130 mmHg, > 130 mmHg). Approximately 200 participants will be randomized to 2 mg baxdrostat QD and 100 to placebo QD.

Randomisation will be performed in balanced blocks of fixed size. The randomisation codes will be computer generated and loaded into the IxRS database.

All randomisations will be performed directly using the IRT/RTSM system. No randomisation codes are to be reused.

The number of randomised participants in each HTN subpopulation (uHTN / rHTN) will be monitored to ensure a minimum of approximately 40% participants being enrolled in the uHTN subpopulation or the rHTN subpopulation. Randomisation may be capped (i.e., no more participants can be randomised in a specific subpopulation) if the predetermined limit is reached, to avoid over- or under-representation.

### (iv) Dose Modification

Investigational medicinal product dose modification is not permitted in this study.

### (v) Prohibited Concomitant Medications

The medications and supplements listed below are prohibited during this study, except in the standard-of-care treatment group.
- Simultaneous use of angiotensin-receptor blockers and ACEIs are not allowed during the study. Participants should be on stable dose of either drug class; any changes to drug dose or class must be closely monitored.
- Potassium-sparing diuretics (e.g., amiloride, triamsterene) and direct renin inhibitor (e.g., Aliskiren).
- Potassium binders (prohibited at Screening but can be started as a corrective action during the study).
- Tacrolimus, calcineurin inhibitors and cyclosporin. Topical / inhaled immunosuppressants is permitted.
- Mineralocorticoid receptor antagonists or aldosterone antagonists (e.g., eplerenone, finerenone, spironolactone).
- Systemic corticosteroids at any dose (e.g., prednisone, prednisolone, dexamethasone [topical and inhaled steroids are allowed]).
- Treatment with K⁺ supplements are not prohibited but should be continuously assessed and monitored throughout the trial.
- Chronic NSAID use. Occasional NSAID usage is permitted, however closer monitoring is warranted at these times.
- Strong CYP3A4 inducers (e.g., apalutamide, avasimibe, carbamazepine, enzalutamide, lumacaftor, mitotane, phenytoin, rifampin, rifapentine, St. John's wort).

### (vi) Blood Pressure Measurements

### Seated Blood Pressure Measurements

Seated BP measurements will be recorded using standardised automated BP machines and using the following standardised procedures:
- Participants should not exercise, smoke, or consume caffeinated beverages or food 30 minutes prior to assessment of AOBPM.
- On visits, when study intervention is administered at site, BP will be assessed pre-dose.
- Blood pressure measurements should be obtained prior to ECG recordings.
- For measuring BP by AOBPM, the following additional standardized procedures are recommended:
   ∘ The participant should be seated for at least 5 minutes in the examination room with the back supported, feet flat on the floor and the measurement arm supported so that the midpoint of the manometer cuff is at heart level.
   ∘ A designated AOBPM device will be provided to each clinical site and must be used for all study-related measurements.
   ∘ An appropriately sized cuff should be used with the bladder centered over the brachial artery.
   ∘ The cuff size and arm used for the measurement should be recorded.
   ∘ The arm with the higher mean BP value at Screening should be used for Screening and subsequent BP measurements.
   ∘ All BP measurements should be obtained at approximately the same time of the day when the Screening measurements were obtained.
   ∘ Three seated BP measurements (each approximately 1 minute apart) should be obtained using the same arm and the AOBPM device at each clinical site visit. Mean seated BP is defined as the average of the last 2 seated BP measurements of the total 3 measurements, at any single clinical site visit.
   ∘ If the difference between the last 2 SBP measurements is > 20 mmHg, additional readings should be performed (up to 6 additional measurements; performed as 3 seated BP readings measured twice). If the difference between the last 2 SBP measurements of these 2 additional triple measurements is still > 20 mmHg, the average of the last two measurements will be used.
   ∘ Right after the seated BP has been determined, orthostatic BP will be measured, only at the visits specified.

### Orthostatic Blood Pressure Measurements

Orthostatic BP measurements include standing BP and will be measured using the AOBPM. The following standardised procedures are recommended:
- After the siSBP has been determined, the participant will be asked to stand and after approximately 1 minute a single standing BP measurement will be obtained, as required.
- Participants should not exercise, smoke, or consume caffeinated beverages or food 30 minutes prior to assessment of AOBPM.
- On visits, when study intervention is administered at site, BP will be assessed pre-dose.
- Blood pressure measurements should be obtained prior to ECG recordings.
- For measuring BP by AOBPM, the following additional standardized procedures are recommended:
   ∘ A designated AOBPM device will be provided to each clinical site and must be used for all study-related measurements.
   ∘ An appropriately sized cuff should be used with the bladder centered over the brachial artery.
   ∘ The cuff size and arm used for the measurement should be recorded.
   ∘ The arm with the higher mean BP value at Screening should be used for Screening and subsequent BP measurements.
   ∘ All BP measurements should be obtained at approximately the same time of the day when the Screening measurements were obtained.

### (vi) Pharmacokinetics

Pre-dose blood samples will be collected for measurement of plasma concentrations of baxdrostat. All PK samples will be collected pre-dose; thus, participants should be reminded not to take their study medication at home on the day of their clinic visit as they will receive study medication in clinic on these days.

For participants who are discontinued from investigational product due to hyperkalaemia, a PK sample should be collected at the EoT Visit.

The timing of sampling may be altered during the study based on newly available data (e.g., to obtain data closer to the time of peak or trough matrix concentrations) to ensure appropriate monitoring.

Plasma samples will be used to analyse the PK of baxdrostat. Samples collected for the analysis of baxdrostat concentration in plasma may also be used to evaluate safety or efficacy aspects related to concerns arising during or after the study.

### (viii) Pharmacodynamics

- Olink^{®}

### C. Objectives/Endpoints

| **Table 39: Objectives and Endpoints** | |
|---|---|
| **Primary** | |
| To assess the effect of 2 mg baxdrostat versus placebo on seated SBP at week 12. | Change from baseline in seated SBP at Week 12. |
| To assess the effect of 1 mg baxdrostat versus placebo on seated SBP at week 12. | Change from baseline in seated SBP at Week 12. |

| **Safety** | |
|---|---|
| To assess the safety and tolerability of baxdrostat versus placebo over up to 52 weeks. | Adverse events, SAEs and DAEs. |
| | Vital signs (BP, pulse rate). |
| | Safety investigations and laboratory tests (ECG, clinical chemistry, haematology). |
| | AESIs (hyperkalaemia, hyponatraemia, and hypotension). |
| | Treatment-emergent MACEs, defined as cardiovascular death, non-fatal myocardial infarction, and non-fatal stroke. |
| | Treatment-emergent MACE-plus, defined as cardiovascular death, non-fatal myocardial infarction, and non-fatal stroke and hospitalisation for heart failure. |

| **Secondary** | |
|---|---|
| To assess the effect of 2 mg baxdrostat versus placebo on seated SBP at 8 weeks after randomized withdrawal. | Change from RWD baseline (Week 24) in seated SBP at Week 32. |
| To assess the effect of 2 mg baxdrostat versus placebo on seated SBP at Week 12 in the rHTN subpopulation. | Change from baseline in seated SBP at Week 12. |
| To assess the effect of 2 mg baxdrostat versus placebo on seated DBP at Week 12. | Change from baseline in seated DBP at Week 12. |
| To assess the effect of 2 mg baxdrostat versus placebo on achieving seated SBP < 130 mmHg at Week 12. | Achieving seated SBP < 130 mmHg at Week 12. |
| To assess the effect of 1 mg baxdrostat versus placebo on seated SBP at Week 12 in the rHTN subpopulation. | Change from baseline in seated SBP at Week 12. |
| To assess the effect of 1 mg baxdrostat versus placebo on seated DBP at Week 12. | Change from baseline in seated DBP at Week 12. |
| To assess the effect of 1 mg baxdrostat versus placebo on achieving seated SBP < 130 mmHg at Week 12. | Achieving seated SBP < 130 mmHg at Week 12. |
| To assess the effect of treatment with baxdrostat 2 mg vs placebo on ambulatory 24-hour average SBP at Week 12. | The change from baseline in the mean ambulatory 24-hour SBP at Week 12 as measured by ABPM. |
| To assess the effect of treatment with baxdrostat 1 mg vs placebo on ambulatory 24-hour average SBP at Week 12. | The change from baseline in the mean ambulatory 24-hour SBP at Week 12 as measured by ABPM. |

| **Exploratory** | |
|---|---|
| To assess the effect of treatment with 2 mg baxdrostat versus placebo on seated SBP at 4 weeks after randomized withdrawal. | Change from RWD baseline (Week 24) in seated SBP at Week 28. |
| To assess the effect of treatment with baxdrostat 2 mg and baxdrostat 1 mg vs placebo on ambulatory 24-hour BP at Week 12. | The change from baseline in ambulatory night-time average SBP at Week 12 as measured by ABPM. |
| | The change from baseline in ambulatory day-time average SBP at Week 12 as measured by ABPM. |
| | Achieving ambulatory 24-hour average SBP of < 130 mmHg at Week 12 as measured by ABPM. |
| | The change from baseline in ambulatory 24-hour average DBP at Week 12 as measured by ABPM. |
| To evaluate the pharmacokinetics of baxdrostat. | Plasma concentrations of baxdrostat. |

### D. Primary estimand

The primary estimand is described by the following attributes:
- Population: participants with uHTN or rHTN, as defined by the inclusion and exclusion criteria. Implemented through the use of the FAS.
- Endpoint: change from baseline in siSBP at Week 12.
- Treatment condition: 2 mg baxdrostat QD and stable regimen of background antihypertensive medication or 1 mg baxdrostat QD and stable regimen of background antihypertensive medication.
- Intercurrent events: the intercurrent event of treatment discontinuation will be handled via a treatment policy strategy, i.e., to ascertain the treatment effect regardless of treatment discontinuation. The intercurrent event of rescue therapy will be handled via a hypothetical strategy, i.e., to ascertain the treatment effect if rescue therapy were not initiated.
- Population-level summary: difference in mean change from baseline between baxdrostat (2 mg or 1 mg separately) versus placebo over a period of up to 52 weeks.

## Claims

1. (R)-Compound 1 for use in the treatment of uncontrolled or treatment-resistant hypertension in a human in need of treatment thereof, comprising administering during a treatment period, 1 mg/day or 2 mg/day of (R)-Compound 1 to the human: 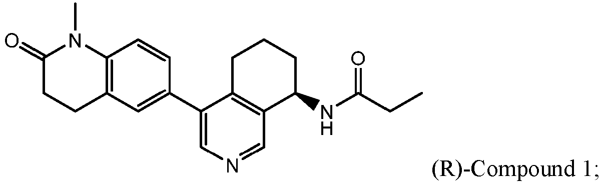 wherein the human is on a stable background anti-hypertensive regimen prior to the administration and during the treatment period.

2. (R)-Compound 1 for use of claim 1, wherein administration of 2 mg/day of the (R)-Compound 1 results in a decrease in the human's mean seated systolic blood pressure (siSBP) as compared to baseline, at or after a 12-week treatment period, as compared to a placebo.

3. (R)-Compound 1 for use of claim 1, wherein administration of 1 mg/day of the (R)-Compound 1 results in a decrease in the human's mean seated systolic blood pressure (siSBP) as compared to baseline, at or after a 12-week treatment period, as compared to a placebo.

4. (R)-Compound 1 for use of any one of claims 1-3, wherein (a) the hypertension is uncontrolled hypertension, the stable background anti-hypertensive regimen comprises at least two antihypertensive medications, and one antihypertensive medication is a diuretic; or
(b) the hypertension is treatment-resistant hypertension, the stable background anti-hypertensive regimen comprises at least three antihypertensive medications, and one antihypertensive medication is a diuretic.

5. (R)-Compound 1 for use of any one of the preceding claims, wherein the administration results in a decrease as compared to baseline in seated diastolic blood pressure (siDBP), at or after a 12-week treatment period, as compared to placebo.

6. (R)-Compound 1 for use of any one of the preceding claims, comprising administering 1 mg/day of (R)-Compound 1 to the human.

7. (R)-Compound 1 for use of claim 6, wherein the administration results in about an 8 mmHg decrease or more than an 8 mmHg decrease in the human's mean seated systolic blood pressure, at or after a 12-week treatment period, as compared to baseline.

8. (R)-Compound 1 for use of any one of claims 1-5, comprising administering 2 mg/day of (R)-Compound 1 to the human.

9. (R)-Compound 1 for use of claim 8, wherein the administration results in about an 11 mmHg decrease or more than an 11 mmHg decrease in the human's mean seated systolic blood pressure, at or after a 12-week treatment period, as compared to baseline.

10. (R)-Compound 1 for use of any one of the preceding claims, wherein administration is oral.

11. (R)-Compound 1 for use of any one of the preceding claims, wherein the (R)-Compound 1 is administered in a tablet, wherein the tablet comprises 1 mg or 2 mg of (R)-Compound 1.

12. (R)-Compound 1 for use of claim 11, wherein one tablet is administered to the human per day.

13. (R)-Compound 1 for use of any one of the preceding claims, wherein the amount of (R)-Compound 1 is administered to the human in a single dose.

14. (R)-Compound 1 for use of any one of the preceding claims, wherein the administration of the (R)-Compound 1 does not result in a clinically significant adverse event in the human, as compared to placebo.

15. (R)-Compound 1 for use of any one of the preceding claims, wherein the administration does not result in a clinically significant lowering in mean serum cortisol levels, as compared to placebo.

## Patentansprüche

1. (R)-Verbindung 1 zur Verwendung bei der Behandlung von unkontrollierter oder behandlungsresistenter Hypertonie bei einem Menschen, der einer Behandlung davon bedarf, umfassend ein Verabreichen während einer Behandlungsperiode von 1 mg/Tag oder 2 mg/Tag der (R)-Verbindung 1 an den Menschen: 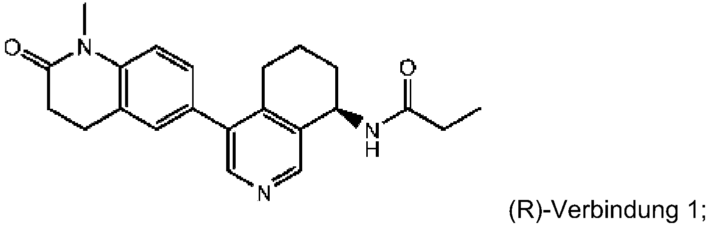 wobei sich der Mensch vor der Verabreichung und während der Behandlungsperiode unter einem stabilen antihypertensiven Hintergrundregimen befindet.

2. (R)-Verbindung 1 zur Verwendung nach Anspruch 1, wobei die Verabreichung von 2 mg/Tag der (R)-Verbindung 1 zu einer Verringerung des mittleren systolischen Blutdrucks im Sitzen (siSBP) des Menschen im Vergleich zu einem Ausgangswert bei oder nach einer 12-wöchigen Behandlungsperiode im Vergleich zu einem Placebo führt.

3. (R)-Verbindung 1 zur Verwendung nach Anspruch 1, wobei die Verabreichung von 1 mg/Tag der (R)-Verbindung 1 zu einer Verringerung des mittleren systolischen Blutdrucks im Sitzen (siSBP) des Menschen im Vergleich zu dem Ausgangswert bei oder nach einer 12-wöchigen Behandlungsperiode im Vergleich zu einem Placebo führt.

4. (R)-Verbindung 1 zur Verwendung nach einem der Ansprüche 1 bis 3, wobei (a) die Hypertonie eine unkontrollierte Hypertonie ist, das stabile antihypertensive Hintergrundregime mindestens zwei antihypertensive Medikamente umfasst und ein antihypertensives Medikament ein Diuretikum ist; oder
(b) die Hypertonie eine behandlungsresistente Hypertonie ist, das stabile antihypertensive Hintergrundregime mindestens drei antihypertensive Medikamente umfasst und ein antihypertensives Medikament ein Diuretikum ist.

5. (R)-Verbindung 1 zur Verwendung nach einem der vorstehenden Ansprüche, wobei die Verabreichung im Vergleich zu dem Ausgangswert zu einer Verringerung des systolischen Blutdrucks im Sitzen (siDBP) bei oder nach einer 12-wöchigen Behandlungsperiode im Vergleich zu einem Placebo führt.

6. (R)-Verbindung 1 zur Verwendung nach einem der vorstehenden Ansprüche, umfassend das Verabreichen von 1 mg/Tag der (R)-Verbindung 1 an den Menschen,

7. (R)-Verbindung 1 zur Verwendung nach Anspruch 6, wobei die Verabreichung zu einer Verringerung um etwa 8 mmHg oder einer Verringerung um mehr als 8 mmHg des mittleren systolischen Blutdrucks im Sitzen des Menschen bei oder nach einer 12-wöchigen Behandlungsperiode im Vergleich zu dem Ausgangswert führt.

8. (R)-Verbindung 1 zur Verwendung nach einem der Ansprüche 1 bis 5, umfassend das Verabreichen von 2 mg/Tag der (R)-Verbindung 1 an den Menschen.

9. (R)-Verbindung 1 zur Verwendung nach Anspruch 8, wobei die Verabreichung zu einer Verringerung um etwa 11 mmHg oder einer Verringerung um mehr als 11 mmHg des mittleren systolischen Blutdrucks im Sitzen des Menschen bei oder nach einer 12-wöchigen Behandlungsperiode im Vergleich zu dem Ausgangswert führt.

10. (R)-Verbindung 1 zur Verwendung nach einem der vorstehenden Ansprüche, wobei die Verabreichung oral ist.

11. (R)-Verbindung 1 zur Verwendung nach einem der vorstehenden Ansprüche, wobei die (R)-Verbindung 1 in einer Tablette verabreicht wird, wobei die Tablette 1 mg oder 2 mg der (R)-Verbindung 1 umfasst.

12. (R)-Verbindung 1 zur Verwendung nach Anspruch 11, wobei eine Tablette pro Tag an den Menschen verabreicht wird.

13. (R)-Verbindung 1 zur Verwendung nach einem der vorstehenden Ansprüche, wobei die Menge der (R)-Verbindung 1 in einer Einzeldosis an den Menschen verabreicht wird.

14. (R)-Verbindung 1 zur Verwendung nach einem der vorstehenden Ansprüche, wobei die Verabreichung der (R)-Verbindung 1 nicht zu einem klinisch signifikanten unerwünschten Ereignis bei dem Menschen im Vergleich zu einem Placebo führt.

15. (R)-Verbindung 1 zur Verwendung nach einem der vorstehenden Ansprüche, wobei die Verabreichung nicht zu einer klinisch signifikanten Senkung von mittleren Serumcortisolspiegeln im Vergleich zu einem Placebo führt.

## Revendications

1. (R)-Composé 1 pour utilisation dans le traitement d'hypertension non contrôlée ou résistante au traitement chez un humain nécessitant un traitement de celle-ci, comprenant l'administration pendant une période de traitement, de 1 mg/jour ou 2 mg/jour de (R)-Composé 1 à l'humain : 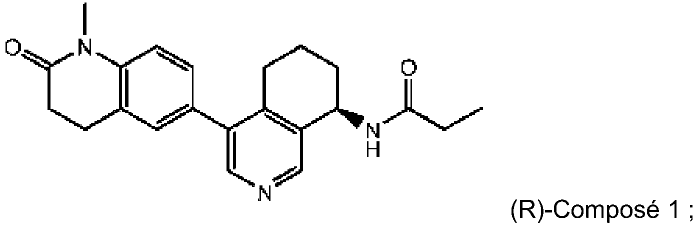 dans lequel l'humain est sous un régime antihypertenseur de fond stable avant l'administration et pendant la période de traitement.

2. (R)-Composé 1 pour utilisation selon la revendication 1, dans lequel une administration de 2 mg/jour du (R)-Composé 1 résulte en une diminution de la pression sanguine systolique en position assise (siSBP) moyenne de l'humain par comparaison avec une ligne de base, à ou après une période de traitement de 12 semaines, par comparaison avec un placebo.

3. (R)-Composé 1 pour utilisation selon la revendication 1, dans lequel une administration de 1 mg/jour du (R)-Composé 1 résulte en une diminution de la pression sanguine systolique en position assise (siSBP) moyenne de l'humain par comparaison avec une ligne de base, à ou après une période de traitement de 12 semaines, par comparaison avec un placebo.

4. (R)-Composé 1 pour utilisation selon l'une quelconque des revendications 1 à 3, dans lequel (a) l'hypertension est une hypertension non contrôlée, le régime antihypertenseur de fond stable comprend au moins deux médicaments antihypertenseurs, et un médicament antihypertenseur est un diurétique ; ou
(b) l'hypertension est une hypertension résistante au traitement, le régime antihypertenseur de fond stable comprend au moins trois médicaments antihypertenseurs, et un médicament antihypertenseur est un diurétique.

5. (R)-Composé 1 pour utilisation selon l'une quelconque des revendications précédentes, dans lequel l'administration résulte en une diminution par comparaison avec une ligne de base de la pression sanguine diastolique en position assise (siDBP), à ou après une période de traitement de 12 semaines, par comparaison avec un placebo.

6. (R)-Composé 1 pour utilisation selon l'une quelconque des revendications précédentes, comprenant l'administration de 1 mg/jour de (R)-Composé 1 à l'humain.

7. (R)-Composé 1 pour utilisation selon la revendication 6, dans lequel l'administration résulte en une diminution d'environ 8 mm Hg ou plus de 8 mm Hg de la pression sanguine systolique en position assise de l'humain, à ou après une période de traitement de 12 semaines, par comparaison avec la ligne de base.

8. (R)-Composé 1 pour utilisation selon l'une quelconque des revendications 1 à 5, comprenant l'administration de 2 mg/jour de (R)-Composé 1 à l'humain.

9. (R)-Composé 1 pour utilisation selon la revendication 8, dans lequel l'administration résulte en une diminution d'environ 11 mm Hg ou plus de 11 mm Hg de la pression sanguine systolique en position assise de l'humain, à ou après une période de traitement de 12 semaines, par comparaison avec la ligne de base.

10. (R)-Composé 1 pour utilisation selon l'une quelconque des revendications précédentes, dans lequel l'administration est orale.

11. (R)-Composé 1 pour utilisation selon l'une quelconque des revendications précédentes, dans lequel le (R)-Composé 1 est administré dans un comprimé, dans lequel le comprimé comprend 1 mg ou 2 mg de (R)-Composé 1.

12. (R)-Composé 1 pour utilisation selon la revendication 11, dans lequel on administre à l'humain un comprimé par jour.

13. (R)-Composé 1 pour utilisation selon l'une quelconque des revendications précédentes, dans lequel la quantité de (R)-Composé 1 est administrée à l'humain dans une dose unique.

14. (R)-Composé 1 pour utilisation selon l'une quelconque des revendications précédentes, dans lequel l'administration du (R)-Composé 1 ne résulte pas en un effet indésirable cliniquement significatif chez l'humain, par comparaison avec un placebo.

15. (R)-Composé 1 pour utilisation selon l'une quelconque des revendications précédentes, dans lequel l'administration ne résulte pas en un abaissement cliniquement significatif des taux moyens de cortisol sérique, par comparaison avec un placebo.
